# EUROPEAN PATENT SPECIFICATION

(11) **EP 3 126 512 B1**
(45) Date of publication and mention of the grant of the patent: **17.04.2019**
(21) Application number: 15706455.1
(22) Date of filing: 23.02.2015
(51) Int. Cl.: C12Q 1/68, A61K 47/50

(54) **PHOTO-SELECTIVE METHOD FOR BIOLOGICAL SAMPLE ANALYSIS FIELD**
PHOTOSELEKTIVES VERFAHREN FÜR ANALYSEFELD EINER BIOLOGISCHEN PROBE
PROCÉDÉ PHOTO-SÉLECTIF UTILISABLE DANS LE DOMAINE DE L'ANALYSE D'ÉCHANTILLONS BIOLOGIQUES

(30) Priority: 26.02.2014 US 201461944996 P
(43) Date of publication of application: 08.02.2017
(73) Proprietor: Ventana Medical Systems, Inc., Tucson, Arizona 85755 (US)
(72) Inventor: ALEXANDER, Nelson, Marana, Arizona 85658 (US); MORRISON, Larry, Oro Valley, Arizona 85737 (US); OZTURK, Sedide, Pleasanton, CA 94588 (US)
(74) Representative: Burger, Alexander
(86) International application number: PCT/EP2015/053681
(87) International publication number: WO 2015/128272

(56) References cited:
- WO-A2-2007/000669
- US-A1- 2011 172 115
- AGASTI SARIT S ET AL: "Photocleavable DNA Barcode-Antibody Conjugates Allow Sensitive and Multiplexed Protein Analysis in Single Cells", November 2012 (2012-11), JOURNAL OF THE AMERICAN CHEMICAL SOCIETY, VOL. 134, NR. 45, PAGE(S) 18499-18502, XP002739484, ISSN: 0002-7863 abstract; figure 1
- ULLAL ADEETI V ET AL: "Cancer Cell Profiling by Barcoding Allows Multiplexed Protein Analysis in Fine-Needle Aspirates", January 2014 (2014-01), SCIENCE TRANSLATIONAL MEDICINE, VOL. 6, NR. 219, XP002739485, ISSN: 1946-6234(print) the whole document
- LEMAIRE R ET AL: "Tag-mass: Specific molecular imaging of transcriptome and proteome by mass spectrometry based on photocleavable tag", JOURNAL OF PROTEOME RESEARCH, ACS, WASHINGTON, DC, US, vol. 6, no. 6, 1 June 2007 (2007-06-01), pages 2057-2067, XP009113669, ISSN: 1535-3893, DOI: 10.1021/PR0700044 [retrieved on 2007-05-04]
- OLEJNIK JERZY ET AL: "Photocleavable aminotag phosphoramidites for 5'-termini DNA/RNA labeling", NUCLEIC ACIDS RESEARCH, OXFORD UNIVERSITY PRESS, GB, vol. 26, no. 15, 1 August 1998 (1998-08-01), pages 3572-3576, XP002188068, ISSN: 0305-1048, DOI: 10.1093/NAR/26.15.3572

## Description

### FIELD

The present disclosure concerns embodiments of a method for performing molecular diagnostics in a tissue sample.

### BACKGROUND

Detecting proteins from formalin-fixed, paraffin-embedded (FFPE) tissue in a multiplexed and quantifiable fashion is an important aspect of tissue diagnostics. Current methods, such as immunohistochemistry, have low sensitivities, narrow dynamic ranges, and limited multiplexing capabilities. Unlike nucleic acids, proteins cannot be amplified; therefore, multiplexed detection techniques and quantifying low expressing protein targets from FFPE tissue can be difficult. Also, proteins of interest are typically expressed in a sample over a wide range of locations, making simultaneous detection of multiple targets from the same tissue sample even more difficult. Highly multiplexed tests, which are useful for detecting multiple targets in a single sample (*e.g.,* a tissue sample), require sample isolation. Lysing is a typical method used to remove testable analytes from the tissue for analysis. Typical lysing methods, such as macroscopic dissection and laser capture microdissection (LCM), can be problematic as these methods disrupt overall tissue context, can cause tissue destruction, and/or require physically removing sections of the tissue from a sample. Multiplexing assays in tissue samples also are limited to analyzing a limited number of targets due to the probes and labels used in these types of assays.

Chromosomal alterations within individual cells also can be analyzed using *in situ* hybridization techniques (ISH). Currently, these analyses are limited to aberrations larger than whole genes. Particular targets or modifications (*e.g.,* SNPs, promoter methylation, miRNA, and structural alterations substantially smaller than 100kb) cannot be detected using ISH. Ullal et al. (Sci Transl Med. 2014 Jan 15;6(219):219) describes the importance of multiplexed diagnostic methods to expand diagnostic capability in oncology. In particular, the use of DNA-barcoded antibodies and the detection of the DNA tag after its release from the target-bound antibody portion is disclosed. Antibodies of interest are tagged with short (∼ 70mer) unique DNA "barcodes" using a photocleavable linker. After exposure to ultraviolet light the DNA is released from the antibody, isolated and detected in a hybridization assay with complementary fluorescently labeled DNA probes. While that disclosure provides robust analysis of the medical value provided by highly multiplexed assays, the samples were limited to fine needle aspirates which are divorced from contextual information as described herein.

US 2011/172115 A1 teaches probes and methods for the imaging of specimens such as tissue samples or other biological specimens or arrays using mass spectrometry. Mass tags are coupled to each probe molecule via a cleavable linker. After irradiating a portion of the specimen with a laser beam ions are released from the specimen, the ions are selected with mass-to-charge ratios corresponding to the mass tags or derivatives of the mass tags, and the amount and type of selected ions is recorded together with the location of the portion of the specimen.

Agasti et al. (J Am Chem Soc. 2012 Nov 14;134(45):18499-18502) disclose the use of photo-cleavable DNA barcode-antibody conjugates for the detection of proteins in single cells. Similar to the mass tag/probe conjugates of US 2011/172115 A1, the DNA barcode is released by photocleavage after the conjugate has bound to its protein target. The barcodes are subsequently amplified by PCR, and the resulting amplicons are analyzed by gel electrophoresis.

A need exists in the art for biological assays that produce quantifiable data and that can combine sensitive and multiplexed target analysis using next generation sequencing, PCR, and/or array technology in a variety of biological samples.

### SUMMARY

The methods disclosed herein can be used to perform contextual molecular diagnostics in a tissue sample.Contextual molecular diagnostics can be performed by contacting a tissue sample with a probe comprising a photo-reactive moiety, a specific binding moiety to a target, and a detection tag using conditions sufficient to facilitate binding of the specific binding moiety to a target in the sample; removing probe that does not bind to the sample; adding a template sequence to the tissue sample, wherein the template sequence is configured to specifically hybridize with the detection tag; selecting a region of the sample for irradiation based on contextual information; irradiating the selected region of the tissue sample with light of a wavelength and an intensity sufficient to cause the photo-reactive moiety to react, thereby freeing the detection tag for further reaction; exposing the tissue sample to an enzyme that acts on the detection tag to effect a change; and detecting the change, wherein the detection tag is an extendable primer, the photo-reactive moiety is a photo-cleavable blocking group, the enzyme is a polymerase, and the change is an extension of the primer sequence the presence of dATP, dCTP, dTTP, and dGTP on the template sequence. In another embodiment, the method further includes ligating adjacent detection tags after irradiating the selected region.

The foregoing and other objects, features, and advantages of the invention will become more apparent from the following detailed description, which proceeds with reference to the accompanying figures.

### BRIEF DESCRIPTION OF THE DRAWINGS

FIG. 1(A) - (E) are a schematic drawings of a probe showing (A) a specific binding moiety and a detection tag coupled through a photo-reactive linker, (B) a probe in which the specific binding moiety is an anti-body, and (C) a probe in which the specific binding moiety is an oligonucleotide/polynucleotide, (D) a specific binding moiety, a detection tag, and a photo-reactive moiety coupled thereto, and (E) a probe in which the specific binding moiety is an oligonucleotide/polynucleotide.
FIG. 2(A) - (B) show (A) a schematic representation of a detection tag with various identification components coupled to a photo-cleavable moiety and (B) a sequence (SEQ ID NO: 1) representation of a detection tag showing exemplary identification parts.
FIG. 3 is a schematic drawing showing a method of performing contextual molecular diagnostics.
FIG. 4 is a photomicrograph showing an approach to selecting two regions of a sample.
FIG. 5(A) - (C) are schematic drawings showing the use of (A) a microscope objective, (B) a liquid crystal screen, and (C) a digital micromirror device to selectively irradiate a region of a sample.
FIGS. 6(A) - 6(F) are schematic drawings illustrating embodiments of a disclosed method, wherein (A) illustrates a probe bound to a sample prior to exposure to a light exposure step illustrated in (B). FIG. 6(C) illustrates subsequent probe extension and (D) shows the extended probe being subjected to next generation sequencing. FIG. 6(E)-(F) show an alternative light-activation approach analogous to FIG. 6(A)-(B).
FIG. 7 is a schematic drawing illustrating an aspect of a disclosed method whereby a probe comprising an oligo/polynucleotide, a photo-cleavable moiety, and a tag sequence modified with a detectable label is added to a sample and irradiated with light.
FIG. 8 is a schematic drawing illustrating an embodiment of a disclosed method whereby a caged ATP moiety is used so that irradiating with light liberates the caged ATP and enables detection.
FIG. 9 is a schematic drawing illustrating an embodiment of a disclosed method whereby a ligase joins two different adjacent probes bound to targets within a sample.
FIG. 10 is a schematic drawing illustrating a method for performing contextual tissue diagnostics which shows iterative irradiation of a sample and separate collection of detection tags therefrom into distinct vessels, which may or may not include location tags.
FIG. 11 is a photographic image illustrating results obtained from a control sample used in an example of a disclosed method.
FIG. 12 is a photographic image illustrating selective irradiation of a sample.
FIG. 13 is a photographic image illustrating selective irradiation of a sample.
FIG. 14 is an exploded view of the photographic image of FIG. 13, further illustrating the difference in signal obtained from a sample that has been selectively irradiated.
FIG. 15 is a photographic image of a patterned sample obtained by selectively irradiating a slide with a digital micromirror device and a patterned mask.
FIG. 16 is an exploded view of the image illustrated in FIG. 15.
FIG. 17 is an image of an electrophoresis gel illustrating the detection of unique sequence identifiers after photoselective cleavage from a specific binding moiety.
FIG. 18 is a schematic drawing illustrating an *in situ* polymerase extension embodiment whereby reverse primers are used to detect regions of the extended probe that are further away from the initial probe sequence.
FIG. 19 is an image of an electrophoresis gel analyzing isolated sequences obtained using *in situ* polymerase extension.
FIG. 20 is an image of an electrophoresis gel analyzing isolated CHR17 sequences obtained using *in situ* polymerase extension.
FIG. 21 is an image of an electrophoresis gel analyzing isolated sequences obtained from extension of two different probes within the same sample using *in situ* polymerase extension.
FIG. 22(A) - (B) is a (A) a schematic drawing illustrating an *in situ* polymerase extension embodiment and (B) is an image of an electrophoresis gel analyzing isolated sequences therefrom.
FIG. 23(A) - (B) are images of an electrophoresis gel analyzing isolated sequences obtained for BRAF Exon 13 (A) and for HER2 Exon 10 (B) probes extended with and without caged thymidines.

### DETAILED DESCRIPTION

### I. Terms

Unless otherwise noted, technical terms are used according to conventional usage. Definitions of common terms in molecular biology may be found in Benjamin Lewin, Genes VII, published by Oxford University Press, 2000 (ISBN 019879276X); Kendrew et al. (eds.), The Encyclopedia of Molecular Biology, published by Blackwell Publishers, 1994 (ISBN 0632021829); and Robert A. Meyers (ed.), Molecular Biology and Biotechnology: a Comprehensive Desk Reference, published by Wiley, John & Sons, Inc., 1995 (ISBN 0471186341); and other similar references.

As used herein, the singular terms "a," "an," and "the" include plural referents unless context clearly indicates otherwise. Similarly, the word "or" is intended to include "and" unless the context clearly indicates otherwise. Also, as used herein, the term "comprises" means "includes." Hence "comprising A or B" means including A, B, or A and B. Nucleotide sizes or amino acid sizes, and molecular weight or molecular mass values, stated for nucleic acids or polypeptides or other compounds are generally approximate, and are provided for description.

Concentrations, amounts, and other numerical data may be expressed or presented herein as a range. Such ranges are used merely for convenience and brevity and should be interpreted to include not only the numerical values explicitly recited as the range limits, but also to include all individual numerical values or sub-ranges encompassed within that range as if each numerical value and sub-range is explicitly recited. As an illustration, a numerical range of "about 1 to about 5" includes not only the explicitly recited values of about 1 to about 5, but also values and sub-ranges within the indicated range, such as 2, 3, and 4 and sub-ranges such as from 1-3, from 2-4, and from 3-5, etc., as well as 1, 2, 3, 4, and 5 individually. This same principle applies to ranges that recite only one numerical value as a minimum or a maximum. Furthermore, such an interpretation should apply regardless of the breadth of the range or the characteristics being described.

Although methods and materials similar or equivalent to those described herein can be used in the practice or testing of the present disclosure, suitable methods and materials are described below. In addition, the materials, methods, and examples are illustrative only and not intended to be limiting.

The following explanations of specific terms are provided to facilitate review of the various disclosed embodiments:

**"Antibody"** occasionally abbreviated "Ab," refers to immunoglobulins or immunoglobulin-like molecules (including by way of example and without limitation, IgA, IgD, IgE, IgG and IgM, combinations thereof), similar molecules produced during an immune response, (*e.g.,* in mammals such as humans, goats, rabbits and mice) as well as antibody fragments, that specifically bind to a molecule of interest (or a group of highly similar molecules of interest) to the substantial exclusion of binding to other molecules. For example, antibodies and antibody fragments typically have a binding constant for the molecule of interest that is at least 10³ M⁻¹ greater, at least 10⁴ M⁻¹ greater, or at least 10⁵ M⁻¹ greater than a binding constant for other molecules in a biological sample. "Antibody" further refers to a polypeptide ligand comprising at least a light chain or heavy chain immunoglobulin variable region that specifically recognizes and binds an epitope of an antigen. Antibodies may comprise of a heavy and a light chain, each of which has a variable region, termed the variable heavy (VH) region and the variable light (VL) region. Together, the VH region and the VL region are responsible for binding an antigen recognized by the antibody. "Antibody" also includes intact immunoglobulins and the variants and portions of them well known in the art. Antibody fragments include proteolytic antibody fragments, such as F(ab')2 fragments, Fab' fragments, Fab'-SH fragments and Fab fragments as are known in the art; recombinant antibody fragments, such as sFv fragments, dsFv fragments, bispecific sFv fragments, bispecific dsFv fragments, F(ab)'2 fragments; single chain Fv proteins ("scFv"); disulfide stabilized Fv proteins ("dsFv"); diabodies; triabodies (as are known in the art); and camelid antibodies (see, for example, U.S. Patent Nos. 6,015,695; 6,005,079, 5,874,541; 5,840,526; 5,800,988; and 5,759,808). "Antibody" also includes monoclonal antibodies, which are characterized by being produced by a single clone of B lymphocytes or by a cell into which the light and heavy chain genes of a single antibody have been transfected. Monoclonal antibodies are produced by methods known to those of skill in the art. Monoclonal antibodies include humanized monoclonal antibodies.

**"Conjugate"** refers to two or more moieties directly or indirectly coupled together. For example, a first moiety may be covalently or noncovalently (*e.g.,* electrostatically) coupled to a second moiety. Indirect attachment is possible, such as by using a linker, which may be a molecule or group of atoms positioned between two moieties.

**"Contacting"** refers to placement that allows association between two or more moieties, particularly direct physical association, for example both in solid form and/or in liquid form (for example, the placement of a biological sample, such as a biological sample affixed to a slide, in contact with a composition, such as a solution containing the probes disclosed herein).

**"Context, -ual"** refers to the environment associated with a sample, wherein the integrity of, or information obtained from, a certain portion of the sample can be determined by comparing it with the entire sample environment. Contextual information is that information that can be understood by considering a sample's spatial and relational characteristics. As an example, contextual information for a tissue sample would include the type of cells and the spatial relationship between the various cells in the tissue sample. According to one aspect, pathologists gather and interpret contextual information as they examine a tissue sample microscopically or through an image using the cellular structures, morphological features of various cells and tissues, or through the interpretation of a stain (*i.e.,* immunohistochemistry (IHC) antibody stain) within the sample. The contextual information can include classification of a cell or region as tumor or normal tissue. Contextual information can also include an observation of spatial tumor heterogeneity or the recognition of an invasive margin or other biological features evident through visual inspection. Contextual information can include identification of tissue features such as the vasculature, or of immune cell infiltration. As used herein, it is understood that contextual information is destroyed in some way when a sample is lysed or ground so as to create a solution from the sample. The creation of this solution takes the various components from their biological locations and places them in a solution where at least some of the spatial relationships are lost. Contextual information can be preserved when the chemical or molecular diagnostic information can be related back to an image or sample location. In another example, chemical or molecular imaging of a sample can be considered as preserving the contextual information of a sample.

**"Detect"** refers to the ability to determine if an agent (such as a signal or particular antigen, protein or nucleic acid) is present or absent, for example, in a sample. In some examples, this can further include quantification, and/or localization, for example localization within a cell or particular cellular compartment. "Detecting" refers to any method of determining if something exists, or does not exist, such as determining if a target molecule is present in a biological sample. For example, "detecting" can include using a visual or a mechanical device to determine if a sample displays a specific characteristic. In certain examples, light microscopy and other microscopic means are used to detect a detectable label bound to or proximally to a target. Detecting can also encompass performing sequencing, array analysis and/or PCR amplification of a detection tag.

**"Detectable Label"** refers to a molecule or material that can be detected or that can produce a detectable (such as visually, electronically or otherwise) signal that indicates the presence, number, location, and/or concentration of a target, such as a target molecule, in a sample, such as a tissue sample. When conjugated to a molecule capable of binding directly or proximally to a target, the detectable label can be used to locate and/or quantify the target. A detectable label can be detected directly or indirectly, and several different detectable labels can be used in combination to detect one or more targets.

**"Hybridization"** refers to forming base pairs between complementary regions of two strands of DNA, RNA, or between DNA and RNA, thereby forming a duplex molecule. Hybridization conditions resulting in particular degrees of stringency will vary depending upon the nature of the hybridization method and the composition and length of the hybridizing nucleic acid sequences. Generally, the temperature of hybridization and the ionic strength (such as the Na⁺ concentration) of the hybridization buffer will determine the stringency of hybridization. The presence of a chemical which decreases hybridization (such as formamide) in the hybridization buffer will also determine the stringency (Sadhu et al., J. Biosci. 6:817-821, 1984). Calculations regarding hybridization conditions for attaining particular degrees of stringency are discussed in Sambrook et al., (1989) Molecular Cloning, second edition, Cold Spring Harbor Laboratory, Plainview, NY (chapters 9 and 11). Hybridization conditions for ISH are also discussed in Landegent et al., Hum. Genet. 77:366-370, 1987; Lichter et al., Hum. Genet. 80:224-234, 1988; and Pinkel et al., Proc. Natl. Acad. Sci. USA 85:9138-9142, 1988.

**"Multiplex, -ed, -ing"** refers to detecting multiple targets in a sample, or multiple targets in multiple samples, concurrently, substantially simultaneously, or sequentially. Multiplexing can include identifying and/or quantifying multiple distinct nucleic acids (*e.g.,* DNA, RNA, mRNA, miRNA) and polypeptides (*e.g.,* proteins), both individually and in any and all combinations.

**"Oligonucleotide"** refers to a plurality of joined nucleotides joined by phosphodiester bonds. While the number of nucleotides can vary, oligonucleotides typically include from about 6 to about 500 nucleotides. "Oligonucleotide" refers to DNA oligonucleotides, RNA oligonucleotides, synthetic oligonucleotides (*e.g.,* sequences that are, or contain, non-naturally occurring DNA or RNA sequences), and oligonucleotide analogs, which are moieties that function similarly to oligonucleotides but which have non-naturally occurring portions. For example, oligonucleotide analogs can contain non-naturally occurring portions, such as altered sugar moieties or inter-sugar linkages, such as a phosphorothioate oligodeoxynucleotide. Functional analogs of naturally occurring polynucleotides can bind to RNA or DNA, and include peptide nucleic acid molecules.

**"Proximal"** refers to being situated at or near a reference point. As used herein, proximal means within about 5000 nm, within about 2500 nm, within about 1000 nm, within about 500 nm, within about 250 nm, within about 100 nm, within about 50 nm, within about 10 nm, or within about 5 nm of the reference point.

**"Sample"** refers to a biological specimen containing biological molecules, such as genomic DNA, RNA (including mRNA), genes, amino acids, peptides, proteins, or combinations thereof, obtained from a subject. Examples include, but are not limited to, blood (*e.g.,* peripheral blood), urine, saliva, tissue biopsy, surgical specimen, amniocentesis samples and autopsy material.

**"Specific binding moiety"** refers to a moiety/compound/sequence that specifically binds to a second member of a specific binding pair or a target to the substantial exclusion of binding to other potential binding pairs or non-targeted areas of a sample. Specific binding pairs are pairs of molecules that bind each other to the substantial exclusion of binding to other molecules (for example, specific binding pairs can have a binding constant that is at least 10³ M⁻¹ greater, 10⁴ M⁻¹ greater or 10⁵ M⁻¹ greater than a binding constant for either of the two members of the binding pair with other molecules in a biological sample). Exemplary specific binding moieties include, but are not limited to, proteins (*e.g.,* antibodies or a binding variable region thereof), polynucleotides (*e.g.,* DNA, RNA, etc.), nucleotides, oligonucleotides, polypeptides, peptides, amino acids, aptamers, a member of a specific binding pair, a primer, a plasmid, or combinations thereof.

**"Target nucleic acid sequence or molecule"** refers to a defined region or particular portion of a nucleic acid molecule, for example a portion of a genome (such as a gene or a region of mammalian genomic DNA containing a gene of interest). In an example where the target nucleic acid sequence is a target genomic sequence, such a target can be defined by its position on a chromosome (*e.g.,* in a normal cell), for example, according to cytogenetic nomenclature by reference to a particular location on a chromosome; by reference to its location on a genetic map; by reference to a hypothetical or assembled contig; by its specific sequence or function; by its gene or protein name; or by any other means that uniquely identifies it from among other genetic sequences of a genome. In some examples, the target nucleic acid sequence is mammalian genomic sequence (for example human genomic sequence).

In some examples, alterations of a target nucleic acid sequence (*e.g.,* genomic nucleic acid sequence) are "associated with" a disease or condition. In some examples, detection of the target nucleic acid sequence can be used to infer the status of a sample with respect to the disease or condition. For example, the target nucleic acid sequence can exist in two (or more) distinguishable forms, such that a first form correlates with absence of a disease or condition and a second (or different) form correlates with the presence of the disease or condition. The two different forms can be qualitatively distinguishable, such as by polynucleotide polymorphisms, and/or the two different forms can be quantitatively distinguishable, such as by the number of copies of the target nucleic acid sequence that are present in a cell.

**"Unique sequence identifier"** refers to a nucleic acid sequence that is configured to have a sequence that is specifically correlated to a particular target and/or specific binding moiety of a probe. Each unique sequence identifier can have a sequence that differs from other unique sequence identifiers present in a sample and can therefore be used to identify a particular target and or specific binding moiety of a probe.

### II. Overview

The present disclosure concerns analytical techniques for tissue samples. The analytical techniques contemplated by the present disclosure can include, but are not limited to, target detection, genetic sequencing (*e.g.,* next generation sequencing), multiplexing, amplification (*e.g.,* polymerase chain reaction, ligase chain reaction, etc.), mass spectrometry-based protein analysis, and the like. The analytical techniques disclosed herein can be facilitated by using photochemical-based technology.

The present disclosure also concerns analytical techniques and isolation techniques that address a current need in the art to provide efficient methods for analyzing multiple targets, and in some situations, a large number of targets. Analytical and isolation techniques also are disclosed that do not destroy the sample undergoing analysis, which thereby maintains sample context integrity. For example, methods for detecting targets in a tissue sample that are currently known in the art are limited to detecting only a few targets, given the types of probes available for tissue analysis. These methods also typically require various cumbersome and tissue-destructive isolation techniques (*e.g.,* tissue lysis or laser capture microdissection) to provide isolated targets that undergo subsequent analysis. The present disclosure addresses these problems and limitations by providing probes, methods of using the probes, and selective isolation techniques exemplified by the disclosure provided herein.

### III. Probes

Disclosed herein are probe embodiments for detecting one or more targets in a sample and/or providing genetic information useful for determining the presence of genetic aberrations. In some embodiments, a probe is used to facilitate analyzing formalin-fixed paraffin embedded (FFPE) samples, fresh samples, or frozen samples. The samples may be tissue samples. Additionally, disclosed probes can provide substantially unlimited numbers of combinations of detection tags. This unique feature is particularly suitable for detecting multiple targets in multiplexing assays.

Particular disclosed probe embodiments comprise a photo-cleavable moiety which confers particular benefits for analytical methods and isolation. For example, using disclosed photo-cleavable moieties may reduce background signals during analysis by decreasing non-specific binding event detection. For example, duplexes formed between a template sequence and a tag sequence attached to a specifically bound probe comprising a photo-cleavable moiety can be cleaved by an irradiation event and can be subsequently isolated and analyzed. In contrast, non-specifically bound template sequences (which contribute to background noise) that hybridize with sample through a non-photo-labile interaction (*e.g.,* electrostatic and/or hydrophobic interactions) may not be cleaved by the irradiation step and therefore can potentially be sequestered on the sample rather than isolated with the desired probes that were cleaved during the irradiation event. Under this scenario, only the desired probes (or desired portions of the probe) undergo subsequent analysis (*e.g.,* amplification, genetic sequencing, array analysis, etc.). As such, non-photo-labile probes can be added to the sample to block sample sequences that are creating background issues. While certain probes described herein include a covalently bound photo-cleavable or photo-reactive moiety, the term probe is not so restricted. In other examples, the probe may include a photo-reactive moiety or a photo-cleavable moiety present on a separate molecule that interacts with the specific binding moiety and/or labeling components of the probe subsequent to irradiation.

### A. Probe Components

Certain embodiments of the probe comprise a specific binding moiety (SBM) suitable for binding to a particular binding pair, such as a biological target. The probe also may comprise a photo-reactive moiety (PRM). In illustrative embodiments, the photo-reactive moiety is a photo-cleavable moiety. In particular, the photo-cleavable moiety may be capable of being cleaved from other probe components, such as the specific binding moiety, upon exposure to light having a suitable wavelength. Embodiments of the probe may further comprise a detection tag (TAG) suitable for downstream analysis (*e.g.,* array-based analysis, sequencing, and/or PCR amplification). Each of these components, as well as other probe components, is discussed in more detail below.

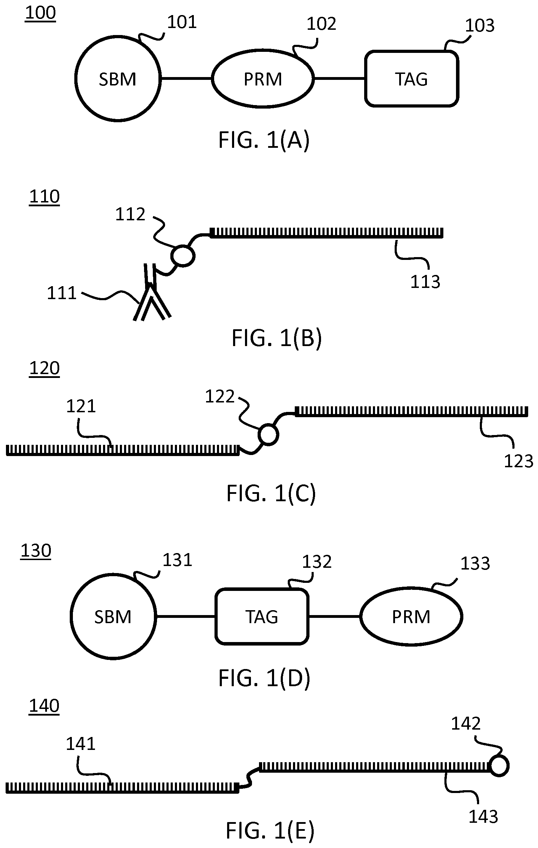

Exemplary probe embodiments are illustrated in FIGS. 1(A) - (E). FIG. 1(A) provides a generic illustration of a probe embodiment 100 comprising specific binding moiety 101, photo-cleavable moiety 102, and detection tag 103. FIG. 1(B) illustrates exemplary probe 110, wherein the specific binding moiety 101 is an antibody 111; the probe further comprising photo-cleavable moiety 102 and unique sequence identifier 113, which acts as the detection tag. Unique sequence identifier 113 may include a particular sequence, or can be configured to have a particular size (*e.g.,* certain number of nucleotides). These variable and selectable characteristics provide the ability to uniquely and discriminately detect each different unique sequence identifier effectively coupled to a sample target by a probe. FIG. 1(C) illustrates exemplary probe 120, wherein the specific binding moiety is an oligonucleotide 121; the probe further comprising photo-cleavable moiety 122 and unique sequence identifier 123.

FIG. 1(D) provides a generic illustration of a probe embodiment 130 comprising specific binding moiety 131, detection tag 132, and photo-cleavable moiety 133. FIG. 1(E) illustrates exemplary probe 140, wherein the specific binding moiety is an oligonucleotide 141; the probe further comprising unique sequence identifier 143 and photo-cleavable moiety 142. While not shown, a probe analogous to exemplary probe 140 and 110 is understood to be within the scope of the present disclosure. In particular, another exemplary probe would include an antibody specific binding moiety with a coupling pattern analogous to 130.

### 1. Photo-Reactive Moiety (PRM)

The photo-reactive moiety is a moiety that reacts or is cleaved by light. In some examples, the photo-reactive moiety is cleaved from one or more probe components. In some examples, a photo-cleavable moiety is capable of being chemically activated by light and being separated, typically chemically separated (such as by bond cleavage), from the component (or components) to which it is coupled. In some examples, the cleavage event separates portions of the probe (*e.g.,* separates the specific binding moiety from the detection tag). In other examples, the cleavage event cleaves a protecting group. In some examples, the photo-cleavable moiety is capable of absorbing light of a certain wavelength. In particular disclosed examples, the photo-cleavable moiety may be cleaved by ultraviolet light and/or visible light. Suitable ultraviolet light typically has a wavelength ranging from about 200 nm to about 400 nm, such as from about 250 nm to about 375 nm, or from about 260 nm to about 365 nm, or from about 280 nm to about 350 nm. Suitable visible light typically has a wavelength ranging from about 400 nm to about 790 nm, such as from about 450 nm to about 750 nm, or from about 500 nm to about 725 nm, or from about 550 nm to about 700 nm.

The photo-cleavable moiety used in certain probe embodiments may be selected based on considering certain physical properties of the probe and conditions associated with using the probe in a biological assay. For example, the photo-cleavable moiety may be selected based on the kinetics of the photo-based cleavage reaction. Thus, certain exemplary criteria for selection include the rate at which the photo-cleavable moiety is photochemically cleaved, the efficiency of photochemical cleavage, its size, and its stability under the analytical/storage conditions selected. In some embodiments, the photo-cleavable moiety should be photochemically cleaved substantially immediately upon exposure to light of the appropriate wavelength, such as at within 1 minute of being exposed to light of the appropriate wavelength, and preferably no more than about to about 5 minutes after being exposed to light of the appropriate wavelength. For example, a photo-cleavable moiety may be used that can be cleaved under 1 minute after being exposed to light of the appropriate wavelength. In some examples, substantially all photo-cleavable moieties exposed to light should be cleaved within this time frame. For example, from about 50% to about 100% of the photo-cleavable moieties exposed to light of the appropriate wavelength should be cleaved, with some examples having from about 60% to about 99% cleavage, or from about 70% to about 95% cleavage, or from about 80% to about 90% cleavage.

The probe comprising the photo-cleavable moiety should be sufficiently stable to conditions associated with a particular analytical technique to substantially preclude degradation. For example, the probe comprising the photo-cleavable moiety should be substantially non-degraded by process solutions used, pH conditions used, and/or temperatures associated with a particular technique. Furthermore, the probe and the photo-cleavable moiety should be sufficiently robust before and after cleavage to prevent any destructive by-products from interfering with sample analysis.

In some disclosed examples, the photo-cleavable moiety can comprise a linker suitable for linking it with probe components disclosed herein. Suitable linkers include, but are not limited to aliphatic linkers, alkylene oxide linkers, disulfide linkers, commercially available linkers, polymeric linkers, and the like. The linker may be selected to impart increased hydrophilicity or hydrophobicity to the probe. Solely by way of example, alkylene oxide linkers disclosed herein can be used to link the photo-cleavable moiety to one or more of the probe components disclosed herein and to increase the hydrophilic nature of the entire probe.

Some examplesof the disclosed photo-cleavable moiety have a Formula I or Formula II, provided below.

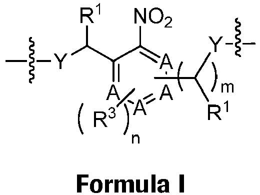

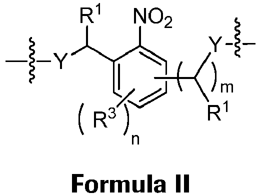

With reference to Formula I or Formula II, each R¹ independently may be halogen, hydrogen or alkyl (*e.g.,* methyl, ethyl, propyl, and the like); each R³ independently may be selected from alkyl, aryl, hydrogen, hydroxyl, alkoxy, ether, ester, amine, amide, or halogen; each Y independently can be selected from oxygen, sulfur, NH, NR⁴ (where R⁴ is aliphatic, particularly alkyl), -C(O)-, -OC(O)-, -NHC(O)-, or halogen (*e.g.,* Cl, Br, Fl, or I); each A independently may be carbon or nitrogen; n can be 0, 1, 2, or 3; and m can be 0 or 1.

Exemplary species of the photo-cleavable moiety are illustrated below. With reference to these species and the general formulas provided herein, a " 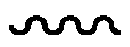 " symbol is used to indicate a truncated bond to other components to which the photo-cleavable moiety may be bound (*e.g.,* a specific binding moiety, a linker, a detection tag, etc.).

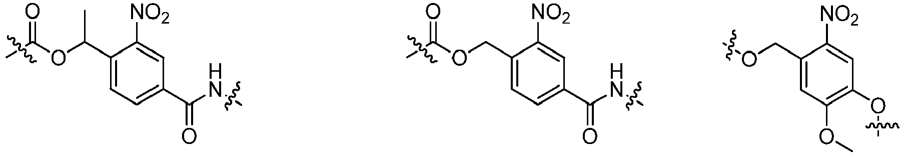

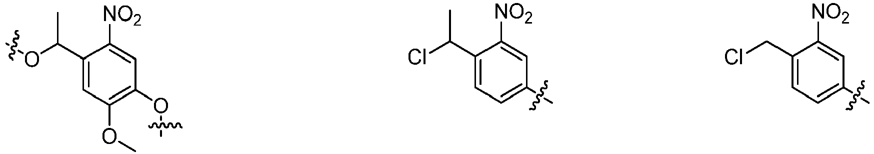

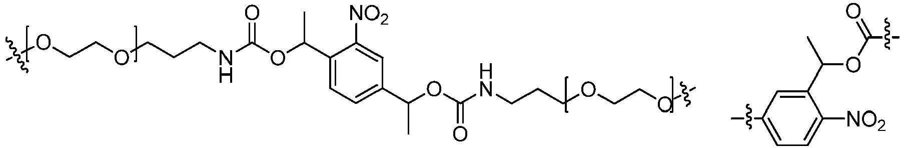

For example, the photo-cleavable moiety may have Formula III or Formula IV, provided below.

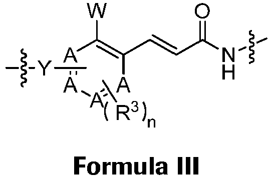

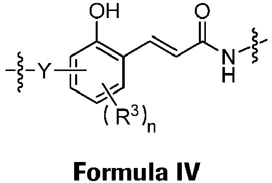

With reference to both Formula III and Formula IV, each of R³, Y, and n may be as indicated above for Formula I. The "W" variable of Formula III can be selected from OH or N(R⁴)₂ (where at least one of R⁴ is hydrogen and the other R⁴ moiety may be hydrogen or aliphatic, particularly alkyl). Examples of a photo-cleavable linker having a Formula II are provided below. With reference to these examples, PG refers to an amine protecting group, which would be readily recognized by persons of ordinary skill in the art.

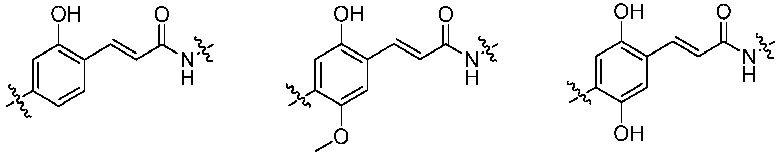

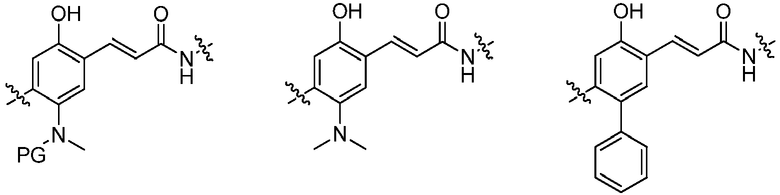

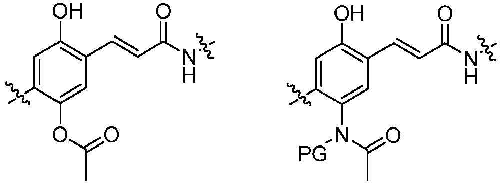

The photo-cleavable moiety may include or be bound to, either directly or indirectly, additional functional groups or moieties suitable for incorporating and/or facilitating the use of the photo-cleavable moiety in the methods disclosed herein. For example, the photocleavable moiety may be bound to a phosphoramidite to facilitate incorporation of the photo-cleavable moiety into a nucleic acid specific binding moiety. In other examples, the photocleavable moiety may be bound to a detectable label, such as a biotin molecule. In particular, the photo-cleavable moiety may be selected from a phosphoramidite-containing photo-cleavable moiety, a biotin-containing photo-cleavable moiety, and/or an amine-containing photo-cleavable moiety. Examples comprising a photo-cleavable moiety are provided below.

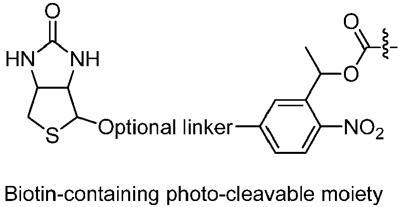

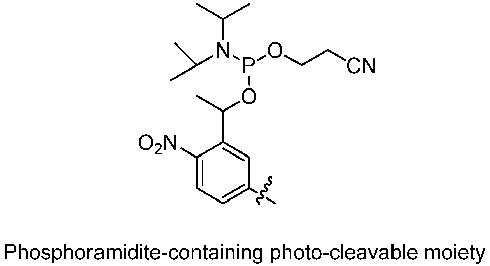

In some examples, the photo-cleavable moiety may act as a linker to couple two different components together. For example, the photo-cleavable moiety couples a specific binding moiety to another probe component, such as a detection tag. In other disclosed examples, such as when the specific binding moiety is a primer sequence, the photo-cleavable moiety may serve as a polymerase blocking moiety on the 3' end of the primer sequence, the 5' end of the primer sequence, or both; but more typically the 3' end of the primer sequence. The photo-cleavable moiety can thereby block the 3' end of the primer sequence from being extended by a polymerase enzyme on those particular portions of the sample that are not exposed to light of a suitable wavelength. Only those primers present on the irradiated portion of the sample are extended by the polymerase enzyme as the blocking portion is removed and the primer is therefore capable of being chemically modified by a polymerase enzyme. In another example, caged thymidines can be used analogously. It was observed that caged thymidines can be used to inhibit *in situ* polymerase extension by interfering with base-pairing.

Photo-cleavable moieties also may be present on a component other than the probe. For example, particular disclosed embodiments of the method use photo-cleavable moieties to block, or cage, ATP molecules - that is, the photo-cleavable moiety prevents ATP from being used in an ATP-dependent enzymatic reaction until a sample is irradiated with light of a suitable wavelength to cleave the photo-cleavable moiety and release the ATP molecule. An exemplary blocked ATP compound (**100**) is provided below in Scheme 1. Upon exposure to light of a suitable wavelength, ATP compound **102** is obtained.

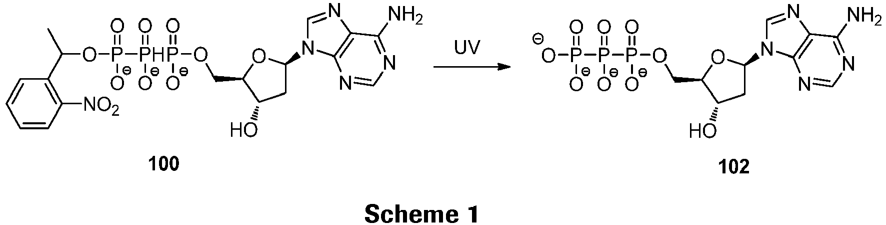

Reference is made to U.S. Patent No. 7,432,368 and U.S. Patent Publication No. 2014/0051605 for disclosure related to methods and compositions for photo-cleavable moieties.

Similarly, in another examplecaged deoxynucleoside triphosphates are employed to prevent extension of a primer hybridized to a target nucleic acid sequence by a polymerase enzyme (*e.g.,* DNA polymerase and its variants, when the target is DNA, or reverse transcriptase when the target is RNA). All four deoxynucleoside triphposphates may be caged or as few as a single nucleoside trip may be caged with the remaining deoxynucleoside triphosphases being uncaged to prevent significant primer extension in the absence of illumination. An example of a caged deoxynucleoside triphosphate is caged dATP, an example of which is pictured in scheme 1 except that a deoxyribose sugar would replace the ribose sugar moiety. An example of synthesizing caged dATP is described in *The Development of Methods for the Time-Resolved Imaging of the Replication of Single DNA Molecules,* Richard David Perrins Ph.D. thesis, the University of Birmingham, August 2005. The preparation of caged dideoxyribosylthymine triphosphate, dideoxyadenosine triphosphate and arabinosylcytosine triphosphate is described by Meldrum et al. in "Kinetics and mechanism of DNA repair, Biochem. J. (1990) 266, 885-890. Walker et al. describe "Photolabile 1-(2-Nitrophenyl)ethyl Phosphate Esters of Adenine Nucleotide Analogues. Synthesis and Mechanism of Photolysis" in J. Am. Chem. Soc. 1988, 110, 7170-7177. In another reference, Meldrum et al. also describe the "Use of Caged Compounds in Studies of the Kinetics of DNA Repair" in METHODS IN ENZYMOLOGY, VOL. 291, 1998.

### 2. Specific Binding Moieties

Specific binding moieties can be selected from an amino acid, a peptide, a protein (*e.g.,* an antibody or a fragment thereof), a nucleic acid, a nucleotide, an oligonucleotide, a polynucleotide (*e.g.,* DNA, RNA, miRNA, etc.), a member of a specific binding pair (*e.g.,* biotin, avidin, streptavidin, etc.), a primer, an aptamer, a plasmid, or combinations thereof. Suitable antibodies for use as specific binding moieties include, but are not limited to, primary antibodies and/or secondary antibodies. For example, the antibody may be a primary antibody that binds directly to a target in a sample, or it may be a secondary antibody (*e.g.,* anti-species antibody) that binds indirectly to a target in a sample through another antibody. The antibody also can be a monoclonal antibody. Suitable exemplary specific antibodies include, but are not limited to, a HER-2/neu rabbit monoclonal antibody, a PR rabbit monoclonal antibody, a topoisomerase IIa rabbit monoclonal antibody, an ER rabbit monoclonal antibody, a Ki-67 rabbit monoclonal antibody, a p53 antibody, and any other antibody that may be suitable for use in analyzing a sample having known or hereafter determined targets, including any of the particular target sequences disclosed herein. The probes disclosed herein are not limited to whole antibodies as specific binding moieties and may comprise an antibody fragment, such as a binding variable region.

Suitable oligo/polynucleotides for use as specific binding moieties include, but are not limited to, those suitable for hybridizing with a particular target sequence (or target sequence fragment). In particular, the specific binding moiety may be an oligo/polynucleotide suitable for complimentary binding with DNA or RNA. In other examples, the specific binding moiety may be a primer, such as a DNA primer or RNA primer that comprises at least one end suitable for extension via polymerase-mediated extension. For example, the primer may comprise a free hydroxyl group which allows extension of the primer using a polymerase enzyme. Other suitable specific binding moieties include aptamers and/or plasmids.

Members of a specific binding pair suitable for practicing the disclosed embodiments include, but are not limited to, those members that are typically employed in immunoassays and/or hybridization procedures. Particular examples of the specific binding pair may include avidin, biotin, streptavidin, and the like.

Certain probe embodiments concern specific binding moieties that are oligo/polynucleotides capable of binding specifically to a target sequence, or a portion of a target sequence, or antibodies capable of binding to an epitope in a target protein. Exemplary target sequences are provided herein, with certain targets of interest being selected from BCL2 (18q21.3; *e.g.,* GENBANK™ Accession No. NC_000018, complement, nucleotides 58941559 59137593), TOP2A (17q21-q22; *e.g.,* GENBANK™ Accession No. NC_000017, complement, nucleotides 35798321 35827695), ERG (21q22.3; *e.g.,* GENBANK™ Accession No. NC_000021, complement, nucleotides 38675671 38955488), PTEN (10q23.3; *e.g.,* GENBANK™ Accession No. NC_000010, nucleotides 89613175 89716382), p53 (17p13.1; e.g., GENBANK™ Accession No. NC_000017, complement, nucleotides 7512464 7531642), EGFR gene (7p12; *e.g.,* GENBANK™ Accession No. NC_000007, nucleotides 55054219 55242525), or any one of SEQ ID NOs: 8-19.

The oligo/polynucleotide specific binding moiety may have any sequence of nucleotides capable of complementarily binding to the sequences, or portion of the sequences, of these targets, and may comprise at least 5 nucleotides to about 5,000 nucleotides (or such as about 5 to about 4,500 nucleotides, about 10 to about 4,000 nucleotides, about 20 to about 3,500 nucleotides) that bind to the target sequence (or portion of the target sequence). The oligo/polynucleotide specific binding moiety can also comprise a photo-cleavable moiety (such as a photo-cleavable phosphoramidite as disclosed herein). These examplesof the specific binding moiety can further comprise a unique sequence identifier comprising any one of (or more) of the following: an adapter sequence, an alignment sequence, a reagent barcode sequence, with the reagent barcode sequence comprising at least 6 to about 200 nucleotides selected to have a random sequence unique to the particular oligo/polynucleotide specific binding moiety present in the probe, and/or a subject index sequence.

Examplesof a probe comprising an antibody specific binding moiety may have the following exemplary conjugated structures: HER (such as HER1, HER2, HER3, or HER4) rabbit monoclonal antibody - photo-cleavable moiety - unique sequence identifier; PR rabbit monoclonal antibody- photo-cleavable moiety - unique sequence identifier; TOP2a rabbit monoclonal antibody- photo-cleavable moiety - unique sequence identifier; ER rabbit monoclonal antibody-photo-cleavable moiety - unique sequence identifier; Ki-67 rabbit monoclonal antibody- photo-cleavable moiety - unique sequence identifier; p53 antibody-photo-cleavable moiety - unique sequence identifier. The unique sequence identifier may have any suitable sequence, such as one of the exemplary sequences of SEQ ID NOs: 1-3 or 21-23. In other examples, each unique sequence identifier provided in these conjugates may be replaced with a tag sequence.

Examplesof an oligo/polynucleotide sequence that may be used as a specific binding moiety in the disclosed probe include a chromosome 17 alpha satellite repeat sequence, such as SEQ ID NO: 4; an ALU repetitive sequence, such as SEQ ID NO: 5, or a chromosome 7 alpha satellite repeat sequence, such as SEQ ID NO: 20.

Other suitable probes include DNP-tagged probes, such as DNP-TATTTT-DNP-TATTTT, and probes comprising primer sites, such as SEQ ID NO: 6 and/or SEQ ID NO: 7. Other probes include, but are not limited to, those comprising olio/polynucleotide sequences selected from SEQ ID NOs: 34-41. In still other examples, the oligonucleotide specific binding moiety can be of the type disclosed in U.S. Patent Publication No. 2011/0160076 for disclosure related to methods for producing uniquely specific nucleic acid probes. For example, reference is made to U.S. Ser. No. 61/645,247 or International Publication No. WO 2013/167387 for disclosure related to uniquely specific probes. In still other examples, the oligonucleotide specific binding moiety can be of the type disclosed in U.S. Patent No. 8,420,798 or U.S. Patent No 7,869,959 for disclosure related to methods for selecting and/or producing nucleic acid probes.

As a specific example, the probe may comprise a primary antibody, 4B5 (available from Ventana Medical Systems, Inc.), which is capable of recognizing an epitope in the Her2 intracellular domain, such as amino acids 1231-1250 of SEQ ID NO: 10 (GAPPSTFKGTPTAENPEYLG), in the intracellular domain. The probe further comprises a photo-cleavable moiety bound to the antibody. A unique sequence identifier having any one of the sequences described in SEQ ID NOs: 1-3 or 21-23 (or a tag sequence) also is bound to the photo-cleavable moiety.

### 3. Detection Tag

Certain disclosed embodiments of the probe comprise a detection tag. For example, the detection tag can be a unique sequence identifier. In other examples, the detection tag can be a tag sequence.

Each unique sequence identifier has a unique sequence of nucleotides. The sequence can be designed to provide desired information. For example, the number of nucleotides in a unique sequence identifier can range from greater than one nucleotide to at least about 100 nucleotides, more typically from about 3 to about 150 nucleotides, even more typically from about 50 to about 90 nucleotides or from about 18 to about 25 nucleotides. For example, the barcode sequences are at least 18 nucleotides, while tag sequences for NGS (index tags) are typically 4-6 nucleotides. The individual sequence of each unique sequence identifier provides the ability to readily discriminate between a potentially infinite number of unique sequence identifiers present in a sample based on the sequence and/or size of the unique sequence identifier. For example, in a sample comprising mixed cell types (*e.g.,* human and mouse cells), the unique sequence identifiers can be used to distinguish the cell types, as human cells can be detected with a probe comprising a first specific unique sequence identifier, and the mouse cells detected with a second, completely different unique sequence identifier. The unique sequence identifier may be amplified to improve detection using techniques known to those of ordinary skill in the art, such as by using the polymerase chain reaction (PCR). The unique sequence identifiers also may be analyzed for encoded information after a desired target is detected. In some examples, the unique sequence identifier may be sequenced, such as by using next generation sequencing. In other examples, the unique sequence identifier may be identified using array hybridization, such as by using a microarray (*e.g.,* DNA microarray).

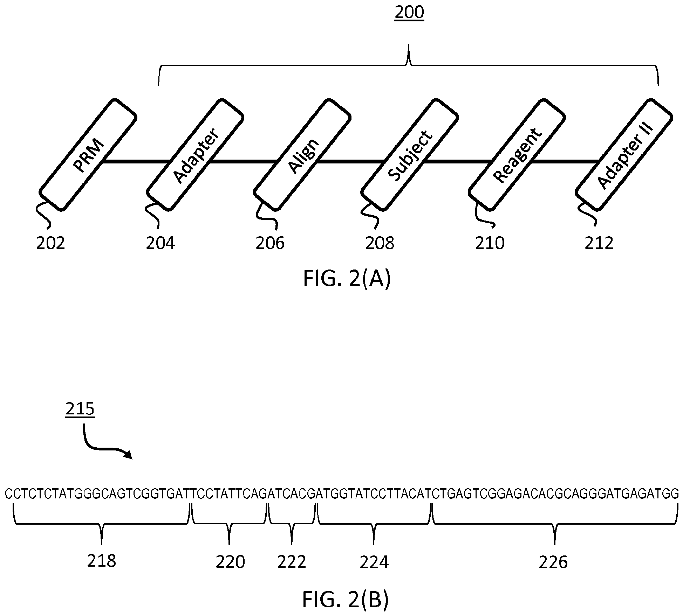

FIG. 2(A) - (B) provides (A) a schematic representation of a detection tag and (B) an illustrative unique sequence identifier contemplated by the present disclosure. Unique sequence identifier 200 can be coupled to photo-reactive moiety (PRM) 202 and comprise one or more adapter sequences (204 and 212), an alignment sequence 206, an optional patient index sequence 208, and a reagent barcode 210.

Adapter sequences 204 and/or 212 typically comprise a short nucleotide sequence that may be manipulated to facilitate analysis of the unique sequence identifier. For example, the adapter sequence may comprise a nucleotide sequence that hybridizes with a corresponding nucleic acid sequence present on a substrate used in a next-generation sequencing technique. The adapter sequence, in some examples, is configured for use in next generation sequencing that utilizes solid phase amplification sequencing techniques and thus uses a solid phase amplification substrate (*e.g.,* flow cell). In other examples, the adapter sequence may be configured for use in emulsion PCR and thereby suitable for binding a microbead substrate. Certain disclosed adapter sequences have a sufficient number of nucleotides to hybridize with the particular next generation sequencing sample (*e.g.,* sequences included on a solid phase amplification flow cell or sequences included on a microbead) being used, such as 1 nucleotide to at least 50 nucleotides, typically about 5 nucleotides to about 40 nucleotides, and more typically from about 15 nucleotides to about 30 nucleotides.

Unique sequence identifier 200 also may comprise one or more alignment sequences, such as sequence 206. Alignment sequences facilitate accurate sequencing by providing a starting point for reading the particular unique sequence identifier. In particular, the alignment sequence may be located near the 5' end of the unique sequence identifier. An alignment sequence comprises a sufficient number of nucleotides to allow accurate alignment for reading such as from about 3 nucleotides to at least about 20 nucleotides, more typically from about 5 to about 15 nucleotides.

Subject index sequences, such as sequence 208, have a particular unique nucleotide sequence that is designed to correlate to and thereby uniquely identify, the particular subject being analyzed. For example, a patient's identity may be equated with a subject index sequence and thus this sequence can be used to identify the unique sequence identifier isolated from a particular sample with a particular patient. For example, including a subject index sequence can be useful when multiple samples are analyzed at one time because a subject can be easily correlated with a particular sample. Subject index sequences comprise a unique nucleotide sequence comprising from about 3 to at least about 10 nucleotides; for example, 5 nucleotides, 6 nucleotides, 7 nucleotides, 8 nucleotides, 9 nucleotides, or 10 nucleotides.

Reagent barcodes, such as reagent bar code 210, provide a sequence of nucleotides that is designed to correlate to each specific binding moiety used to position the probe on targets within the sample (*e.g.,* through hybridization, covalent bonding, antigen/antibody recognition, and the like). For example, a probe comprising an antibody as a specific binding moiety may be bound to a unique sequence identifier comprising a reagent barcode that comprises a sequence of nucleotides unique to (or that correlates to) that particular antibody. In other examples, the reagent barcode can be configured to encode other information, such as lot data, manufacturing data, date information, source information, or combinations thereof. In some examples, the reagent barcode sequence may comprise from about 6 nucleotides to about 200 nucleotides, such as about 6 nucleotides to about 150 nucleotides, or 6 nucleotides to about 100 nucleotides, or about 6 nucleotides to about 50 nucleotides.

As illustrated in FIG. 2(A), unique sequence identifier 200 may comprise an adapter sequence 204 and 212 at each end of the component. Typically, at least one adapter sequence (204 or 212) is bound to photo-reactive moiety 202. Photo-reactive moiety 202 may couple unique sequence identifier 200 to a specific binding moiety (not shown)) or photo-reactive moiety 202 may be a protecting group appending unique sequence identifier 200. Alignment sequence 206 typically is located next to adapter sequence 204 that is bound to photo-cleavable moiety 202, and reagent barcode 210 may be located directly next to, or adjacent to, alignment sequence 206. Subject index sequence 208 may be placed in between alignment sequence 206 and reagent barcode 210. Finally, second adapter sequence 212 typically is located next to the reagent barcode. Second adapter sequence 212 may facilitate linking unique sequence identifier 200 to the specific binding moiety.

A person of ordinary skill in the art will recognize that a suitable unique sequence identifier may comprise all components discussed above, or it may omit certain components. Each component can be positioned in the order illustrated by FIG. 2(A), or different component ordering may be used. For example, a person of ordinary skill in the art would recognize that the particular method of next generation sequencing used may or may not require adapter sequences. Additionally, the subject index sequence is an optional component and therefore may be present or omitted. Particular examplesof the disclosed unique sequence identifier may be generated by a computer algorithm, which is capable of generating a substantially infinite number of unique sequence identifier sequences each having a reagent barcode comprising a different sequence of nucleotides and/or combination of adapter sequences, alignment sequences, subject index sequences, and/or reagent barcode sequences.

An exemplary unique sequence identifier 215 is illustrated in FIG. 2(B). Unique sequence identifier may be coupled to a photo-cleavable linker (not shown) at one end, which is coupled, either directly or indirectly, to an adapter sequence 218. Exemplary unique sequence identifier 215 further comprises an alignment sequence 220, a subject index sequence 222, a reagent barcode sequence 224, and a second adapter sequence 226. Other exemplary unique sequence identifiers include SEQ ID NOs: 1-3 and 21-23.

In other disclosed examples, the detection tag may be a tag sequence. A tag sequence, which is separate and distinct from the unique sequence identifier disclosed herein, may be used to encode information concerning a specific target on a tissue sample (*e.g.,* probe identity, lot data, manufacturing data, date information and source information, and the like). The tag sequence also facilitates binding of a template strand to a sample that is suitable for PCR amplification. For example, the tag sequence may be bound to a protein target of the tissue sample through a specific binding moiety. Solely by way of example, a tag sequence may be bound to an antibody or an oligo/polynucleotide by a photo-cleavable linker, with the antibody or the oligo/polynucleotide serving as the specific binding moiety that binds to a particular target in the sample. The tag sequence has a sufficient number of nucleotides to facilitate hybridization with a template strand, such as from at least one nucleotide to about 25 nucleotides (such as at least one nucleotide to about 22 nucleotides, or at least one nucleotide to about 20 nucleotides, or at least one nucleotide to about 18 nucleotides).

In some examples, the tag sequence may comprise, or be modified to further comprise, one or more flanking primer sequences. For example, the tag sequence comprises a flanking primer sequence before it is bound to the sample. For example, the tag sequence may be bound to the sample and then modified with a primer sequence. In yet other examples, the specific binding moiety itself may be a tag sequence and can comprise, or be modified to comprise, a flanking primer sequence bound to the 5' end of the tag sequence, the 3' end of the tag sequence, or both the 5' and 3' ends of the tag sequence. Each tag sequence may include the same flanking primer sequence or different flanking primer sequences. For example, the same flanking primer sequence may be used for all tag sequences within the sample. This allows simultaneous amplification of all tag sequences present within a sample.

The tag sequence may be combined with a template sequence prior to analysis in some embodiments. Template sequences, which generally comprise longer strands of nucleotides, are used to facilitate amplification techniques, such as PCR. The template sequence may be, for example, an oligonucleotide having a length suitable for quantitative PCR (*e.g.,* an oligonucleotide having from about 50 nucleotides to about 100 nucleotides, or from about 60 nucleotides to about 90 nucleotides, or from about 70 nucleotides to about 80 nucleotides). In some embodiments, the template sequence can be configured to specifically hybridize (*e.g.,* through sequence complementarity) with the tag sequence to form a stable duplex. A stable duplex, as disclosed herein, typically has a melting temperature (Tₘ) of greater than 60 °C. In some examples, this duplex can be transferred into solution for further analysis via cleavage of a photo-cleavable moiety that binds the tag sequence to the specific binding moiety. Using this tag-template duplex probe avoids the need to attach one or more additional recognition biomolecules/peptide sequences directly to the template sequence, which typically is used in the art. For example, immuno-PCR methods used in the art directly conjugate a template strand to an antibody that will be bound to a target in a sample. These methods however can result in reduced antibody-antigen binding as the large template strand can often interfere with this specific binding. As disclosed herein, the template sequence may be added after the specific binding moiety is bound, which thereby reduces and preferably substantially eliminates specific binding interferences that are induced by large template sequences.

In other disclosed examples, the tag sequence may be modified to include one or more peptide nucleic acids and/or locked nucleic acids. A peptide nucleic acid (PNA) is an artificially synthesized polymer comprising a backbone of N-(2-aminoethyl)-glycine units linked by peptide bonds to which purine and pyrimidine bases are linked. PNAs may be used to improve hybridization with a template sequence (or a tag sequence) as PNAs do not include charged phosphate groups typically found in DNA and therefore exhibit stronger hybridization with the template sequence than would a natural DNA strand. Locked nucleic acids (LNA) comprise one or more modified nucleotides that comprise a bridge in the ribose moiety of the nucleotide that locks the ribose in the 3'-endo conformation. This conformation enhances base stacking and thereby increases the ability of the tag sequence (or a template sequence) to hybridize with the template sequence (or tag sequence). Accordingly, using either PNA or LNA in the tag sequence can reduce background signals produced by non-specific binding of template sequences and further strengthen the duplex formed between the tag sequence and the template sequence.

The disclosed detection tags are not limited in the types of nucleotides that each detection tag may comprise. For example, detection tags like unique sequence identifiers and tag sequences can comprise one or more naturally occurring nucleotides, synthesized nucleotides, chemically modified nucleotides, and combinations thereof. In some examples, the unique sequence identifier and/or the tag sequence can comprise one or more peptide nucleic acid or locked nucleic acid components.

The base ratio of the unique sequence identifier or tag sequence may be configured to impart chemical and/or physical modifications to the structure of the unique sequence identifier and/or tag sequence. For example, the base composition present in the tag sequence may be configured to influence the thermal melting temperature of this component upon hybridization with a template sequence. That is, the base ratio of the tag sequence may be modified to promote hybridization with a template sequence, or it may be modified to reduce tag/template hybridization. The base ratio of the template also may be modified in a similar manner. Such manipulations are not limited to the detection tag and also may be applied to specific binding moieties such as an oligo/polynucleotide specific binding moiety. These specific binding moieties can be configured to have certain base ratios that promote specific or non-specific binding with a target sequence.

The base ratio of certain probe components also can be configured to convey information about the probe. For example, the base ratio of a unique sequence identifier and/or tag sequence can be configured to have a certain base ratio that can be used to represent that particular unique sequence identifier and/or tag sequence. Once a probe comprising this particular base ratio is deposited and then isolated, the particular base ratio can be used to track the origin of that particular isolated unique sequence identifier and/or tag on the original sample.

The base ratio also may be configured to facilitate isolation of the detection tag. For example, a detection tag may be isolated for array analysis and/or next generation sequencing. In these examples, the base ratio of the substrate to which the detection tag binds during the array and/or next generation sequencing analysis can be configured to specifically hybridize with the complementary base ratio present in the detection tag.

The disclosed probes also may have one or more linking facilitators that facilitate linking two or more probe components. Suitable linking facilitators include, but are not limited to, functional groups typically used in the art to promote forming one or more amide bonds, ester bonds, and the like. The linking facilitator may be a chemical compound that promotes linkage between one or more probe components, but is not incorporated into the probe. Exemplary linking facilitators include, but are not limited to, carbodiimide, NHS esters, imidoesters, maleimide, haloacetyls, pyridyldisulfides, hydrazides, alkoxyamines, diazirines, aryl azides, isocyanates, and the like.

Illustrative detection tags may be selected to be uniquely distinct tags as disclosed by U.S. Patent Publ. No. 2012/0070862 for disclosure related to methods for producing uniquely distinct nucleic acid tags.

### B. Making the Probe

The probes described herein may be made by any method now known or hereafter developed. The method for making the probe may be selected based, at least in part, on the particular components being included in the probe. The methods disclosed herein may be altered and modified to accommodate different components than those expressly disclosed herein as will be understood by a person of ordinary skill in the art.

Probes comprising specific binding moieties, a photo-cleavable moiety, and a detection tag may be made using coupling conditions known in the art. For example, if the specific binding moiety is an antibody, the method may comprise coupling an antibody to the photo-cleavable moiety through a functional group of the antibody (*e.g.,* amine, thiol, carbonyl, etc.). The photo-cleavable moiety may include (or be modified to include) a reactive functional group, such as an NHS ester, maleimide, etc. These two components can be coupled using coupling reagents typically used in the art, such as EDCI, DCC, EDC, etc. Scheme 2 illustrates an examplewherein photo-cleavable moiety **300** is coupled with an NHS group to provide activated NHS ester **302.** Ester 302 is then reacted with an antibody to provide an antibody labeled with the photo-cleavable moiety (conjugate **304**). A person of ordinary skill in the art will recognize that this method can be suitably modified for any of the particular photo-cleavable moieties disclosed herein.

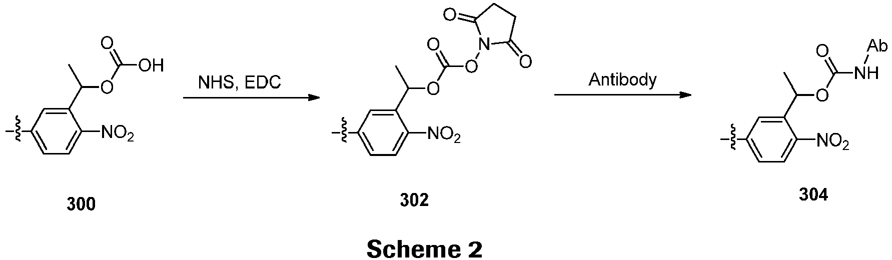

In some embodiments using an oligo/polynucleotide as the specific binding moiety, the photo-cleavable moiety may be chemically integrated into the oligo/polynucleotide by using phosphoramidite reagents comprising the photo-cleavable functional group. An oligonucleotide synthesizer may be used to integrate the photo-cleavable phosphoramidite. An exemplary embodiment is illustrated below in Scheme 4. As illustrated in Scheme 3, phosphoramidite reagent **400,** which comprises a photo-cleavable moiety, is coupled to nucleotide **402** of an oligo/polynucleotide to provide labeled oligo/polynucleotide **404,** which is then oxidized to corresponding phosphate **406.** The cyanoester moiety is then removed to provide unprotected phosphate **408.** As previously indicated, the photo-cleavable moiety may comprise other functional groups that may be further manipulated to be coupled with the tag sequence, unique sequence identifier, or other probe components disclosed herein. As illustrated in Scheme 3, and provided solely as an example, photo-cleavable moiety **400** further comprises a protected amine group -NH-PG, where PG is an amine protecting group. The protecting group may be removed to allow coupling the photo-cleavable moiety to one of more of the probe components disclosed herein.

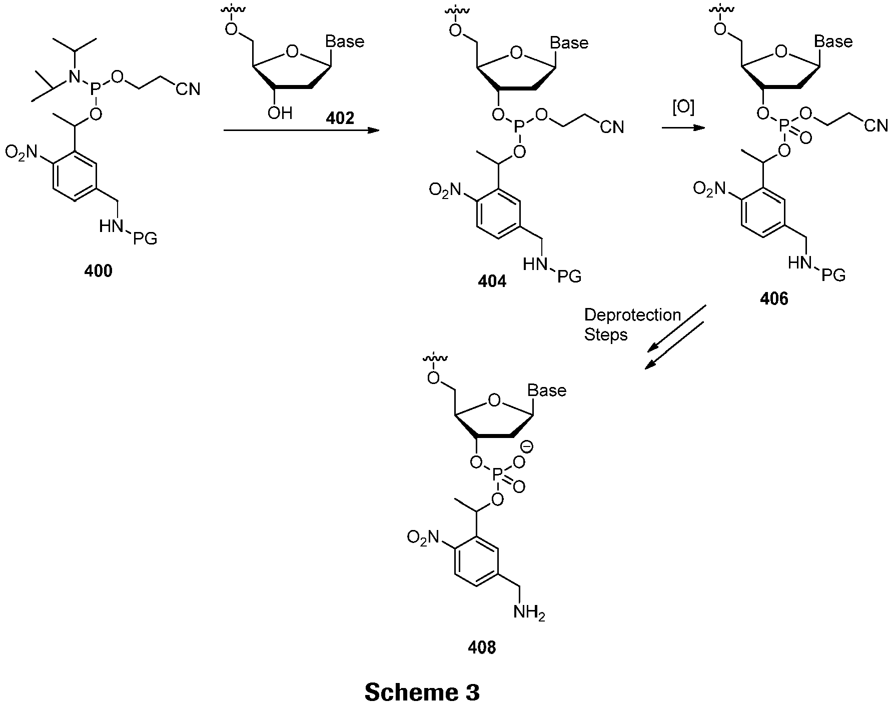

In some examples, the specific binding moiety may be bound to a detectable label using coupling conditions known in the art, such as Fc-specific coupling conditions, non-specific coupling conditions, and the like. In some examples, the detectable label may be coupled to an antibody through a thiol moiety of the antibody. In some examples, a detectable label may be coupled to an antibody through an aldehyde moiety generated on the antibody.

The detectable label also may be coupled to the specific binding moiety through a linker. Suitable linkers include, but are not limited to aliphatic linkers, alkylene oxide linkers, disulfide-containing linkers, commercially available linkers, polymeric linkers, and the like. One particular exampleof a class of linkers for use with disclosed probe components is a polyalkyleneglycol linker having the general structure shown below: 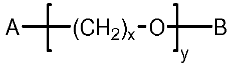 wherein A and B include similar or different reactive groups suitable for reacting with the probe components disclosed herein (*e.g.,* carbonyl-reactive groups, amine-reactive group, thiol-reactive group, and the like, and that are suitable for reacting with a detectable label or a specific binding moiety), x is an integer from 2 to 10 (such as 2, 3 or 4), and y is an integer from 1 to 50, for example, from 2 to 30 such as from 3 to 20 or from 4 to 12. Exemplary linkers, such as PEG linkers, and methods for coupling a specific binding moiety to a detectable label using such linkers, are provided in U.S. Patent No. 7,695,929. These disclosed linkers also may be used to couple various components of the probe other than just coupling the detectable label and the specific binding moiety. For example, a linker may be used to couple the specific binding moiety to a detection tag and/or a detectable label, or one or more linkers may be used to couple the photo-cleavable moiety with one or more of the other probe components disclosed herein. Linkers may be cleaved using any of the primary and/or secondary cleavage techniques disclosed herein.

Some aspectsof the disclosed method concern using an irradiation event that photochemically induces formation of a compound or radical species that can cleave a chemically-cleavable linker. The compound or radical species formed by the irradiation event should be capable of interacting with one or more functional groups of a linker present in the probe (or a different probe species) in a manner that causes cleavage of the linker. Solely by way of example, a probe moiety may comprise a photo-cleavable moiety that can be cleaved by an irradiation event to provide a species, such as a radical species, that can then subsequently cleave another non-photo-labile linker in the probe. In other examples, the light-induced cleavage event can promote cleavage of a photo-cleavable moiety that comprises one or more moieties that can be cleaved and/or released from the photo-cleavable moiety to form a separate chemical species capable of interacting with a linker species present in the probe (or a different probe).

### C. Probe Modifications

One or more of the probe components disclosed herein may be configured to be more or less robust under certain conditions. For example, a linker and/or a photo-cleavable moiety present in the probe may be photochemically and/or chemically induced to become a species that is more or less robust after such photochemical and/or chemical manipulation. In some examples, specifically bound probes can be configured to have a linker and/or photo-cleavable moiety that become(s) more robust before or after being bound to the sample. These probes will therefore comprise detection tags that are not cleaved upon exposure to light of a particular wavelength. Probes that do not comprise the more robust photo-cleavable moiety are cleaved. A secondary cleavage event may then be implemented to cleave the specifically bound probes, such as by using light of a different wavelength, longer time periods of irradiation, more intense light sources, thermal cleavage, chemical cleavage, enzymatic cleavage, or combinations thereof.

Such techniques also may be applied to components of the probe that are not photochemically labile, but rather are chemically labile. For example, a linker (other than a photo-cleavable moiety) present in the probe may be configured to be more robust after the probe is bound to the sample (*e.g.,* through chemical modification of one or more functional group of the linker). The robust linker is then not susceptible to chemical cleavage under conditions where it would otherwise normally be cleaved. Manipulation and/or analysis of the sample may then be conducted using such conditions that do not chemically alter the robust linkers. The robust linker may then be subsequently cleaved (such as chemically cleaved) using conditions selected particularly for the robust linker (*e.g.,* conditions that are more basic and/or acidic than typical physiologically desired).

In some examples, the linker and/or photo-cleavable moiety may be configured to be so robust as to be impervious to a cleavage event (*e.g.,* photo-chemical and/or chemical cleavage) used for an initial analysis. Accordingly, a sample comprising probes having these robust components can be stored without degrading. Such samples can then be analyzed at a later time.

The probe also may be configured to have components that facilitate cell wall permeation. For example, the probe is specifically designed to permeate a cell wall to facilitate analysis of cell components (*e.g.,* the nucleus, mitochondria, ribosomes, and the like). Such probes can be configured to comprise one or more components that promote lipid-solubility of the probe (*e.g.,* aliphatic linkers, aliphatic functional groups on a photo-cleavable moiety or other probe component).

In other disclosed examples, the hydrophobicity or hydrophilicity of the probe can be configured to attain solubility (or insolubility) in certain process solutions used for sample analysis. For example, the probe can be configured to be more or less hydrophilic, and therefore more or less soluble in developing solutions used with photoresist materials. A person of ordinary skill in the art will recognize that the type of developing solution that is being used will govern whether the probe should be configured to be more or less hydrophobic (or hydrophilic).

The hydrophobicity and/or hydrophilicity of the probe can also be configured to promote solubility in process solutions, such as the elution solutions used to isolate detection tags after a cleavage event. For example, if an aqueous buffer is used as the elution solution, the portion of the probe that will end up being cleaved (*e.g.,* detection tag) can be configured to be hydrophilic so as to promote its elution from the sample. If the cleaved portion is not to be isolated, the probe can be configured (prior to or after being added to the sample) to be less hydrophilic so that it does not elute with aqueous elution buffers.

### IV. Targets

Embodiments of the probe and method disclosed herein may be used to identify and/or quantify many different biological targets. Throughout this disclosure when reference is made to a target protein, it is understood that any polynucleotides associated with that protein can also be used as a target. The target may be a protein or nucleic acid molecule from a pathogen, such as a virus, bacteria, or intracellular parasite, such as from a viral genome. For example, a target protein may be produced from a target polynucleotide associated with (*e.g.,* correlated with, causally implicated in, etc.) a disease. In certain disclosed examples, the target (or targets) of interest may be a particular nucleic acid sequence that may comprise a genetic aberration, such as a single nucleotide polymorphism, promoter methylation, mRNA expression, siRNA, a particular copy number change, a mutation, a certain expression level, a rearrangement, or combination thereof. In some examples, the targets are soluble proteins obtained from biological samples, such as serum, plasma, and/or urine. For example, the disclosed method may be used to detect and quantify DNA, RNA, and proteins of the same target (*e.g.,* HER2) simultaneously from the same sample (*e.g.,* from the same tissue section).

The disclosed method may be used to detect microRNA (miRNA or miR). MicroRNAs are small, non-coding RNAs that negatively regulate gene expression, such as by translation repression. For example, miR-205 regulates epithelial to mesenchymal transition (EMT), a process that facilitates tissue remodeling during embryonic development. However, EMT also is an early step in tumor metastasis. Down-regulation of microRNAs, such as miR-205, may be an important step in tumor progression. For instance, expression of miR-205 is down-regulated or lost in some breast cancers. MiR-205 also can be used to stratify squamous cell and non-small cell lung carcinomas (J. Clin. Oncol., 2009, 27(12):2030-7). Other microRNAs have been found to modulate angiogenic signaling cascades. Down-regulation of miR-126, for instance, may exacerbate cancer progression through angiogenesis and increased inflammation. Thus, microRNA expression levels may be indicative of a disease state.

A target nucleic acid sequence can vary substantially in size. Without limitation, the nucleic acid sequence can have a variable number of nucleic acid residues. For example a target nucleic acid sequence can have at least about 10 nucleic acid residues, or at least about 20, 30, 50, 100, 150, 500, 1000 residues. Similarly, a target polypeptide can vary substantially in size. The target polypeptides typically include at least one epitope that binds to a peptide specific antibody, or fragment thereof. In some examplesthat polypeptide can include at least two epitopes that bind to a peptide specific antibody, or fragment thereof.

In specific, non-limiting examples, a target protein is produced by a target nucleic acid sequence (*e.g.,* genomic target nucleic acid sequence) associated with a neoplasm (for example, a cancer). Numerous chromosome abnormalities (including translocations and other rearrangements, amplification or deletion) have been identified in neoplastic cells, especially in cancer cells, such as B cell and T cell leukemias, lymphomas, breast cancer, colon cancer, neurological cancers and the like. In some examples, therefore, at least a portion of the target molecule is produced by a nucleic acid sequence (*e.g.,* genomic target nucleic acid sequence) amplified or deleted in at least a subset of cells in a sample. In one example, the genomic target nucleic acid sequence is selected to include a gene (*e.g.,* an oncogene) that is reduplicated in one or more malignancies (*e.g.,* a human malignancy). Oncogenes are known to be responsible for several human malignancies. For example, chromosomal rearrangements involving the SYT gene located in the breakpoint region of chromosome 18q11.2 are common among synovial sarcoma soft tissue tumors.

For example, HER2, also known as c-erbB2 or HER2/neu, is a gene that plays a role in the regulation of cell growth (a representative human HER2 genomic sequence is provided at GENBANK™ Accession No. NC_000017, nucleotides 35097919-35138441). The gene codes for a 185 kd transmembrane cell surface receptor that is a member of the tyrosine kinase family. HER2 is amplified in human breast, ovarian, and other cancers; therefore, a HER2 gene (or a region of chromosome 17 that includes the HER2 gene) can be used as a genomic target nucleic acid sequence. Other breast cancer relevant proteins include the estrogen receptor (ER) and progesterone receptor (PR).

In other examples, a target protein produced from a nucleic acid sequence (*e.g.,* genomic target nucleic acid sequence) is selected that is a tumor suppressor gene that is deleted (lost) in malignant cells. For example, the p16 region (including D9S1749, D9S1747, p16(INK4A), p14(ARF), D9S1748, p15(INK4B), and D9S1752) located on chromosome 9p21 is deleted in certain bladder cancers. Chromosomal deletions involving the distal region of the short arm of chromosome 1 (that encompasses, for example, SHGC57243, TP73, EGFL3, ABL2, ANGPTL1, and SHGC-1322), and the pericentromeric region (*e.g.,* 19p13-19q13) of chromosome 19 (that encompasses, for example, MAN2B1, ZNF443, ZNF44, CRX, GLTSCR2, and GLTSCR1) are characteristic molecular features of certain types of solid tumors of the central nervous system.

The aforementioned examples are provided solely for purpose of illustration and are not intended to be limiting. Numerous other cytogenetic abnormalities that correlate with neoplastic transformation and/or growth are known to those of ordinary skill in the art. Target proteins that are produced by nucleic acid sequences (*e.g.,* genomic target nucleic acid sequences), which have been correlated with neoplastic transformation and which are useful in the disclosed methods, also include (GENBANK™ RefSeq Accession No. in parentheses) the EGFR gene (7p12; *e.g.,* NC_000007, nucleotides 55054219-55242525), the C-MYC gene (8q24.21; *e.g.,* NC_000008, nucleotides 128817498-128822856), D5S271 (5p15.2), lipoprotein lipase (LPL) gene (8p22; *e.g.,* NC_000008, nucleotides 19841058-19869049), RB1 (13q14; *e.g.,* NC_000013, nucleotides 47775912-47954023), p53 (17p13.1; *e.g.,* NC_000017, complement, nucleotides 7512464-7531642), N-MYC (2p24; *e.g.,* NC_000002, complement, nucleotides 151835231-151854620), CHOP (12q13; *e.g.,* NC_000012, complement, nucleotides 56196638-56200567), FUS (16p11.2; *e.g.,* NC_000016, nucleotides 31098954-31110601), FKHR (13p14; *e.g.,* NC_000013, complement, nucleotides 40027817-40138734), as well as, for example: ALK (2p23; *e.g.,* NC_000002, complement, nucleotides 29269144-29997936), Ig heavy chain, CCND1 (11q13; *e.g.,* NC_000011, nucleotides 69165054-69178423), BCL2 (18q21.3; *e.g.,* NC_000018, complement, nucleotides 58941559-59137593), BCL6 (3q27; *e.g.,* NC_000003, complement, nucleotides 188921859-188946169), MALF1, AP1 (1p32-p31; *e.g.,* NC_000001, complement, nucleotides 59019051-59022373), TOP2A (17q21-q22; *e.g.,* NC_000017, complement, nucleotides 35798321-35827695), TMPRSS (21q22.3; *e.g.,* NC_000021, complement, nucleotides 41758351-41801948), ERG (21q22.3; *e.g.,* NC_000021, complement, nucleotides 38675671-38955488); ETV1 (7p21.3; *e.g.,* NC_000007, complement, nucleotides 13897379-13995289), EWS (22q12.2; *e.g.,* NC_000022, nucleotides 27994271-28026505); FLI1 (11q24.1-q24.3; *e.g.,* NC_000011, nucleotides 128069199-128187521), PAX3 (2q35-q37; *e.g.,* NC_000002, complement, nucleotides 222772851-222871944), PAX7 (1p36.2-p36.12; *e.g.,* NC_000001, nucleotides 18830087-18935219), PTEN (10q23.3; *e.g.,* NC_000010, nucleotides 89613175-89716382), AKT2 (19q13.1-q13.2; *e.g.,* NC_000019, complement, nucleotides 45431556-45483036), MYCL1 (1p34.2; *e.g.,* NC_000001, complement, nucleotides 40133685-40140274), REL (2p13-p12; *e.g.,* NC_000002, nucleotides 60962256-61003682) and CSF1R (5q33-q35; *e.g.,* NC_000005, complement, nucleotides 149413051-149473128).

In other examples, a target protein is selected from a virus or other microorganism associated with a disease or condition. Detection of the virus- or microorganism-derived target nucleic acid sequence (*e.g.,* genomic target nucleic acid sequence) in a cell or tissue sample is indicative of the presence of the organism. For example, the target peptide, polypeptide or protein can be selected from the genome of an oncogenic or pathogenic virus, a bacterium or an intracellular parasite (such as *Plasmodium falciparum* and other *Plasmodium* species, *Leishmania* (sp.), *Cryptosporidium parvum, Entamoeba histolytica,* and *Giardia lamblia,* as well as *Toxoplasma, Eimeria, Theileria,* and *Babesia* species).

In some examples, the target protein is produced from a nucleic acid sequence (*e.g.,* genomic target nucleic acid sequence) from a viral genome. Exemplary viruses and corresponding genomic sequences (GENBANK™ RefSeq Accession No. in parentheses) include human adenovirus A (NC_001460), human adenovirus B (NC_004001), human adenovirus C (NC_001405), human adenovirus D (NC_002067), human adenovirus E (NC_003266), human adenovirus F (NC_001454), human astrovirus (NC_001943), human BK polyomavirus (V01109; GI:60851) human bocavirus (NC_007455), human coronavirus 229E (NC_002645), human coronavirus HKU1 (NC_006577), human coronavirus NL63 (NC_005831), human coronavirus OC43 (NC_005147), human enterovirus A (NC_001612), human enterovirus B (NC_001472), human enterovirus C (NC_001428), human enterovirus D (NC_001430), human erythrovirus V9 (NC_004295), human foamy virus (NC_001736), human herpesvirus 1 (Herpes simplex virus type 1) (NC_001806), human herpesvirus 2 (Herpes simplex virus type 2) (NC_001798), human herpesvirus 3 (Varicella zoster virus) (NC_001348), human herpesvirus 4 type 1 (Epstein-Barr virus type 1) (NC_007605), human herpesvirus 4 type 2 (Epstein-Barr virus type 2) (NC_009334), human herpesvirus 5 strain AD169 (NC_001347), human herpesvirus 5 strain Merlin Strain (NC_006273), human herpesvirus 6A (NC_001664), human herpesvirus 6B (NC_000898), human herpesvirus 7 (NC_001716), human herpesvirus 8 type M (NC_003409), human herpesvirus 8 type P (NC_009333), human immunodeficiency virus 1 (NC_001802), human immunodeficiency virus 2 (NC_001722), human metapneumovirus (NC_004148), human papillomavirus-1 (NC_001356), human papillomavirus-18 (NC_001357), human papillomavirus-2 (NC_001352), human papillomavirus-54 (NC_001676), human papillomavirus-61 (NC_001694), human papillomavirus-cand90 (NC_004104), human papillomavirus RTRX7 (NC_004761), human papillomavirus type 10 (NC_001576), human papillomavirus type 101 (NC_008189), human papillomavirus type 103 (NC_008188), human papillomavirus type 107 (NC_009239), human papillomavirus type 16 (NC_001526), human papillomavirus type 24 (NC_001683), human papillomavirus type 26 (NC_001583), human papillomavirus type 32 (NC_001586), human papillomavirus type 34 (NC_001587), human papillomavirus type 4 (NC_001457), human papillomavirus type 41 (NC_001354), human papillomavirus type 48 (NC_001690), human papillomavirus type 49 (NC_001591), human papillomavirus type 5 (NC_001531), human papillomavirus type 50 (NC_001691), human papillomavirus type 53 (NC_001593), human papillomavirus type 60 (NC_001693), human papillomavirus type 63 (NC_001458), human papillomavirus type 6b (NC_001355), human papillomavirus type 7 (NC_001595), human papillomavirus type 71 (NC_002644), human papillomavirus type 9 (NC_001596), human papillomavirus type 92 (NC_004500), human papillomavirus type 96 (NC_005134), human parainfluenza virus 1 (NC_003461), human parainfluenza virus 2 (NC_003443), human parainfluenza virus 3 (NC_001796), human parechovirus (NC_001897), human parvovirus 4 (NC_007018), human parvovirus B19 (NC_000883), human respiratory syncytial virus (NC_001781), human rhinovirus A (NC_001617), human rhinovirus B (NC_001490), human spumaretrovirus (NC_001795), human T-lymphotropic virus 1 (NC_001436), human T-lymphotropic virus 2 (NC_001488).

In certain examples, the target protein is produced from a nucleic acid sequence (*e.g.,* genomic target nucleic acid sequence) from an oncogenic virus, such as Epstein-Barr Virus (EBV) or a Human Papilloma Virus (HPV, *e.g.,* HPV16, HPV18). In other examples, the target protein produced from a nucleic acid sequence (*e.g.,* genomic target nucleic acid sequence) is from a pathogenic virus, such as a Respiratory Syncytial Virus, a Hepatitis Virus (*e.g.,* Hepatitis C Virus), a Coronavirus (*e.g.,* SARS virus), an Adenovirus, a Polyomavirus, a Cytomegalovirus (CMV), or a Herpes Simplex Virus (HSV).

### V. Method of Use

Disclosed herein are several embodiments of a method of using probe embodiments described herein, as well as novel methods for isolating and/or analyzing a tissue sample, or other cellular specimen, while maintaining tissue context and integrity. In particular disclosed embodiments, the method may further comprise employing subsequent analytical techniques (*e.g.,* PCR, next generation sequencing, array hybridization, etc.) to provide improved multiplexing capabilities, increased sensitivity, relative quantification, and/or improved resolution.

### A. General Method

In particular disclosed embodiments, the method comprises providing a tissue sample, comprising one or more targets of interest. The tissue sample is contacted with a probe using conditions sufficient to facilitate binding of the specific binding moiety to the target. In some embodiments, the probe may comprise a specific binding moiety, a photo-cleavable moiety, and a detection tag. In other examples, the probe may not comprise a photo-cleavable moiety; rather, the sample is exposed to a probe and a composition comprising a separate component comprising a photo-cleavable moiety. The method may further comprise exposing particular selected sections of the biological sample to light having a wavelength and intensity suitable to cleave the photo-cleavable moiety thereby freeing the detection tag. The detection tag may then be detected. The presence and/or location of target may be determined by detecting (*e.g.,* visually and/or with instrumentation) a detectable label. The identity of the target also may be determined by photochemically isolating portions of the probe, such as a tag sequence, tag-template duplex, and/or a unique sequence identifier. The method may have one or more additional steps, one or more steps may be performed manually, or certain or all of the steps may be performed by an automated system.

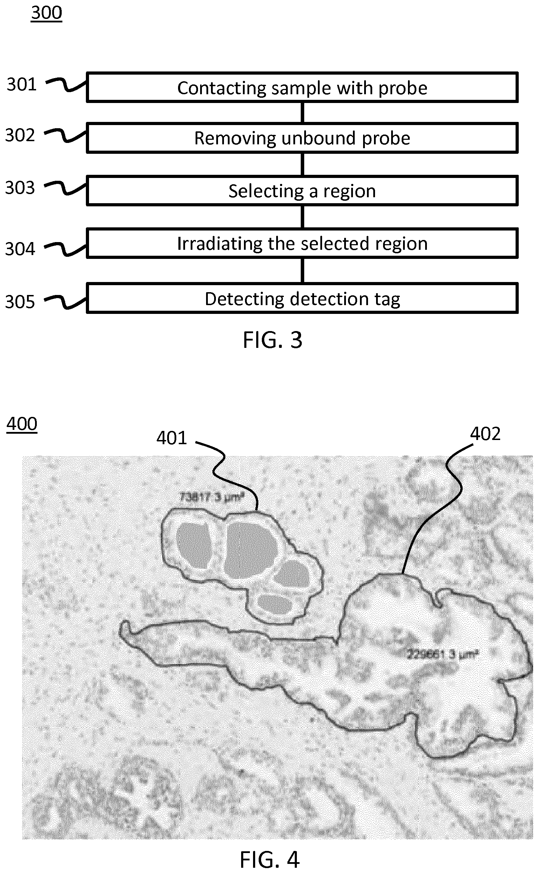

FIG. 3 is a schematic that illustrates a method for performing contextual molecular diagnostics 300 showing a step of contacting a sample with a probe 301, removing unbound probe 302, selecting a region 303, irradiating the selected region 304, and detecting the detection tag 305.

FIG. 4 is a photomicrograph showing that a first region 401 and a second region 402 can be selected. In particular, an image of the sample can be acquired and stored as a digital file. The digital file can be manipulated by a user or through the application of image analysis algorithms to select one or more regions of interest. These selected regions can then been irradiated. For example, the regions can be irradiated concurrently or sequentially. The detection tags coming from the first and second region can either be detected concurrently or sequentially.

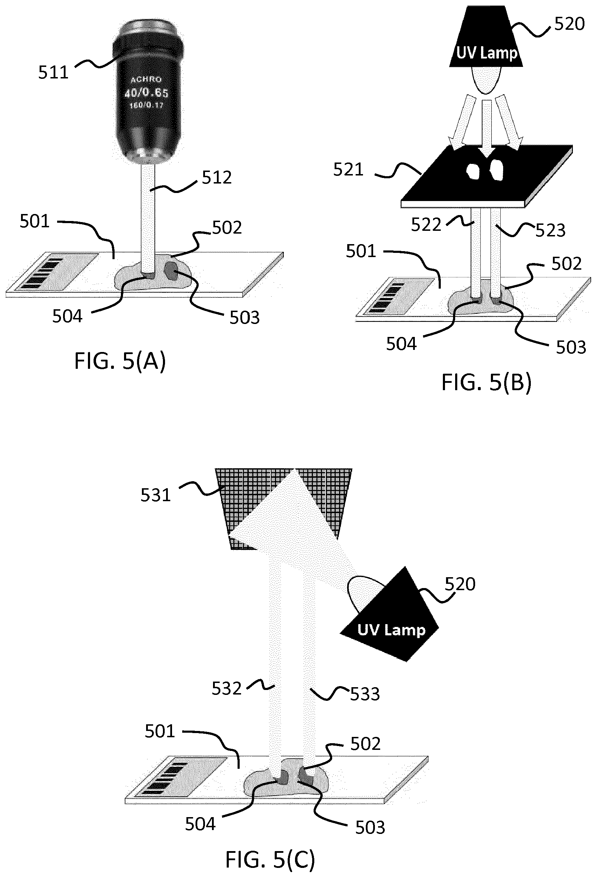

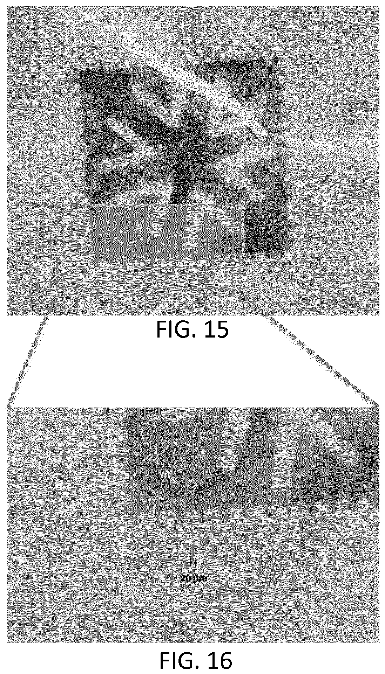

FIG. 5(A) - (C) are schematic drawings showing several approaches to irradiating a first selected region 504 and/or a second selected region 503 of a sample 502 disposed on a slide 501. Referring now to FIG. 5(A), a microscope objective 511 can be used to view the sample so that a region of interest can be selected. The microscope objective can also be used to direct light irradiating the sample. For instance, region of interest 504 may be selected in the view of objective 511. Light from an appropriate source can then be directed through objective 511 to region region 504. Another similar approach would be to combine this with raster scanning of the specimen to illuminate the entire selected region. Raster scanning would be achieved by stage translation in the X and Y directions or deflection of the light with rotating mirrors. The intensity of the light beam can be modulated (*e.g.,* UV LED with electronic control of output intensity) as the beam position is scanned across a rectangular region of the specimen to create the illuminated region, or the beam can always be on and the raster scan is restricted to only the boundaries of the selected region. FIG. 5(C) shows a schematic depiction of a digital micromirror device (DMD) being used to selectively irradiate portions of the slide. This aspect was used to make the elaborate and high resolution design shown in FIGS. 15 and 16.

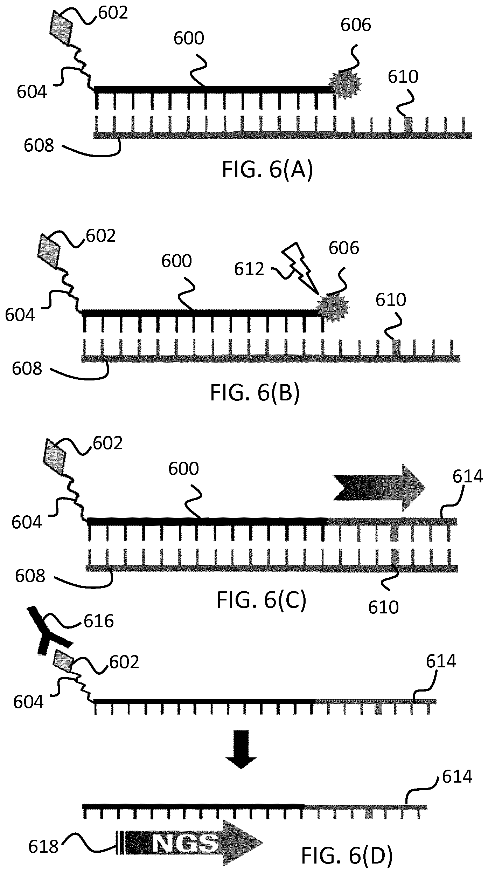

FIG. 6(A) - (D) is a schematic depicting an approach to using photochemistry to facilitate an *in situ* polymerase extension reaction wherein polymerase enzymes may be added to tissue for *in situ* polymerase-mediated primer extension. Referring now to FIG. 6(A), oligonucleotide probe 600 is added to a sample having a target sequence 608. Probe 600 includes a photo-reactive moiety (PRM) 606 and optionally a linker 604 and label 602. FIG. 6(B) shows light 612 irradiating PRM 606 so as to enable extension of probe using a polymerase enzyme. In some examples, target 608 includes a mutation 610, wherein extension captures mutation information in probe extension 614. In some examples, an antibody 616 can be used to purify probe extension. Antibody 616 could be bound to a bead, plate, in gel, etc. and could be selected to bind label 602. FIG. 6(D) shows an arrow 618 representing that probe extension 614 may be analyzed by an appropriate technology, such as next generation sequencing. The analyzed sequence may contain mutation information, a tag sequence (not shown), and the probe sequence.

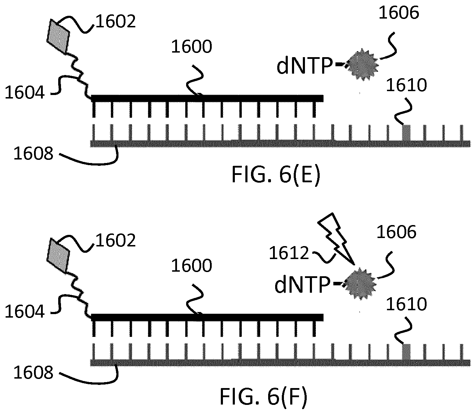

FIG. 6(E) - (F) is analogous to FIG. 6(A) - (B) and can be used replaceably in the workflow of FIG. 6(A) - (D). According to this embodiment, a probe 1600 and a separate photo-activatable moiety 1606 are used for the photo-activated selection. According to this example, photo-activatable moiety 1606 includes 1 or more of the 4-deoxynucleoside triphosphates (i.e. dNTP), rather than an oligonucleotide probe as shown in FIG. 6(A). Oligonucleotide probe 1600 is added to a sample having a target sequence 1608. Probe 1600 optionally includes a linker 1604 and label 1602. FIG. 6(F) shows light 1612 irradiating photo-activatable moiety 1606 so as to enable extension of probe using a polymerase enzyme. In some examples, target 1608 includes a mutation 1610, wherein extension captures mutation information in the probe extension (as shown in FIG. 6(C)). FIG. 6(D) shows an arrow 618 representing that any probe extension may be analyzed by an appropriate technology, such as next generation sequencing. The analyzed sequence may contain mutation information, a tag sequence (not shown), and the probe sequence.

The extent of polymerase chain extension that can be obtained using embodiments of the disclosed method ranges from hundreds of extended nucleotides to thousands of extended nucleotides. For example, the number of nucleotides added to the initial probe can range from about 10 nucleotides to about 2000 nucleotides (such as about 50 nucleotides to about 1500 nucleotides, or about 100 nucleotides to about 1000 nucleotides, or about 200 nucleotides to about 400 nucleotides). The extent of the chain extension can be determined using one or more reverse primers that have some degree of complementarity (*e.g.,* about 70% to about 99% complementarity) to certain regions of the extended probe. Polymerase-mediated chain extension may be carried out using a range of probe concentrations (*e.g.,* 100 ng/mL to about 100 µg/mL, such as about 10 ng/mL to about 10 µg/mL), and can be carried out for any suitable amount of time, such as from about 20 minutes to about 90 minutes.

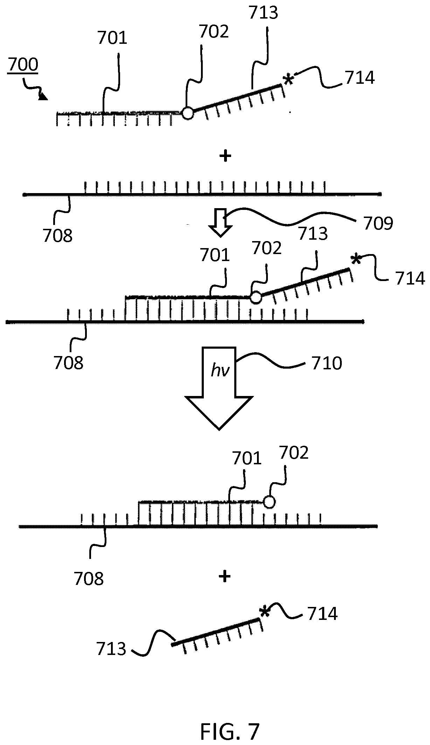

FIG. 7 similarly shows a detection motif within the scope of the present application. In particular, a probe 700 can be brought into contact with a sample having a target 708. Probe 700 comprises specific binding oligonucleotide 701, a photo-cleavable moiety 702, and a detection tag 713. Probe 700 may optionally include a label 714, for example to aid in purification. Contacting probe 700 and sample, under the appropriate conditions results in hybridization of probe 700 to target 708 as indicated by an arrow 709. Subsequent to removal of unbound probe, irradiating a selected portion of the sample with light, as indicated by arrow 710, cleaves photo-cleavable moiety 702, freeing detection tag 713. Detection tag 713 can then be removed from the sample (*e.g.,* through pipetting, contact transfer, absorption, rinsing, microfluidics, rinsing, or other appropriate method), optionally purified (*e.g.,* through use of optional label 714), and analyzed by an appropriate technology (*e.g.,* an array, NGS, PCR, etc.).

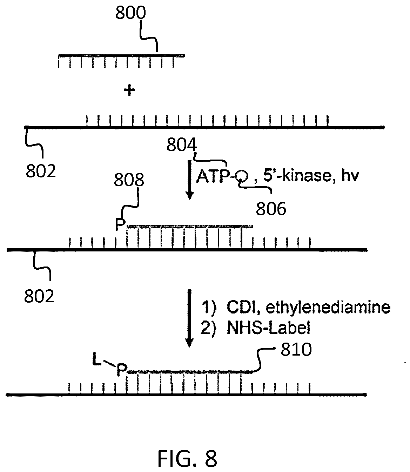

Referring now to FIG. 8, shown is a schematic of a particular embodiment. Oligo/polynucleotide 800 is combined with sample 802. Probe 800 associates with a complimentary sequence sample 802. A composition comprising caged ATP 804 caged with photo-cleavable moiety 806 is added to the sample 802. Irradiation with light liberates caged ATP 804. Exposure to a suitable kinase then phosphorylates probe 800 at the 5' end to provide phosphorylated probe 808. Subsequent modification of probe 808 may be carried out in order to provide, for example, labeled probe 810.

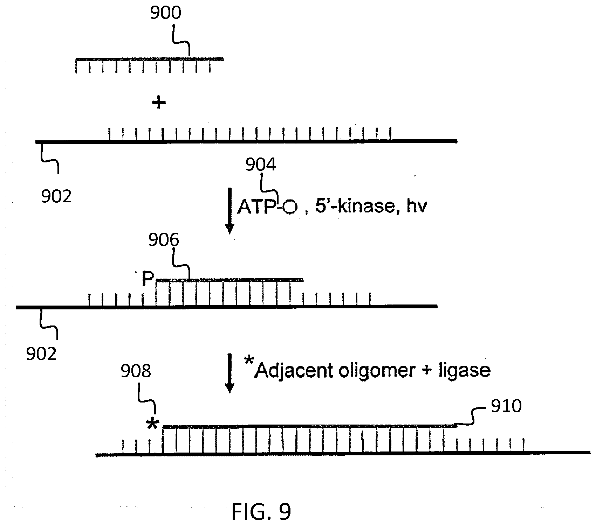

In yet other embodiments, adjacent probes may be linked via a ligase and then a particular tag sequence or unique sequence identifier of one probe may become detectable through ligation to a probe comprising a detectable label. The ligated probes may be amplified using PCR (or ligase chain reaction), if greater sensitivity is desired. A particular embodiment is exemplified schematically by FIG. 9. With reference to FIG. 9, oligo/polynucleotide probe 900 is added to sample 902. Probe 900 associates with a complimentary sequence of sample 902. Sample 902 is then exposed to caged-ATP 904. Activation with light releases the caged ATP. Adding a suitable kinase phosphorylates oligo/polynucleotide probe 900 to provide phosphorylated probe 906. A ligase then ligates oligo/polynucleotide probe 906 to an adjacent oligomer deposited on sample 902 comprising a detectable label 908, thereby forming ligated probe 910. Only those oligomer sequences that are adjacent to the phosphorylated end of oligo/polynucleotide probe 906 will be ligated to the probe. By forming ligated probe 910, a target sequence may be identified once the ligated probe is detected (through detectable label 908) and subsequently quantified using array hybridization or sequencing. In some examples, ligase chain reaction or PCR can be used to amplify ligated probe 910 if greater sensitivity is needed.

### 1. Selecting and Preparing the Sample

Samples may be fresh, frozen, formalin-fixed, paraffin-embedded, or combinations thereof. In some examples, the method also can be applied to samples that do not have genetic abnormalities, diseases, disorders, etc., referred to as "normal" samples. Such normal samples are useful, among other things, as controls for comparison to other samples. The samples can be analyzed for many different purposes. For example, the samples can be used in a scientific study or for the diagnosis of a suspected malady, or as prognostic indicators for treatment success, survival, etc. Samples can include a single target, or can include multiple targets that can be specifically bound by one or more probe embodiments disclosed herein. While the methods may be used on a variety of samples, it is particularly advantageous for tissue samples which have inherent contextual information that is not present in solution based samples. In particular, the relationship between cells, and indeed the size, shape, and distribution of cells provides an enormous amount of information that is lost if that tissue is lysed, digested, ground. Evidencing the importance of this contextual information is the significance of primary staining (i.e. staining with hematoxylin and eosin, H&E) to the diagnosis of cancer. Being non-specific stains, H&E staining provides only contextual information yet it is the gold-standard in diagnosing cancer.

In particular disclosed examples, the sample tissue sample is a formalin-fixed, paraffin-embedded tissue sample. Such tissue samples may be prepared by extracting tissue from a subject, exposing the tissue to a buffered formalin (or paraformaldehyde) solution, and then embedding the tissue with paraffin. The tissue sample(s) may then be placed on a substrate, such as a microscope slide or a microarray slide.

### 2. Contacting the sample with the probe

After a sample is prepared, the biological sample can then be exposed to one or more probes. For example, a sample may be exposed to a probe by contacting the sample with a solution comprising the probe, or a mixture of probes. The solution containing the probe can be made with any biologically-acceptable composition, such as a buffer composition. In some examples, the buffer comprises 50 mM Tris/HCI, 10 mM KCI, 5 mM (NH₄)₂SO₄, 2 mM MgCl₂. For example, the buffer may be selected from those typically used in the art, such as TES, HEPES, Tris, and the like.

In particular disclosed embodiments, the probe is deposited onto a sample region and may be hybridized to, or chemically coupled to (*e.g.,* electrostatically coupled, covalently coupled, or ionically coupled), a particular target sequence or moiety present in the target, or that has also been associated with the target, such as a primary antibody. After the probe is associated with the target, the sample may be washed and any unassociated probe components can be washed away.

In some embodiments, the deposited probe may be detected and particular portions of the sample comprising the deposited probe may be marked for analysis. In some embodiments, visual detection of a signal produced by a detectable label (*e.g.,* a chromophore, a fluorophore, a hapten, etc.) during a primary and/or secondary recognition event can be used to detect the particular portion of the sample to be marked. In some embodiments, one or more portions of the sample may be selected for further analysis by using a stain (*e.g.,* a primary stain, counterstain, or combination thereof). In other embodiments, fluorescence of a fluorophore deposited on one or more portions of the sample may be used to indicate where the sample should be marked for further analysis. In yet other embodiments, morphological characteristics may be used to distinguish portions of a sample for analysis, particularly in cell or tissue samples. For example, fluorescence microscopy and/or phase contrast microscopy techniques known in the art can be used to visualize the shape and/or arrangement of cell components thereby facilitating selection of these particular regions for analysis. Digital pathology techniques also can be used to image the sample and facilitate sample selection.

Once the portion of the sample that will be analyzed has been identified, it may be marked. For example, the portion of the sample that will be analyzed may be manually marked, such as marking a sample portion on a slide to which the sample is attached, or it may be marked on a separate digital viewing screen with the selected locations being stored electronically. In some examples, a marking/selection step may involve a substantially instantaneous irradiation event. In other examples, a marking/selection step may occur first, followed by a subsequent irradiation step. The act of marking/selecting a portion of the sample may be configured to initiate an irradiation event.

### 3. Exposing the sample to light

When the sample is exposed to light, the entire sample may be irradiated, or a portion (or portions) of the sample may be selectively irradiated. In some examples, the sample is selectively irradiated to facilitate isolation of only certain portions of the probe deposited on the sample (*e.g.,* the unique sequence identifier or tag sequence of a probe) in a targeted region of the sample. In some examples, the sample may be selectively irradiated to facilitate isolation of probes deposited on the sample in non-selected regions. That is, selected portions of the sample identified for analysis may be marked and then unmarked portions of the sample to which one or more non-selected probes are bound may then be irradiated, thereby cleaving the non-selected probes. The selected probes may then be isolated using a second irradiation step and/or secondary cleavage technique as disclosed herein and a suitable elution step. In other examples, the sample may be selectively irradiated to facilitate isolation of probes deposited on the sample in the selected regions. Selective irradiation can be performed iteratively to provide multiple different irradiation events. In such examples, targets and/or detection tags optionally can be isolated between each iterative irradiation step to provide a quantitative, multiplexed map of the sample and the various different targets therein.

Selective irradiation can be accomplished using a photomask, a focused beam of light from a light source, or by iteratively tuning (for a particular photo-cleavable moiety) light of selected wavelengths. For example, selective irradiation can be used to activate a photo-cleavable moiety that is present in the probe and/or other component to which the sample is exposed. In some examples, activating the photo-cleavable moiety facilitates cleaving portions of the probe. For example, if the photo-cleavable moiety is used to link two components of a probe, selective irradiation separates the two components.

In some examples, irradiation may be used to generate a reactive species that is capable of reacting with other moieties present, such as by cleaving one or more linkers. Suitable reactive species do not interfere with sample analysis or destroy the sample. Suitable reactive species include, but are not limited to, photoinitiators such as azobisisobutyronitrile (AIBN), benzoyl peroxide (BPO). The reactive species may cleave linkers selected from, for example, photo-cleavable moieties used as linkers, aliphatic linkers, alkylene oxide linkers, disulfide linkers, commercially available linkers, polymeric linkers, and the like.

In some embodiments, the photo-cleavable moiety functions as a blocking agent. In these embodiments, selective irradiation cleaves the photo-cleavable moiety from the particular probe (e.g., a primer) and frees at least one end of the probe for further manipulation. An exemplary embodiment is illustrated below in Scheme 5. Conjugate **500** includes a nitrophenyl moiety that readily absorbs light having wavelengths of about 300 nm to about 400 nm, such as about 325 nm to about 375 nm, or about 345 nm to about 370 nm. The nitrophenyl moiety is attached to at least one nucleotide of an oligo/polynucleotide via an ester group. UV irradiation cleaves the benzyl carbon-oxygen bond, followed by release of CO₂ to form conjugate **502,** which has a free hydroxyl group suitable for chain extension.

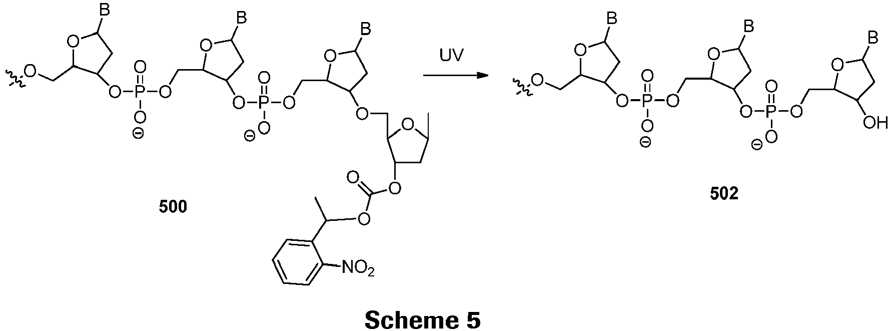

Selective irradiation also can be used to cleave photo-cleavable moieties from a photo-activatable primer extension reagent, such as caged dATP molecules, which are disclosed herein. Other photo-activatable primer extension reagents can include activatable degenerate primers for whole genome amplification or specific genetic lesions, dGTP, dCTP, dTTP, and the like.

Disclosed herein are various different examples for irradiating the sample to provide selective target irradiation. During the irradiation step, the sample is exposed to light capable of providing the energy sufficient to induce a desired result, such as to activate the photo-cleavable moiety. In particular disclosed examples, the light is ultraviolet light having a wavelength ranging from about 200 nm to about 400 nm. In some examples, light is visible light having wavelengths ranging from about 400 nm to about 790 nm. Suitable light sources are capable of providing low intensity light to preserve sample integrity. For example, the light source provides from about 1 mW/cm² to about 1W/cm², such as from about 1 mW/cm² to about 500 mW/cm², or from about 1 mW/cm² to about 200 mW/cm², or from about 5 mW/cm² to about 150 mW/cm², or from about 10 mW/cm² to about 150 mW/cm². Exemplary light sources include, but are not limited to, lasers, LEDs, or lamps (such as incandescent lamps, halogen lamps, mercury lamps, fluorescent lamps, and the like).

In illustrative examples, the irradiated tissue area would be selected dependent on the surface area of diseased tissue, more specifically the number of diseased cells. In many cases, such as biopsies, this area would range from a single cell (10-20 micrometers in diameter) to a few thousand cells (1-50 millimeters in diameter). In resected tissues, the diseased area may include hundreds of thousands to millions of cells covering the majority of a glass slide (75 x 25 mm). In yet other examplesin which a DMD is used, the irradiated area could be about 10 µm x10 µm or larger regions made up by 10 µm x10 µm regions

Selective irradiation may comprise direct irradiation or indirect irradiation. Direct irradiation typically comprises exposing the sample to light, particularly a light source capable of producing light of any suitable wavelength (or wavelengths), without filtering the light produced by the light source. For indirect irradiation, an intermediate filter or masking material may be placed between the light source and the sample, thereby filtering or blocking some of the light produced by the light source from the sample.

Selective irradiation also may comprise exposing the sample to light positioned to selectively expose a particular portion of the sample. For example, an LCD screen may be used to direct light of a selected wavelength (or wavelengths) to a particular location in the sample. A tumor-containing portion of a tissue may be irradiated and a non-tumor-containing portion of the sample may be irradiated (typically sequentially). These particular examplesprovide the ability for collecting portions of a probe (e.g., a tag-template duplex or unique sequence identifier) that are liberated by the selective irradiation (via cleavage of the photo-cleavable moiety) and sequencing a tag-template duplex or unique sequence identifier that correlates to the tumor portion of the sample and a different tag-template duplex or unique sequence identifier that correlates to the normal portion of the sample. In some examples, selective irradiation can be used to cleave a pull-down tag in an unselected tissue area (*e.g.,* an area that does not comprise a tumor). For example, a detectable label (*e.g.,* biotin) attached to a sample through one or more probe components can be cleaved from regions of the sample other than the tumor-containing area.

FIG. 5(A) - (C) are schematic drawings showing several approaches to irradiating a first selected region 504 and/or a second selected region 503 of a sample 502 disposed on a slide 501. Referring now to FIG. 5(A), a microscope objective 511 can be used to view the sample so that a region of interest can be selected. The microscope objective can also be used to direct light irradiating the sample. For instance, region of interest 504 may be selected in the view of objective 511. Light from an appropriate source can then be directed through objective 511 to region 504. The effect of this approach is irradiation of the field of view, as such, the field of view for the microscope is the selected region. Referring now to FIG. 5(B), shown is an alternative approach that uses a light source 520 to irradiate a screen 521 having selective optical density. Screen 521can block incident irradiation to the bulk of sample 502 while allowing irradiation to impact regions 504 and 503 as depicted by beams 522 and 523 passing through the screen to the respective selected regions.

The selective optical density screen may be a liquid crystal display (LCD) screen. The sample regions of interest may be marked by configuring an LCD screen to have the desired area outlined (for example, the positions may be marked manually, and the particular area/pattern transferred to the LCD screen using a projector). The areas that are not to be irradiated are "blacked-out." Portions of the LCD screen that are not "blacked-out" enable passage of the light produced by the light source to the selected region. Referring now to FIG. 5(C), as another example, a digital micromirror device 531 is used selectively irradiate regions 504 and 503. Beams 532 and 533 are generated by irradiating digital micromirror device 531 with light source 520. In a particular example, the digital micromirror device comprises a 1400 x 1050 array of micro-mirrors (12.88 µm²).

In yet other examples, selective irradiation may be obtained by using a digital micromirror device, which can be used to focus light from the light source onto the sample. The digital micromirror device may comprise various different portions/mirror segments of a particular size (*e.g.,* squares having a size ranging from about 4.5x4.5 µm to about 15x15 µm) that can be adapted to irradiate particular locations and/or features within the sample.

The sample typically is exposed to the light for a time sufficient to cleave the desired photo-cleavable moieties, but not so long as to effect sample degradation. For example, the time period during which the sample is irradiated typically is no longer than about 5 minutes, about 4 minutes, about 3 minutes, or about 2 minutes. For example, the sample may be irradiated for an effective period of time ranging from about 1 second to about 60 seconds, typically from about 10 seconds to about 60 seconds, more typically from about 20 seconds to about 60 seconds, with certain working embodiments irradiating the sample for about 20 seconds or less.

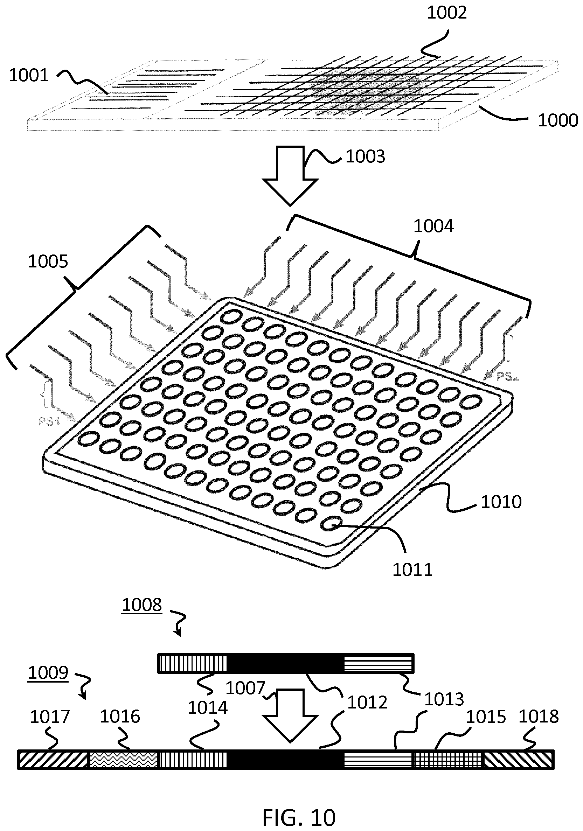

Referring now to FIG. 10, shown is a schematic that illustrates an example of the present disclosure wherein a multiplexed image of the sample is constructed using the approaches described herein. Across a substrate 1000, for example a microscope slide labeled with a barcode 1001, a grid 1002 or other appropriate sectioning strategy is created to overlap the sample (not shown, but understood to be within the grid). Grid 1002 is arranged to correspond to the resolution of the chemical image that is sought. Illustrative of the method, the grid may be a series of raster locations on which the sample is iteratively irradiated. The arrangement and structure of the grid is not material, rather it can be variably designated according to the intended need. In some samples, specific cells are irradiated and the "grid" does not follow a geometric pattern, but instead matches a biologic pattern associated with the sample. Referring back to FIG. 10, arrow 1003 represents removal of detection tags from substrate 1000 to a multi-well plate 1010. Multi-well plates are well known in the art and come in various configurations having various numbers of wells from 2 to hundreds. While shown as a typical macroscopic (*e.g.,* 64-well plate), microscopic vessels are also within the scope of the present disclosure. For example, multi-well plate may be a microfluidic device having small volume chambers. Multi-well plate 1010 is shown to include a plurality of reaction wells 1011.

According to illustrative examples, a light is used to irradiate a selected region (*e.g.,* a single grid location), thereby releasing detection tags from the selected region. The detection tags are removed from substrate 1003, as represented by arrow 1003, and deposited into one of the plurality of reaction wells 1011. The process is repeated iteratively so that detection tags from each selected region are deposited into a different reaction well. While the detection tags present in each cell could be independently analyzed, a more elegant solution is shown in FIG. 10. According to this solution, different forward primers (represented as arrows 1005) can be placed in each row of multi-well plate 1010. Similarly, different reverse primers (represented as arrows 1004) can be placed in each column of multi-well plate 1010. Within each well, detection tag 1008 (represented in a block diagram), includes a portion 1014 corresponding to a portion of forward primer 1005 and a portion 1013 corresponding to a portion of reverse primer 1004. Arrow 1007 represents a PCR step which amplifies the detection tags in each well and incorporates forward and reverse primers to create a PCR product 1009. PCR product 1009 may include a first adapter sequence 1017 (*e.g.,* for NGS), a unique row identifier 1016, the portion 1014 corresponding to the portion of the forward primer 1005, a unique identification tag sequence (*e.g.,* which would include target or reagent information), the portion 1013 corresponding to the portion of the reverse primer 1004, a unique column identifier 1018, and a second adapter sequence 1018 (*e.g*.,also for NGS). The elegance of this approach is that all the wells could then be pooled and analyzed concurrently. The location from where the detection tag came on the sample is encoded in the sequence. As such, all detection tags from each location on the sample could be binned together to read out a highly multi-plexed chemical image of each selected region of the sample.

### 4. Detecting the tag and/or the target

The target may be detected using various detection schemes now known or hereafter developed. In particular disclosed examples, detecting may first comprise obtaining an isolated portion of the probe and analyzing/sequencing the isolated portion of the probe. For example, after the photo-cleavable moiety is cleaved, a portion of the probe may be released and the released portion isolated from the sample, such as by washing the sample with an appropriate buffer solution. If the isolated portion includes information encoded by the nucleotide sequence, the isolated portion is then sequenced, such as by using next generation sequencing. If necessary, or desirable, an isolated portion of the probe may be amplified, such as by performing a polymerase chain reaction. In particular disclosed examples, amplifying and sequencing steps may be combined. For example, portions of the sample that are irradiated may undergo amplification while portions of the sample that are not irradiated are not amplified.

Next generation sequencing provides the ability to sequence a large number of samples, wherein each sample may comprise multiple targets and/or specific binding moieties bound to unique sequence identifiers. As each unique sequence identifier is different, each of the multiple different targets and/or specific binding moieties can be bound to its own unique sequence identifier. Once isolated, unique sequence identifiers can be differentiated during sequencing and the particular target and/or specific binding moiety identified based on its correlated unique sequence identifier. Thus, the identity of the target and/or specific binding moiety may be detected with increased accuracy as compared to typical tissue analytical techniques. Additionally, the quantity of specific binding moieties bound in a particular portion of a sample may be determined based on the isolated unique sequence identifiers.

For example, the liberated unique sequence identifier may be collected out of a suitable microfluidic platform after sample irradiation. The microfluidic platform may be a microarray platform, such as a microarray slide. For example, a microarray slide can be used comprising a surface designed to spatially arrange particular targets in locations that facilitate probe addition and selective retrieval of moieties that are cleaved from the probe (*e.g.,* unique sequence identifiers, tag sequences, primers, and the like). The microarray slide can comprise multiple, spatially arranged locations that can comprise one particular target, multiple targets, or subsets of targets. Due to the spatial arrangement on the microarray slide, a selected region containing a portion of the moieties that are cleaved from the probe after selective irradiation can be eluted without eluting substantial amounts of non-selected regions. The selected region may be exposed to an elution buffer and/or a denaturing buffer. These buffers facilitate removal of the desired moieties within the selected region (*e.g.,* unique sequence identifier, tag sequence, primer, etc.). The microarray slide typically may be used in combination with a microarray scanner that is configured to scan the microarray slide's surface and identify the region of interest. A dispensing instrument also may be used, which can be configured to receive input from the microarray scanner and dispense the desired elution and/or denaturing buffers to a particular region of the slide. An exemplary microarray system comprising these components (and other suitable components) is disclosed in U.S. Patent Publication No. 2010/0076185.

### B. Photochemical Immunohistochemical Analysis

In particular disclosed examples, the method may comprise a histochemical assay suitable for detecting one or more targets in a sample. The probes used in these examplestypically comprise an antibody as the specific binding moiety, a photo-cleavable moiety, and a tag sequence or a unique sequence identifier. Particular examplesof the method concern exposing a biological sample, such as a tissue sample (*e.g.,* an FFPE tissue sample), to one or more probes comprising an antibody that can either bind directly to a target in the sample, or indirectly to a target. For example, the sample may first be contacted with a primary antibody, followed by contacting the sample with a probe comprising a secondary antibody, a photo-cleavable moiety and a unique sequence identifier (or tag sequence), where the secondary antibody specifically recognizes the primary antibody. If a unique sequence identifier is used, then it can be released via selective irradiation and the sequence determined to identify the target detected. This examplealso can be used to quantify the number of bound antibodies in the tissue as the number of sequencing reads can equate directly to the number of bound antibody-unique sequence identifier conjugates.

### C. Photochemical In Situ Hybridization

Also disclosed herein are examplesof a method wherein one or more targets are detected using a photochemical-based *in situ* hybridization assay. In particular disclosed embodiments, the method concerns exposing a sample to a probe comprising an oligo/polynucleotide, a photo-cleavable moiety, and a tag sequence or unique sequence identifier. The probe is allowed to hybridize to the tissue sample and then portions of the sample are irradiated using a light source.

In other disclosed examples, the method may concern exposing a sample to a probe comprising an oligo/polynucleotide, a photo-cleavable moiety, and a first member of a binding pair, typically selected from biotin, streptavidin, or a hapten. After the probe is hybridized to the particular target of interest, the sample is washed and then certain portions are irradiated with light to dissociate a first member of the specific binding pair from any hybridized probes within the particular zone of irradiation. After performing a dehybridization step on the sample, the de-hybridized probes are passed through a column comprising an immobilized second member of a specific binding pair. Probes that were irradiated elute through the column, as specific binding cannot occur between the probe and the second member of the specific binding pair present on the column. Those probes that were not selectively irradiated will not elute through the column, as the still-attached first member of the specific binding pair binds with the second member and is retained on the column. In some examples, the method facilitates isolation of the probes located in the particular regions of interest.

### D. In Situ Polymerase Extension

In particular disclosed embodiments, photochemistry may be used to facilitate an *in situ* polymerase extension reaction wherein polymerase enzymes may be added to tissue for *in situ* polymerase-mediated extension, *in situ* PCR, and/or *in situ* nick-translation. For example, a nucleic acid target with a point mutation may be analyzed by exposing the nucleic acid target to a probe comprising an oligo/polynucleotide and a photo-cleavable moiety that blocks one end, typically the 3' end, of the oligo/polynucleotide. The oligo/polynucleotide may further comprise a detectable label.

In another embodiment one or more of the deoxynucleoside triphposphates are blocked with a photo-cleavable moiety and the oligo/polynucleotide may or may not be blocked with a photo-cleavable moiety.

The extent of polymerase chain extension that can be obtained using embodiments of the disclosed method ranges from hundreds of extended nucleotides to thousands of extended nucleotides. For example, the number of nucleotides added to the initial probe can range from about 10 nucleotides to about 2000 nucleotides (such as about 50 nucleotides to about 1500 nucleotides, or about 100 nucleotides to about 1000 nucleotides, or about 200 nucleotides to about 400 nucleotides). The extent of the chain extension can be determined using one or more reverse primers that have some degree of complementarity (*e.g.,* about 70% to about 99% complementarity) to certain regions of the extended probe. Polymerase-mediated chain extension may be carried out using a range of probe concentrations (*e.g.,* 100 ng/mL to about 100 µg/mL, such as about 10 ng/mL to about 10 µg/mL), and can be carried out for any suitable amount of time, such as from about 20 minutes to about 90 minutes.

### E. Multiplexing

The components, methods, kits, and systems described herein primarily provide description for one or two probes used on a particular sample. While useful in this context, one significant advantage of the present approach is that the detection modality is based on oligonucleotides. As such, the detection and quantification of binding events is performed using robust and well-known oligonucleotide approaches which are capable (and routinely applied to) high levels of multiplexing. In illustrative examples, between about 2 and about 100,000 targets are detected. In other examples, between about 10 and about 100,000 targets are detected. In another example, between about 10 and about 1000 targets are detected. In yet another example, greater than about 5 targets are detected. In another example, greater than about 10 targets are detected.

### VI. Advancements over the Art

Embodiments of the methods disclosed herein provide advancements over methods currently used in the art for histopathology. In some embodiments, the disclosed method provides quantifiable results because labels and probes used in embodiments of the method are directly detected and do not require further amplification. Embodiments of the disclosed method are suitable for multiplexing assays as the detection tags used in certain embodiments of the method are uniquely distinct with an infinite number of different species available for use in the disclosed probes. Many multiplexing methods known in the art are limited by the number of detectable tags that can be attached to ISH probes and/or the type of detection system used for immunohistochemistry (IHC) analysis (*e.g.,* colorimetric and/or fluorescent detection schemes). While methods exist to address such limitations, these methods typically require disassociating and lysing the tissue sample, thereby destroying all tissue context. The method disclosed herein avoids these undesirable steps and provides a level of maintained tissue contextual information that is important in IHC and ISH analysis. The disclosed method also provides the benefit of preserving tissue samples for repeated analyses, archiving, and/or conformational analysis. The disclosed method not only facilitates multiplexing assays, but certain embodiments further provide the ability to repeat multiple distinct multiplexing assays on different portions of a particular sample. Methods currently known in the art are unable to address tissue heterogeneity in the same way.

Embodiments of the disclosed method also offer levels of specificity and sensitivity that far exceed methods currently known in the art. For example, some embodiments concern using primer extension techniques to produce detectable moieties that can be isolated from a sample for further analysis. Such *in situ* extension can be conducted solely on the particular gene of interest, whereas all other sequence reads can be ignored.

In addition, embodiments of the disclosed method provide higher spatial resolution than is achieved with tissue microdissection methods used in the art. Methods disclosed herein also facilitate probing cellular structure and rather than solely the cell itself. The methods disclosed herein also can be automated, thereby providing fast, efficient, methods that can be conducted on bench-top automated devices.

### VII. Working Embodiments

### Probe Design:

In some examples, oligonucleotide DNA probes were synthesized with a 3' binding sequence to either a chromosome 17 alpha satellite repeat sequence (SEQ ID NO: 4) or an ALU repetitive sequence (SEQ ID NO: 5), and a 5' non-binding sequence. In some experiments, a nick-translated, DIG labeled clone of the α7t1 alpha satellite repeat from the centromeric region of chromosome 7 was used as a non-UV sensitive control probe. Probes used for demonstration of tissue irradiation were designed with a 5' non-binding portion that contained two DNP (dinitrophenol) haptens (DNP-TATTTT-DNP-TATTTT), whereas probes used to demonstrate PCR detection of a DNA barcode were designed with a non-binding 5' tail that contained priming sites for M13 primers (Forward: 5' being SEQ ID NO: 6, Reverse: 5' being SEQ ID NO: 7) flanking a DNA unique sequence identifier (SEQ ID NO: 1). The two sequence elements were linked via a photo-cleavable moiety (Ambergen).

### In Situ Hybridization:

*In Situ* Hybridization (ISH) procedures were executed on a BENCHMARK XT automated slide stainer. Probes were hybridized at 42 °C for two hours in a formamide containing buffer. Probes were washed, and slides were removed from the staining instrument and irradiated. Following UV irradiation, the hybridized probes were detected using Ventana SISH detection on a Benchmark XT instrument.

### UV Irradiation:

An LED light source emitting at 365nm (XeLED-Ni1UV-R3-365, Xenopus Electronix) was used to irradiate the tissue at a distance of 2 cm for 30 seconds. Aluminum foil was placed over the tissue section to block the UV light. Subsequent irradiation methods used UV light from a metal-halide lamp (X-Cite, Lumen Dynamics) passed through a 20x microscope objective. UV energy doses ranged from 50 - 10 mW for ten seconds to one minute. Intensity of the DMD device UV source was 130 mW/cm2. The slides were irradiated for 20 seconds.

UV irradiation using an LCD screen also was used to shine a pattern on the tissue. The field diaphragm of a microscope using Kohler irradiation was replaced with a liquid crystal panel to turn the microscope into a projector. This allowed controlling which regions of the slide to irradiate by masking specific regions of this tissue from irradiation. For tissue irradiation, the LCD panel was transitioned into a checkerboard pattern. Slides were irradiated using 100 mW for 30 to 60 seconds.

### Example 1

To determine whether UV-cleavable DNA oligonucleotides are suitable DNA probes, a CHR-17 oligonucleotide was designed with a photo-cleavable moiety between the target binding region and the detection region, which comprised detectable DNP labels. The probes were hybridized to a tissue sample; the slides were taken off the instrument and then irradiated with UV light. The slides were then put back on the instrument and the presence of the UV-cleavable DNA tag was determined using a silver ISH [SISH] detection kit. A control also was used with a nick-translate DIG CHR-7 probe.

The results indicated the ability of a probe containing a photo-cleavable moiety to be selectively irradiated and therefore useful in detecting a DNA target in a tissue sample. No DNP tags were detected in the control sample as the portion of the sample was exposed to light and all DNP tags were cleaved with the photo-cleavable moiety. In comparison, the masked areas (*i.e.,* non-irradiated portions) of the sample did contain DNP-labeled probes as the photo-cleavable moiety in these probes were not cleaved. The DNP labels attached to the probe through the photo-cleavable moiety were therefore detectable using SISH detection.

### Example 2

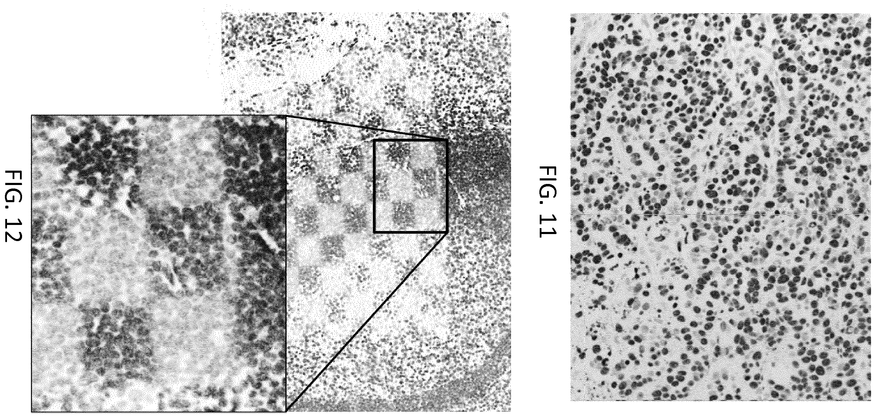

In this example, an ALU DNA ISH probe (comprising an oligonucleotide, photo-cleavable moiety, and tag sequence, labeled with DNP label) was designed to deposit a significant amount of SISH signal off of a DNA hapten attached to a DNA detection region of a DNA probe. The probe was hybridized to various spots on the tissue sample, excess probe was washed, and the slides comprising the tissue samples were removed from the automated stainer and irradiated using one of three methods. In one example, a sample was irradiated at 50 mW for approximately 20 seconds using a 20x objective. FIG. 11 provides an image of a control ALU probe that was not irradiated with light.

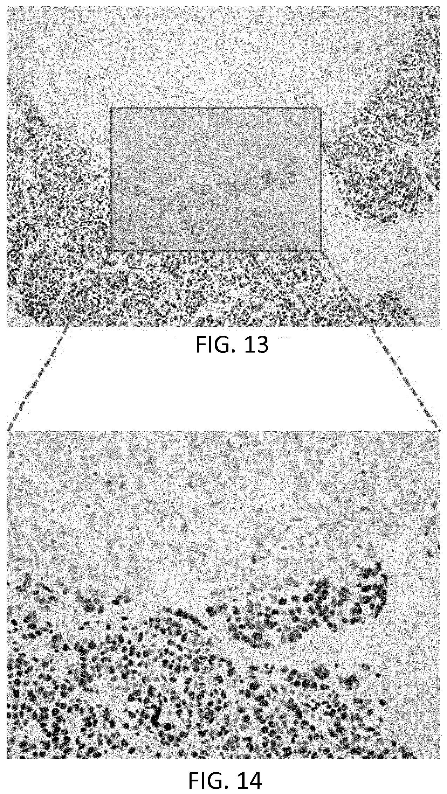

An additional, different sample slide was exposed to a different UV irradiation method. In this example, an LCD screen was placed between a UV lamp and the slide. The LCD display was transitioned to provide a pattern by projecting a patterned slide and thereby making the UV light in the blacked-out portions of the pattern. The slide was irradiated for approximately 60 seconds and provided the patterned sample, to which a photomicrograph is shown in FIG. 12. As shown in FIG. 12, portions of the sample that were exposed to light did not provide a detectable signal, as the DNP tags attached to the probe through a photo-cleavable linker were detached from the sample, whereas those portions that were not irradiated did provide a detectable signal produced by SISH detection of the DNP tags. Additional examples are illustrated in FIGS. 13-16, which also show that aspects of the disclosed method can be used to obtain patterned samples using selective irradiation. In particular, FIGS. 13 and 14 show two levels of magnification for a region that had been directly irradiated using a microscope objective. The spherical aspect of the irradiation is clearly observed in FIG. 13, while the contrast and sharpness of the interface between the irradiated and non-irradiated sections can be seen in FIG. 14.

FIGS. 15 and 16 show the versatility in shape and provide an indication of the resolution which can be accomplished using a light source with a digital micromirror device (DMD). In particular, FIG. 15 shows that an elaborate symbol can be illuminated while FIG. 16 shows the resolution of the approach, where each of the speckles represents quality control patterns associated with the DMD.

### Example 3

In this particular example, a CHR-17 oligonucleotide was used as a primer for DNA polymerase I. The probe was hybridized to tissue, which was then washed. Using nick-translation protocol, DNA polymerase I (*e.g.,* 10, 1, or 0.1 units of DNA polymerase per slide) and other extension reagents were added to the sample to extend the 3' end of the CHR-17 oligonucleotide, with an extension time of 2 hours. DIG-dUTP was then added to detect primer extension using an anti-DIG, AP Red detection kit. The results for these particular examples indicated that polymerase chain extension had occurred as the AP red labels were detected in the sample.

### Example 4

In this particular example, a DNA probe, comprising a photo-cleavable moiety and a unique sequence identifier (comprising a primer portion, a sequence identifier portion, and a second primer portion) was used. The DNA probe was hybridized to tonsil tissue on a BenchMark XT instrument. The slides were then removed and irradiated with UV light at 50 mW for approximately 20 seconds using a 20x objective. Liquid on the slides containing the liberated unique sequence identifiers was collected and used as a template in subsequent PCR reactions.

### Example 5

In one example, a biotinylated secondary antibody was bound to a biotinylated tag sequence using streptavidin. An antigen retrieved target was first labeled with a primary antibody, followed by binding with the biotinylated anti-species secondary antibody. Streptavidin was used to connect the biotinylated tag with the biotinylated antibody. After a stringent wash, the tag was hybridized to a template oligonucleotide. The slide was then treated with exonuclease. All steps were performed on a VMSI Benchmark XT stainer. After washing and air-drying, the template was transferred into a solution for PCR quantification.

Series dilutions of two particular template oligonucleotides were made and the quantification dynamic range determined by using corresponding primers. A seven orders of magnitude linear range was achieved with the detection of 120 molecules possible (2 µL of 0.1 fM). Run-to-run reproducibility also was demonstrated. There was little discrepancy between the two template oligonucleotides and therefore it is possible to develop multiplexed assays by using different template oligonucleotides with the same probe. The detection and quantification of the template oligonucleotides with sufficient sensitivity and linear dynamic range have been demonstrated. In some examples, a specific template was quantified even in the presence of other template oligonucleotides using a corresponding unique primer.

### Example 6

In this particular example, ALU and CHR17 oligo/polynucleotide sequences (SEQ ID NO: 5 and SEQ ID NO: 4, respectively) attached to the three unique sequence identifier sequences (SEQ ID NOS: 21-23) via a photocleavable linker (commercially available from TriLink, structure provided below) were designed. The probes (1 µg/ml final concentration) were hybridized to tonsil tissue and were exposed to UV irradiation using a digital mirror display (DMD) irradiation technique. Irradiation was carried out using the entire DMD array field (19,151 x 14,363 µm) with a UV intensity of 130 mW/cm². Control slides were left untreated with UV light.

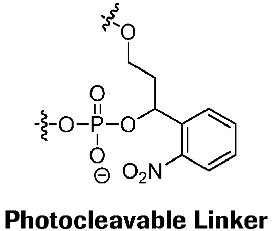

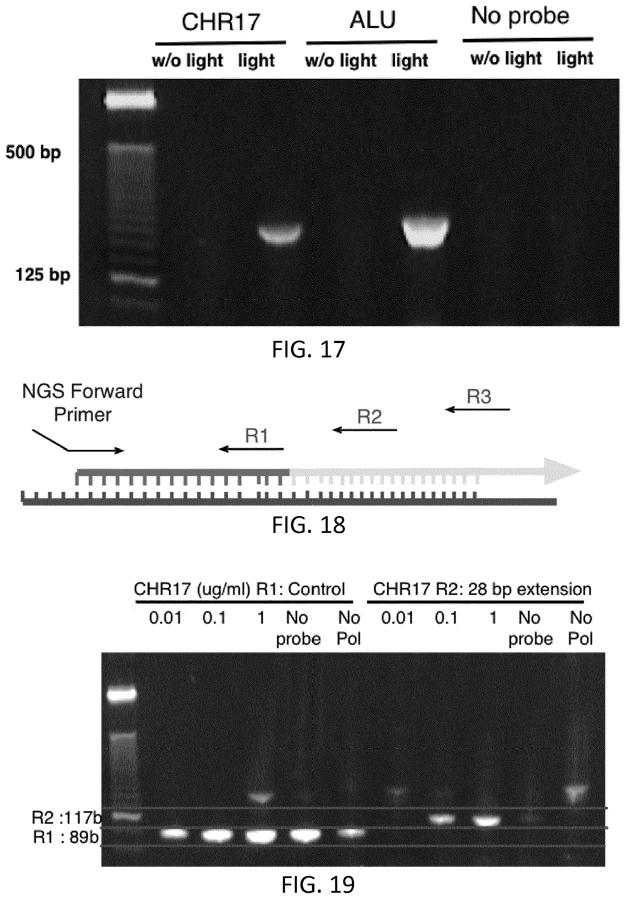

After the irradiation, the liberated unique sequence identifiers were released into 200 µL of TE buffer solution and collected using a microfluidic platform. PCR amplification was performed in a 50 µL of total reaction using 20 µL of the solution comprising the unique sequence identifiers as a template and NGS specific (Illumina) adaptor sequences (SEQ ID NOS: 26 and 27) as primers. Amplification was performed using the following thermal cycling conditions: 98 °C for 10 seconds, 60 °C for 10 s, 72 °C for 1 min (15-20 cycles). The amplified unique sequence identifiers with Illumina adaptors were analyzed using gel electrophoresis (Invitrogen 4% EZ Gel). The results are shown in FIG. 17. As shown in FIG. 17, no signal was obtained for control samples that were not irradiated (and therefore did not provide unique sequence identifiers capable of being isolated) and for control samples that were not exposed to the probe comprising the unique sequence identifiers. Bands were obtained, however, for the unique sequence identifiers (attached to the NGS specific adaptor sequences) that were exposed to light.

Furthermore, we demonstrated the detection of single and multiplexed DNA barcodes corresponding to diagnostic biomarkers via Illumina Sequencing. In particular, ALU, CHR7 and CHR17 probes attached to the three unique barcode sequences were hybridized to the tonsil tissue separately and/or in multiplex. After washing, the barcodes were then released into the solution by UV illumination. The Illumina adaptors were attached to the barcodes in a PCR reaction using the solution containing the barcodes as the template (as described previously). Each PCR sample was purified, and the gel band of the target size (211 bp) was selected on 2% E-gel Size Select agarose gel (Invitrogen) resulting in sequencing-ready library for Illumina paired-end sequencing. The library was then quantified using the PicoGreen assay (Invitrogen Molecular Probes) and multiplexed with other libraries and ran on a Illumina MiSeq instrument (150 x 150 paired end).

The counts from the sequencing run were mapped using BOWTIE2 with a 68.79 % overall alignment rate (Table 1). In this particular experiment, barcode counts corresponding to CHR7 and CHR17 are in multiplex, *i.e.,* the probe cocktail containing CHR7 and CHR17 probes were hybridized simultaneously on the same tissue slide. The barcode counts corresponding to ALU is from a separate single ISH experiment. The libraries were multiplexed and analyzed on a single MiSeq run.

**Table 1**

| Barcode | Centromeric Probe | MiSeq Counts | Binding events/cell |
|---|---|---|---|
| BC1 | CHR7 | 37602 | ^{∼}200-1000 |
| BC2 | CHR17 | 118013 | ^{∼}3000 |
| BC3 | ALU | 1398884 | ^{∼}1,000000 |

The barcode counts corresponding to CHR7 and CHR17 in multiplex correlate very well with the binding events demonstrating the sensitivity of the NGS sequencing to quantify biomarkers in multiplex.

### Example 7

In this particular embodiment, the ability to extend off of target sequences within the tissue was tested using PE1.0-CHR7 (SEQ. ID. NO. 45) and PE1.0-CHR17 (SEQ. ID. NO. 46) probes. These experiments demonstrate polymerase extension without the added complexity of a photo-reactive step. The sequences were:
PE1.0-CHR7 (SEQ. ID. NO: 45):
   - **5'**-AATGATACGGCGACCACCGAGATCTACACTCTTTCCCTACACGACG CTCTTCCGATCT**TCTAGCCATTTGATGCCAACAGTAGAAAGGG-3'**
PE1.0-CHR17 (SEQ. ID. NO: 46):
   - **5'**AATGATACGGCGACCACCGAGATCTACACTCTTTCCCTACACGACGCT CTTCCGATCT**CATTCAAATCCCCGAGTTGAACTTTCCTTTCAAAGTTC ACGT-3'**

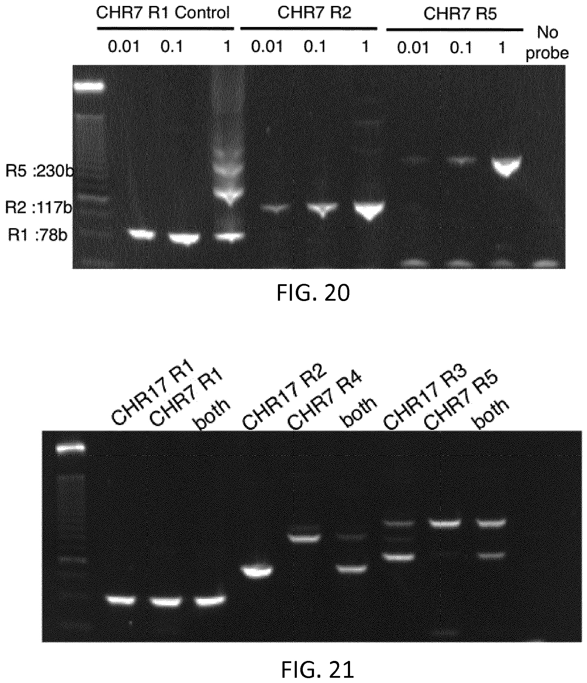

The probes were hybridized to tissue with increasing concentrations of 10 ng/ml, 0.1 µg/ml and 1 µg/ml per slide. Following deparaffinization and cell conditioning on the Benchmark XT, single or a cocktail of PE1.0-CHR7 and PE1.0-CHR17 probes, dNTPs and FastTaq DNA PoLymerase (Roche Applied Sciences) were added on the slides. Following hybridization and extension at 60°C for 1 hour in a buffer comprising 50 mM Tris/HCl, 10 mM KCl, 5 mM (NH₄)₂SO₄, 2 mM MgCl₂, the probe sequences were collected off of the slides following a high temperature denaturation. The extended sequences were selectively amplified using a forward primer within the Illumina NGS adaptor sequence (SEQ ID NO: 26). The distance of the *in situ* polymerase extension was detected using reverse primers (SEQ ID NOS: 29-33) complementary to the regions further from the probe sequences (FIG. 18). Amplification was performed using the following thermal cycling conditions: 98 °C for 10 seconds, 60 °C for 10 s, 72 °C for 1 min (15-20 cycles), with modifications of the annealing temperatures based on the melting temperatures of the primer pairs. The products were analyzed using gel electrophoresis (Invitrogen 4% EZ Gel), and the results are provided in FIGS. 19 and 20.

As illustrated in FIGS. 19 and 20, as the concentration of each probe is increased, the obtained signal increases. FIGS. 19 and 20 also show signals obtained from the reverse primer extension, which indicates that the extension reaction is capable of producing extended products having high numbers of base pairs. As illustrated in FIG. 20, the method produced an extended product having about 230 base pairs. The results obtained from this particular embodiment illustrate the ability to extend primers that are hybridized to tissue without having to first isolate a section of the tissue sample. Rather, the primer can be added to a tissue sample, extended, amplified, and detected without ever having to destroy tissue context by physically excising and/or manipulating the tissue sample. Furthermore, it demonstrates the ability to change the detection length. The three extension lengths were selected to show an ability to extend to various lengths.

### Example 8

In this particular embodiment, CHR7 and CHR17 primers (SEQ ID NOs: 20 and 4, respectively) were hybridized to a tissue sample simultaneously with concentrations of 0.1 µg/ml each. The collected, extended sequences were amplified via duplex PCR using both of the extended CHR7 and CHR17 reverse primers (FIG. 21). This particular embodiment establishes that the disclosed methods are suitable for multiplexing assays. Again, these were done in the absence of a photo-reactive step to show robustness of the underlying extension approach.

### Example 9

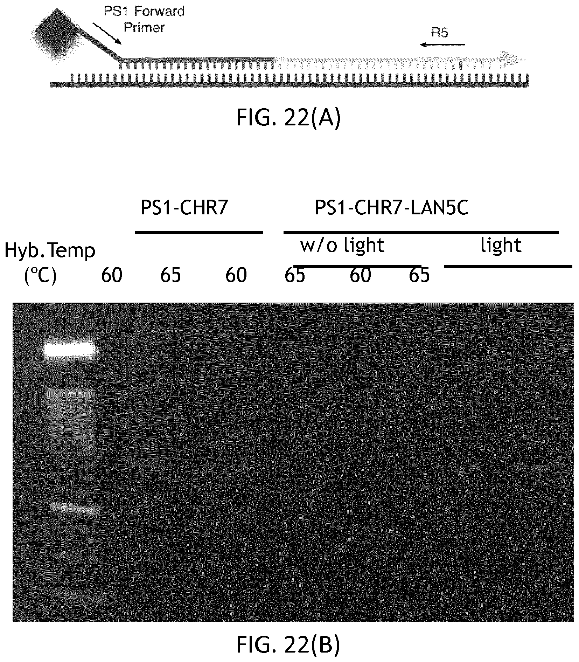

Referring now to FIGS. 22(A) - (B), FIG 22(A) is a schematic showing that an approach by which the sequences could be purified using a biotin molecule at the 5'end (square). PS1 and CHR 7R5 (which is complementary to the region further from the probe sequence) were used to detect *in situ* polymerase extension. FIG. 22(B) is a photograph of a gel showing that subsequent PCR amplification, a 230 bp extended product was detected with the control probe Biotin-PS1-CHR7 with no caged thymidines (Lanes 1-2).

In this particular embodiment, light selective *in situ* polymerase extension using light-activated oligos (LANs) is demonstrated. In particular, light-activated oligonucleotides (LANs) (TriLink technologies) were used to demonstrate selective polymerase extension in tissue. LANs contain photolabile nucleobase modifications, nitropiperonyloxymethyl (NPOM), to one or more of the thymidines. The NPOM-caging group interrupts hybridization by sterically blocking Watson-Crick base pairing. Reference is made to Young et al., 2008 Light triggered polymerase chain reaction, Chem. Commun., 2008, 462-464 for disclosure relate to *in situ* polymerase extension.

CHR7 probe was synthesized with a nonhuman priming sequence (PS1: SEQ. ID NO. 42) and a biotin molecule at the 5'-end and 'caged' thymidines distributed towards to 3'-end. The non-caged version of the probe was used as a positive control (FIG. 22(B), lanes 1-2).

The probes were hybridized to tissues on BenchMark XT instrument at 60°C and 65°C for 1 hour. After the hybridization, slides that will be illuminated by UV were taken off the instrument (FIG. 22(B), lanes 5-6) while the rest were kept dark (FIG. 22(B), lanes 3-4) in the instrument. The slides were treated with UV for 1 min with a UV lamp for slide illumination and put back in the instrument. The unbound material was removed from the slide with a stringency wash. The extension buffer containing Sequenase V2.0 DNA polymerase (Affymetrix, Inc) and dNTPs (Roche) were added on the slides to allow polymerase extension. The probe sequences were collected from the slides following a high temperature denaturation and purified with Dynabeads M-280 Streptavidin beads (Invitrogen). Purified sequences bound to the streptavidin beads were used as a template for detection PCR. Light selective in-situ polymerase extension was demonstrated by using PS1 as a forward primer and CHR7R5 reverse primer complementary to the regions 230 bp further from the probe sequence. The products were analyzed using gel electrophoresis (Invitrogen 4% EZ Gel).

While the probes from the slides without UV illumination failed to produce extended product due to the caged thymidines (Lanes 3-4), the probes on the UV illuminated slides were successfully extended and produced the expected size band (Lanes 5-6) at both hybridization temperatures. Primer and probe sequences used in this study:
- PS1 (SEQ. ID NO: 42): 5'-TAACTTACGGAGTCGCTCTACG-3'
- CHR7 (SEQ. ID NO: 43): 5'-TCTAGCCATTTGATGCCAACAGTAG AAAGGG-3'
- Biotin-PS1-CHR7LAN5C (SEQ. ID NO: 44): 5'/{Biotin}/ TAACTTACGGA GTCGCTCTACGTCTAGCCAT*T*T*GAT*GCCAACAGT*AGAAAGGG-3'
- *T denotes positions of caged thymidines in caged versions of the probes.

### Example 10

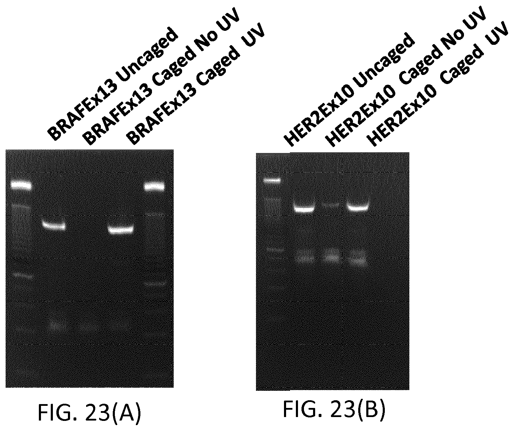

In this embodiment, we demonstrate photo-selective *in situ* polymerase extension of single genes. Referring now to FIG. 23(A)-(B), probes specific for BRAF Exon 13 (FIG. 23(A)) and for HER2 Exon 10 (FIG. 23(B)), was designed with and without caged thymidines. Both primers were hybridized and polymerase extended at 60 °C for 1 hour. The slide which was hybridized with the caged thymidines was treated with UV light, while the remainder was kept in the dark. Lane 1 (exclusive of the marker lanes) shows the control probe with no caged thymidines; thus, extension products are generated and detected as expected. Lane 2 shows the caged thymidine containing probe which had not been subjected to light. It is apparent from the gel that no extension occurred. Lane 3 shows the caged thymidine containing probe subjected to UV light and the extended product is detected at a level similar to that expected from the control probe (i.e. probe without caged thymidine). The light activated nucleotides were included at 5 different locations in both primers. Both exon primers were biotin labelled at the 5' end, and extended primers were purified with streptavidin beads following denaturation at 98 °C for 3-4 min. The aliquots of the purified products were PCR amplified with the corresponding primer pairs and detected with Invitrogen 4 % E-gel. The following probe sequences used in this study:
- Biotin-HER2Exon10 (SEQ. ID NO: 47): 5'-Biotin-TAACTTACGGAGTC GCTCTACGGCATGGAGCACT*T*GCGAG AGGT*GAGGGCAGT*T*A-3'
- BiotinBRAFExon13 (SEQ. ID NO: 48): 5'-Biotin-TAACTTACGGAG TCGCTCTACGAGTGGTGTGAGGGCTCCAGCTTGTAT*CACCAT*CT*A T*T*G-3'
- *T denotes positions of caged thymidines in caged versions of the probes.

### SEQUENCE LISTING

The nucleic and amino acid sequences provided herein are shown using standard letter abbreviations for nucleotide bases, and three letter code for amino acids, as defined in 37 C.F.R. § 1.822. Only one strand of each nucleic acid sequence is shown, but the complementary strand is understood as included by any reference to the displayed strand.
SEQ ID NO: 1 is an exemplary sequence for a unique sequence identifier.
   ATGGTATCCTTACATCGTCATTTATC
SEQ ID NO: 2 is another exemplary sequence for a unique sequence identifier.
SEQ ID NO: 3 is yet another exemplary sequence for a unique sequence identifier.
SEQ ID NO: 4 is an exemplary sequence for an oligo/polynucleotide specific binding moiety.
   CATTCAAATCCCCGAGTTGAACTTTCCTTTCAAAGTTCACGT
SEQ ID NO: 5 is another exemplary sequence for an oligo/polynucleotide specific binding moiety.
   CGGGAGGCGGAGGTTGCAGTGAGCC
SEQ ID NO: 6 is an exemplary primer sequence for use with a probe disclosed herein.
   GTAAAACGACGGCCAGT
SEQ ID NO: 7 is another exemplary primer sequence for use with a probe disclosed herein.
   CAGGAAACAGCTATGAC
SEQ ID NO: 8 is a Her1 amino acid sequence (NCBI Reference Sequence No. NP_005219.2).
SEQ ID NO: 9 is an exemplary cDNA sequence encoding Her1 (NCBI Reference Sequence No. NM_005228.3).
SEQ ID NO: 10 is a Her2 amino acid sequence (NCBI Reference Sequence No. NP_004439.2).
SEQ ID NO: 11 is an exemplary cDNA sequence encoding Her2 (NCBI Reference Sequence No. NM_004448.2).
SEQ ID NO: 12 is a Her3 amino acid sequence (NCBI Reference Sequence No. NP_001973.2).
SEQ ID NO: 13 is an exemplary cDNA sequence encoding Her3 (NCBI Reference Sequence No. NM_001982.3).
SEQ ID NO: 14 is a Her4 amino acid sequence (Genbank Accession No. AAI43750).
SEQ ID NO: 15 is an exemplary cDNA sequence encoding Her4 (GenBank Accession No. BC143749.1).
SEQ ID NO: 16 is an Estrogen Receptor amino acid sequence (NCBI Reference Sequence No. NP_000116.2).
SEQ ID NO: 17 is an exemplary cDNA sequence encoding an Estrogen Receptor (NCBI Reference Sequence No. NM_000125.3).
SEQ ID NO: 18 is a Progesterone Receptor amino acid sequence (Genbank Accession No. AAD01587.1).
SEQ ID NO: 19 is an exemplary cDNA sequence encoding a Progesterone Receptor (GenBank Accession No. AF016381.1).
SEQ ID NO: 20 is an exemplary oligo/polynucleotide probe sequence.
   TCTAGCCATTTGATGCCAACAGTAGAAAGGG
SEQ ID NO: 21 is an exemplary unique sequence identifier sequence.
   CTTGACTGAGCGACTGAGC
SEQ ID NO: 22 is another exemplary unique sequence identifier sequence.
   CTCCAGGGTTAGGCAGATC
SEQ ID NO: 23 is yet another exemplary unique sequence identifier sequence.
   CAGCGGGATAGTGCGATTC
SEQ ID NO: 24 is an exemplary oligo/polynucleotide sequence that can be used in *in situ* polymerase extension methods.
   TAACTTACGGAGTCGCTCTACG
SEQ ID NO: 25 is an exemplary oligo/polynucleotide sequence that can be used in *in situ* polymerase extension.
   GGATGGGATTCTTTAGGTCCTG
SEQ ID NO: 26 is an exemplary forward primer sequence comprising an NGS adaptor sequence for use in *in situ* polymerase extension.
SEQ ID NO: 27 is an exemplary reverse primer sequence comprising an NGS adaptor sequence for use in *in situ* polymerase extension.
SEQ ID NO: 28 is an exemplary primer sequence that can be used for PCR analysis of the SEQ ID NO: 27.
   CGACCACCGAGATCTAC
SEQ ID NO: 29 is an exemplary reverse primer that can be used in PCR analysis of isolated probes that have been extended using *in situ* polymerase extension.
   ACGTGAACTTTGAAAGG
SEQ ID NO: 30 is an exemplary reverse primer that can be used in PCR analysis of isolated probes that have been extended using *in situ* polymerase extension.
   GTAGATCCTGCAAAAAGAGTG
SEQ ID NO: 31 is an exemplary reverse primer that can be used in PCR analysis of isolated probes that have been extended using *in situ* polymerase extension.
   CCTTTCTACTGTTGGCATC
SEQ ID NO: 32 is an exemplary reverse primer that can be used in PCR analysis of isolated probes that have been extended using *in situ* polymerase extension.
   CTGAGAATGATTCTGTCTGG
SEQ ID NO: 33 is an exemplary reverse primer that can be used in PCR analysis of isolated probes that have been extended using *in situ* polymerase extension.
   CAATGCGGTCCATATATCC
SEQ ID NO: 34 is an exemplary oligo/polynucleotide sequence comprising a detectable label, an oligo/polynucleotide sequence, and photocleavable moiety. {Biotin}/CATTCAAATCCCCGAGTTGAACTTTCCTTTCAAAGTTCACGT/{PCBiotin}
SEQ ID NO: 35 is an exemplary oligo/polynucleotide sequence comprising a detectable label, an oligo/polynucleotide sequence, and photocleavable moiety. {Biotin}/TCTAGCCATTTGATGCCAACAGTAGAAAGGG/{PCBiotin}
SEQ ID NO: 36 is an exemplary oligo/polynucleotide sequence that can be labeled with a photocleavable moiety for use in the methods disclosed herein. {PCBiotin}/TAACTTACGGAGTCGCTCTACGCATTCAAATCCCCGAGTTGAACTTTC CTTTCAAAGTTCACGT
SEQ ID NO: 37 is an exemplary oligo/polynucleotide sequence that can be labeled with a photocleavable moiety for use in the methods disclosed herein. {PCBiotin}/TAACTTACGGAGTCGCTCTACG
   TCTAGCCATTTGATGCCAACAGTAGAAAGGG
SEQ ID NO: 38 is an exemplary oligo/polynucleotide sequence that is modified to comprise a caged thymidine and further comprises a biotin moiety.
   {Biotin}/TAACTTACGGAGTCGCTCTACGCATTCAAATCCCCGAGTTGAACTTTCCT TTCAAAGT**T**CACGT
SEQ ID NO: 39 is an exemplary oligo/polynucleotide sequence that is modified to comprise a caged thymidine and further comprises a biotin moiety.
   {Biotin}/TAACTTACGGAGTCGCTCTACGCATTCAAATCCCCGAGTTGAACTTTCCT TTCAAAGT**T**CACG**T**T
SEQ ID NO: 40 is an exemplary oligo/polynucleotide sequence that is modified to comprise a caged thymidine and further comprises a biotin moiety.
   {Biotin}/TAACTTACGGAGTCGCTCTACGTCTAGCCATTTGATGCCAACAGTAGAA AGGGAAA**T**A
SEQ ID NO: 41 is an exemplary oligo/polynucleotide sequence that is modified to comprise a caged thymidine and further comprises a biotin moiety.
   {Biotin}/TAACTTACGGAGTCGCTCTACGTCTAGCCATTTGATGCCAACAGTAGAA AGGGAAA**T**ATC**T**T
SEQ ID NO. 42: is an exemplary primer sequence (PS1) according to Example 9. 5'-TAACTTACGGAGTCGCTCTACG-3'
SEQ ID NO. 43: is another exemplary sequence for Chr7 according to Example 9.
   5'-TCTAGCCATTTGATGCCAACAGTAGAAAGGG-3'
SEQ ID NO. 44 is a Biotin-PS1-CHR7LAN5C probe according to Example 9. 5'-{Biotin}/TAACTTACGGAGTCGCTCTACGTCTAGCCATTTGATGCCAACAGTAGAA AGGG-3'
SEQ ID NO. 45 is a probe according to Example 7 (PE1.0-CHR7).
SEQ ID NO. 46 is a probe according to Example 7 (PE1.0-CHR17).
SEQ ID NO. 47 is a BiotinHER2Exon10 probe according to Example 10, wherein *T denotes positions of caged thymidines in caged versions of the probes:
   5'-{Biotin}/TAACTTACGGAGTC GCTCTACGGCATGGAGCACT*T*GCGAGAGGT*GAGGGCAGT*T* A-3'
SEQ ID NO.48 is a BiotinBRAFExon13 probe according to Example 10, wherein *T denotes positions of caged thymidines in caged versions of the probes.
   5'-{Biotin}/TAACTTACGGAG TCGCTCTACGAGTGGTGTGAGGGCTCCAGCTTGTAT*CACCAT*CT*A T*T*G-3'

## Claims

1. A method for performing contextual molecular diagnostics in a tissue sample, the method comprising:
contacting the tissue sample with a probe comprising a photo-reactive moiety, a specific binding moiety to a target, and a detection tag using conditions sufficient to facilitate binding of the specific binding moiety to a target in the sample;
removing probe that does not bind to the sample;
adding a template sequence to the tissue sample, wherein the template sequence is configured to specifically hybridize with the detection tag;
selecting a region of the sample for irradiation based on contextual information;
irradiating the selected region of the tissue sample with light of a wavelength and an intensity sufficient to cause the photo-reactive moiety to react, thereby freeing the detection tag from the selected region of the tissue sample for further reaction;
exposing the tissue sample to an enzyme that acts on the detection tag to effect a change; and detecting the change, wherein the detection tag is an extendable primer, the photo-reactive moiety is a photo-cleavable blocking group, the enzyme is a polymerase, and the change is an extension of a primer sequence in the presence of dATP, dCTP, dTTP, and dGTP on the template sequence.

2. The method of claim 1, further comprising ligating adjacent detection tags after irradiating the selected region.

3. The method of claim 1, wherein the photo-cleavable moiety includes a caged-ATP molecule and the caged-ATP molecule is converted to an ATP molecule capable of modifying a probe present on the sample with a phosphate group.

4. The method of claim 3, further comprising modifying the phosphate group with a detectable label, a member of a specific binding pair, or a combination thereof, and detecting the detectable label or isolating the probe using the member of the specific binding pair.

5. The method of any of claims 1 to 4, wherein contacting includes multiple probes having specific binding moieties to multiple targets, the multiple different probes comprising unique detection tags and unique specific binding moieties and detecting the detection tag includes detecting the multiple unique detection tags.

6. The method of any of claims 1 to 5, wherein selecting further comprises selecting additional regions; the method further comprising:
iteratively and separately irradiating the additional regions;
iteratively and separately detecting the detection tag from the additional selected regions; and
relating the detecting of the detection tags from the selected regions to the sample so as to create a molecular profile of the sample, wherein the molecular profile relates each selected area with the detection tags detected from the selected area across the sample.

7. The method of any of claims 1 to 6, wherein detecting the detection tag includes quantifying the number of targets in a sample.

8. The method of any of claims 1 to 7, wherein the specific binding moiety is selected from an amino acid, a peptide, a protein, an antibody, a nucleotide, an oligonucleotide, a polynucleotide, a primer, an aptamer, a binding variable region, a plasmid, DNA, RNA, miRNA, or combinations thereof.

9. The method of claim 1, wherein the method further comprises:
irradiating a second selected region of the sample to cause the photo-reactive moiety to react, thereby freeing the detection tag for further reaction from the second selected region of the sample; and
detecting the detection tag that was bound to the target within the second selected region of the sample.

10. The method of claim 1, wherein selecting the region of the sample comprises manually marking one or more portions of the sample or digitally marking one or more portions of the sample.

11. The method of any of claims 1 to 10, wherein the contextual information used for selecting is created using one or more of primary staining, secondary staining, fluorescence imaging, phase contrast imaging, morphological sample characteristics imaging, image analysis, digital image analysis, cell selection, or grouping selection.

12. The method of claim 1, wherein detection of the detection tag further comprises sequencing the detection tag using next generation sequencing, analyzing the detection tag using PCR, or analyzing the detection tag using an array platform.

13. The method according to claim 1, further comprising contacting the sample with a second probe comprising a second specific binding moiety, a second photo-reactive moiety, and a second detection tag, wherein the photo-reactive moiety and the second photo-reactive moiety are the same, the detection tag and the second detection tag are different, and the specific binding moiety and the second specific binding moiety are different.

## Patentansprüche

1. Verfahren zur Durchführung kontextueller molekularer Diagnostik in einer Gewebeprobe, wobei das Verfahren Folgendes umfasst:
Inkontaktbringen der Gewebeprobe mit einer Sonde, umfassend eine photoreaktive Einheit, eine spezifische, an ein Ziel bindende Einheit und einen Nachweismarker, unter Verwendung von Bedingungen, die ausreichen, um die Bindung der spezifischen Bindungseinheit an ein Ziel in der Probe zu erleichtern;
Entfernen von Sonde, die nicht an die Probe bindet;
Zugeben einer Matrizensequenz zu der Gewebeprobe, wobei die Matrizensequenz konfiguriert ist, um spezifisch mit dem Nachweismarker zu hybridisieren;
Auswählen einer Region der Probe zur Bestrahlung, basierend auf kontextueller Information;
Bestrahlen der ausgewählten Region der Gewebeprobe mit Licht einer Wellenlänge und einer Intensität, die ausreichen, um die Reaktion der photoreaktiven Einheit zu bewirken, wodurch der Nachweismarker von der ausgewählten Region der Gewebeprobe für eine weitere Reaktion befreit wird;
Aussetzen der Gewebeprobe einem Enzym, das auf den Nachweismarker agiert, um eine Veränderung zu verursachen; und Nachweisen der Veränderung, wobei der Nachweismarker ein verlängerbarer Primer ist, die photoreaktive Einheit eine photospaltbare blockierende Gruppe ist, das Enzym eine Polymerase ist und die Veränderung eine Verlängerung einer Primersequenz in Anwesenheit von dATP, dCTP, dTTP und dGTP auf der Matrizensequenz ist.

2. Verfahren nach Anspruch 1, ferner umfassend das Ligieren benachbarter Nachweismarker nach dem Bestrahlen der ausgewählten Region.

3. Verfahren nach Anspruch 1, wobei die photospaltbare Einheit ein caged-ATP-Molekül einschließt und das caged-ATP-Molekül in ein ATP-Molekül umgewandelt wird, das in der Lage ist, eine auf der Probe vorhandene Sonde mit einer Phosphatgruppe zu modifizieren.

4. Verfahren nach Anspruch 3, ferner umfassend das Modifizieren der Phosphatgruppe mit einer nachweisbaren Markierung, einem Mitglied eines spezifischen Bindungspaars oder einer Kombination davon, und das Nachweisen der nachweisbaren Markierung oder das Isolieren der Sonde unter Verwendung des Mitglieds des spezifischen Bindungspaars.

5. Verfahren nach einem der Ansprüche 1 bis 4, wobei das Inkontaktbringen mehrere Sonden einschließt, die spezifische, an mehrere Ziele bindende Einheiten aufweisen, wobei die mehreren verschiedenen Sonden einzigartige Nachweismarker und einzigartige spezifische Bindungseinheiten umfassen und das Nachweisen des Nachweismarkers das Nachweisen der mehreren einzigartigen Nachweismarker einschließt.

6. Verfahren nach einem der Ansprüche 1 bis 5, wobei das Auswählen ferner das Auswählen zusätzlicher Regionen umfasst; wobei das Verfahren ferner Folgendes umfasst:
iteratives und getrenntes Bestrahlen der zusätzlichen Regionen;
iteratives und getrenntes Nachweisen des Nachweismarkers von den zusätzlichen ausgewählten Regionen; und
in Beziehung bringen des Nachweisens der Nachweismarker von den ausgewählten Regionen mit der Probe, sodass ein molekulares Profil der Probe erzeugt wird, wobei das molekulare Profil jeden ausgewählten Bereich mit den von dem ausgewählten Bereich der über die gesamte Probe nachgewiesenen Nachweismarker in Beziehung bringt.

7. Verfahren nach einem der Ansprüche 1 bis 6, wobei das Nachweisen des Nachweismarkers das Quantifizieren der Anzahl an Zielen in einer Probe einschließt.

8. Verfahren nach einem der Ansprüche 1 bis 7, wobei die spezifische Bindungseinheit ausgewählt ist aus einer Aminosäure, einem Peptid, einem Protein, einem Antikörper, einem Nukleotid, einem Oligonukleotid, einem Polynukleotid, einem Primer, einem Aptamer, einer variablen Bindungsregion, einem Plasmid, DNS, RNS, miRNS oder Kombinationen davon.

9. Verfahren nach Anspruch 1, wobei das Verfahren ferner Folgendes umfasst:
Bestrahlen einer zweiten ausgewählten Region der Probe, um die Reaktion der photoreaktiven Einheit zu bewirken, wodurch der Nachweismarker von der zweiten ausgewählten Region der Probe für eine weitere Reaktion befreit wird; und
Nachweisen des an das Ziel gebundenen Nachweismarkers innerhalb der zweiten ausgewählten Region der Probe.

10. Verfahren nach Anspruch 1, wobei das Auswählen der Region der Probe das manuelle Markieren eines oder mehrerer Abschnitte der Probe oder das digitale Markieren eines oder mehrerer Abschnitte der Probe umfasst.

11. Verfahren nach einem der Ansprüche 1 bis 10, wobei die für das Auswählen verwendete kontextuelle Information unter Verwendung von einer oder mehreren aus Primärfärbung, Sekundärfärbung, Fluoreszenzbildgebung, Phasenkontrast-Bildgebung, Bildgebung von morphologischen Probeneigenschaften, Bildanalyse, digitaler Bildanalyse, Zellenauswahl oder Gruppierungsauswahl erzeugt wird.

12. Verfahren nach Anspruch 1, wobei der Nachweis des Nachweismarkers ferner das Sequenzieren des Nachweismarkers unter Verwendung von Sequenzieren der nächsten Generation, das Analysieren des Nachweismarkers unter Verwendung von PCR oder das Analysieren des Nachweismarkers unter Verwendung einer Arrayplattform umfasst.

13. Verfahren nach Anspruch 1, ferner umfassend das Inkontaktbringen der Probe mit einer zweiten Sonde, umfassend eine zweite spezifische Bindungseinheit, eine zweite photoreaktive Einheit und einen zweiten Nachweismarker, wobei die photoreaktive Einheit und die zweite photoreaktive Einheit gleich sind, der Nachweismarker und der zweite Nachweismarker verschieden sind und die spezifische Bindungseinheit und die zweite spezifische Bindungseinheit verschieden sind.

## Revendications

1. Procédé permettant d'effectuer des diagnostics moléculaires contextuels dans un échantillon de tissu, le procédé comprenant :
la mise en contact de l'échantillon de tissu avec une sonde comprenant une fraction photo-réactive, une fraction de liaison spécifique à une cible, et une étiquette de détection en utilisant des conditions suffisantes pour faciliter la liaison de la fraction de liaison spécifique à une cible dans l'échantillon ;
la suppression de la sonde qui ne se lie pas à l'échantillon ;
l'ajout d'une séquence modèle à l'échantillon de tissu, dans lequel la séquence modèle est conçue pour s'hybrider spécifiquement à l'étiquette de détection ;
la sélection d'une région de l'échantillon pour une irradiation sur la base d'informations contextuelles ;
l'irradiation de la région sélectionnée de l'échantillon de tissu avec une lumière d'une longueur d'onde et d'une intensité suffisantes pour amener la fraction photo-réactive à réagir, libérant ainsi l'étiquette de détection de la région sélectionnée de l'échantillon de tissu pour une réaction ultérieure ;
l'exposition de l'échantillon de tissu à une enzyme qui agit sur l'étiquette de détection pour effectuer un changement ; et la détection du changement, dans laquelle l'étiquette de détection est une amorce extensible, la fraction photo-réactive est un groupe de blocage photo-clivable, ladite enzyme est une polymérase, et ledit changement est une extension d'une séquence d'amorce en présence de dATP, dCTP, dTTP et dGTP sur la séquence modèle.

2. Procédé selon la revendication 1, comprenant en outre la ligature d'étiquettes de détection adjacentes après irradiation de la région sélectionnée.

3. Procédé selon la revendication 1, dans lequel la fraction photo-clivable comprend une molécule d'ATP cagé et la molécule d'ATP cagé est convertie en une molécule d'ATP capable de modifier une sonde présente sur l'échantillon avec un groupe phosphate.

4. Procédé selon la revendication 3, comprenant en outre la modification du groupe phosphate avec un marqueur détectable, un élément d'une paire de liaison spécifique, ou une combinaison de ceux-ci, et la détection du marqueur détectable ou l'isolement de la sonde en utilisant l'élément de la paire de liaison spécifique.

5. Procédé selon l'une quelconque des revendications 1 à 4, dans lequel la mise en contact comprend de multiples sondes comportant des fractions de liaison spécifiques à de multiples cibles, les multiples sondes différentes comprenant des étiquettes de détection uniques et des fractions de liaison spécifiques uniques et la détection de l'étiquette de détection comprend la détection de multiples étiquettes de détection uniques.

6. Procédé selon l'une quelconque des revendications 1 à 5, dans lequel la sélection comprend en outre la sélection de régions supplémentaires ; le procédé comprenant en outre :
l'irradiation de manière itérative et séparée des régions supplémentaires ;
la détection de manière itérative et séparée de l'étiquette de détection à partir des régions sélectionnées supplémentaires ; et
la corrélation de la détection des étiquettes de détection des régions sélectionnées avec l'échantillon de façon à créer un profil moléculaire de l'échantillon, dans laquelle le profil moléculaire se rapporte à chaque zone sélectionnée avec les étiquettes de détection détectées à partir de la zone sélectionnée à travers l'échantillon.

7. Procédé selon l'une quelconque des revendications 1 à 6, dans lequel la détection de l'étiquette de détection comprend la quantification du nombre de cibles dans un échantillon.

8. Procédé selon l'une quelconque des revendications 1 à 7, dans lequel la fraction de liaison spécifique est choisie parmi un acide aminé, un peptide, une protéine, un anticorps, un nucléotide, un oligonucléotide, un polynucléotide, une amorce, un aptamère, une région variable de liaison, un plasmide, un ADN, un ARN, un miARN, ou des combinaisons de ceux-ci.

9. Procédé selon la revendication 1, dans lequel le procédé comprend en outre :
l'irradiation d'une seconde région sélectionnée de l'échantillon pour amener la fraction photo-réactive à réagir, libérant ainsi l'étiquette de détection pour une réaction ultérieure à partir de la seconde région sélectionnée de l'échantillon ; et
la détection de l'étiquette de détection qui a été liée à la cible au sein de la seconde région sélectionnée de l'échantillon.

10. Procédé selon la revendication 1, dans lequel la sélection de la région de l'échantillon comprend le marquage manuel d'une ou plusieurs parties de l'échantillon ou le marquage numérique d'une ou plusieurs parties de l'échantillon.

11. Procédé selon l'une quelconque des revendications 1 à 10, dans lequel les informations contextuelles utilisées pour la sélection sont créées en utilisant une ou plusieurs d'une coloration primaire, d'une coloration secondaire, d'une imagerie par fluorescence, d'une imagerie à contraste de phase, d'une imagerie des caractéristiques morphologiques de l'échantillon, d'une analyse d'image, d'une analyse d'image numérique, d'une sélection de cellule, ou d'une sélection de groupement.

12. Procédé selon la revendication 1, dans lequel la détection de l'étiquette de sélection comprend en outre le séquençage de l'étiquette de détection en utilisant un séquençage nouvelle génération, l'analyse de l'étiquette de détection en utilisant une PCR, ou l'analyse de l'étiquette de détection en utilisant une plateforme de réseau.

13. Procédé selon la revendication 1, comprenant en outre la mise en contact de l'échantillon avec une seconde sonde comprenant une seconde fraction de liaison spécifique, une seconde fraction photo-réactive, et une seconde étiquette de détection, dans laquelle la fraction photo-réactive et la seconde fraction photo-réactive sont identiques, l'étiquette de détection et ladite seconde étiquette de détection sont différentes, et la fraction de liaison spécifique et la seconde fraction de liaison spécifique sont différentes.


### SEQUENCE LISTING

<110> Ventana Medical Systems, Inc.
<120> PHOTO-SELECTIVE METHOD FOR BIOLOGICAL SAMPLE ANALYSIS
<130> 32014-WO
<150> US61/944996
   <151> 2014-02-26
<160> 48
<170> PatentIn version 3.5
<210> 1
   <211> 26
   <212> DNA
   <213> Homo sapiens
<400> 1
   atggtatcct tacatcgtca tttatc 26
<210> 2
   <211> 84
   <212> DNA
   <213> Homo sapiens
<400> 2
   cctctctatg ggcagtcggt gattcctatt cagatcacga tggtatcctt acatctgagt 60
   cggagacacg cagggatgag atgg 84
<210> 3
   <211> 78
   <212> DNA
   <213> Homo sapiens
<400> 3
   cctctctatg ggcagtcggt gattcctatt cagatggtat ccttacatct gagtcggaga 60
   cacgcaggga tgagatgg 78
<210> 4
   <211> 42
   <212> DNA
   <213> Homo sapiens
<400> 4
   cattcaaatc cccgagttga actttccttt caaagttcac gt 42
<210> 5
   <211> 25
   <212> DNA
   <213> Homo sapiens
<400> 5
   cgggaggcgg aggttgcagt gagcc 25
<210> 6
   <211> 17
   <212> DNA
   <213> Homo sapiens
<400> 6
   gtaaaacgac ggccagt 17
<210> 7
   <211> 17
   <212> DNA
   <213> Homo sapiens
<400> 7
   caggaaacag ctatgac 17
<210> 8
   <211> 1210
   <212> PRT
   <213> Homo sapiens
<400> 8
<210> 9
   <211> 5616
   <212> PRT
   <213> Homo sapiens
<400> 9
   Cys Cys Cys Cys Gly Gly Cys Gly Cys Ala Gly Cys Gly Cys Gly Gly
   1               5                   10                  15
   Cys Cys Gly Cys Ala Gly Cys Ala Gly Cys Cys Thr Cys Cys Gly Cys
               20                  25                  30
   Cys Cys Cys Cys Cys Gly Cys Ala Cys Gly Gly Thr Gly Thr Gly Ala
           35                  40                  45
   Gly Cys Gly Cys Cys Cys Gly Ala Cys Gly Cys Gly Gly Cys Cys Gly
       50                  55                  60
   Ala Gly Gly Cys Gly Gly Cys Cys Gly Gly Ala Gly Thr Cys Cys Cys
   65                  70                  75                  80
   Gly Ala Gly Cys Thr Ala Gly Cys Cys Cys Cys Gly Gly Cys Gly Gly
                   85                  90                  95
   Cys Cys Gly Cys Cys Gly Cys Cys Gly Cys Cys Cys Ala Gly Ala Cys
               100                 105                 110
   Cys Gly Gly Ala Cys Gly Ala Cys Ala Gly Gly Cys Cys Ala Cys Cys
           115                 120                 125
   Thr Cys Gly Thr Cys Gly Gly Cys Gly Thr Cys Cys Gly Cys Cys Cys
       130                 135                 140
   Gly Ala Gly Thr Cys Cys Cys Cys Gly Cys Cys Thr Cys Gly Cys Cys
   145 150 155 160
   Gly Cys Cys Ala Ala Cys Gly Cys Cys Ala Cys Ala Ala Cys Cys Ala
   165 170 175
   Cys Cys Gly Cys Gly Cys Ala Cys Gly Gly Cys Cys Cys Cys Cys Thr
   180 185 190
   Gly Ala Cys Thr Cys Cys Gly Thr Cys Cys Ala Gly Thr Ala Thr Thr
   195 200 205
   Gly Ala Thr Cys Gly Gly Gly Ala Gly Ala Gly Cys Cys Gly Gly Ala
   210 215 220
   Gly Cys Gly Ala Gly Cys Thr Cys Thr Thr Cys Gly Gly Gly Gly Ala
   225 230 235 240
   Gly Cys Ala Gly Cys Gly Ala Thr Gly Cys Gly Ala Cys Cys Cys Thr
   245 250 255
   Cys Cys Gly Gly Gly Ala Cys Gly Gly Cys Cys Gly Gly Gly Gly Cys
   260 265 270
   Ala Gly Cys Gly Cys Thr Cys Cys Thr Gly Gly Cys Gly Cys Thr Gly
   275 280 285
   Cys Thr Gly Gly Cys Thr Gly Cys Gly Cys Thr Cys Thr Gly Cys Cys
   290 295 300
   Cys Gly Gly Cys Gly Ala Gly Thr Cys Gly Gly Gly Cys Thr Cys Thr
   305 310 315 320
   Gly Gly Ala Gly Gly Ala Ala Ala Ala Gly Ala Ala Ala Gly Thr Thr
   325 330 335
   Thr Gly Cys Cys Ala Ala Gly Gly Cys Ala Cys Gly Ala Gly Thr Ala
   340 345 350
   Ala Cys Ala Ala Gly Cys Thr Cys Ala Cys Gly Cys Ala Gly Thr Thr
   355 360 365
   Gly Gly Gly Cys Ala Cys Thr Thr Thr Thr Gly Ala Ala Gly Ala Thr
   370 375 380
   Cys Ala Thr Thr Thr Thr Cys Thr Cys Ala Gly Cys Cys Thr Cys Cys
   385 390 395 400
   Ala Gly Ala Gly Gly Ala Thr Gly Thr Thr Cys Ala Ala Thr Ala Ala
                   405                 410                 415
   Cys Thr Gly Thr Gly Ala Gly Gly Thr Gly Gly Thr Cys Cys Thr Thr
               420                 425                 430
   Gly Gly Gly Ala Ala Thr Thr Thr Gly Gly Ala Ala Ala Thr Thr Ala
           435                 440                 445
   Cys Cys Thr Ala Thr Gly Thr Gly Cys Ala Gly Ala Gly Gly Ala Ala
       450                 455                 460
   Thr Thr Ala Thr Gly Ala Thr Cys Thr Thr Thr Cys Cys Thr Thr Cys
   465                 470                 475                 480
   Thr Thr Ala Ala Ala Gly Ala Cys Cys Ala Thr Cys Cys Ala Gly Gly
                   485                 490                 495
   Ala Gly Gly Thr Gly Gly Cys Thr Gly Gly Thr Thr Ala Thr Gly Thr
               500                 505                 510
   Cys Cys Thr Cys Ala Thr Thr Gly Cys Cys Cys Thr Cys Ala Ala Cys
           515                 520                 525
   Ala Cys Ala Gly Thr Gly Gly Ala Gly Cys Gly Ala Ala Thr Thr Cys
       530                 535                 540
   Cys Thr Thr Thr Gly Gly Ala Ala Ala Ala Cys Cys Thr Gly Cys Ala
   545                 550                 555                 560
   Gly Ala Thr Cys Ala Thr Cys Ala Gly Ala Gly Gly Ala Ala Ala Thr
                   565                 570                 575
   Ala Thr Gly Thr Ala Cys Thr Ala Cys Gly Ala Ala Ala Ala Thr Thr
               580                 585                 590
   Cys Cys Thr Ala Thr Gly Cys Cys Thr Thr Ala Gly Cys Ala Gly Thr
           595                 600                 605
   Cys Thr Thr Ala Thr Cys Thr Ala Ala Cys Thr Ala Thr Gly Ala Thr
       610                 615                 620
   Gly Cys Ala Ala Ala Thr Ala Ala Ala Ala Cys Cys Gly Gly Ala Cys
   625                 630                 635                 640
   Thr Gly Ala Ala Gly Gly Ala Gly Cys Thr Gly Cys Cys Cys Ala Thr
                   645                 650                 655
   Gly Ala Gly Ala Ala Ala Thr Thr Thr Ala Cys Ala Gly Gly Ala Ala
               660                 665                 670
   Ala Thr Cys Cys Thr Gly Cys Ala Thr Gly Gly Cys Gly Cys Cys Gly
           675                 680                 685
   Thr Gly Cys Gly Gly Thr Thr Cys Ala Gly Cys Ala Ala Cys Ala Ala
       690                 695                 700
   Cys Cys Cys Thr Gly Cys Cys Cys Thr Gly Thr Gly Cys Ala Ala Cys
   705                 710                 715                 720
   Gly Thr Gly Gly Ala Gly Ala Gly Cys Ala Thr Cys Cys Ala Gly Thr
                   725                 730                 735
   Gly Gly Cys Gly Gly Gly Ala Cys Ala Thr Ala Gly Thr Cys Ala Gly
               740                 745                 750
   Cys Ala Gly Thr Gly Ala Cys Thr Thr Thr Cys Thr Cys Ala Gly Cys
           755                 760                 765
   Ala Ala Cys Ala Thr Gly Thr Cys Gly Ala Thr Gly Gly Ala Cys Thr
       770                 775                 780
   Thr Cys Cys Ala Gly Ala Ala Cys Cys Ala Cys Cys Thr Gly Gly Gly
   785                 790                 795                 800
   Cys Ala Gly Cys Thr Gly Cys Cys Ala Ala Ala Ala Gly Thr Gly Thr
                   805                 810                 815
   Gly Ala Thr Cys Cys Ala Ala Gly Cys Thr Gly Thr Cys Cys Cys Ala
               820                 825                 830
   Ala Thr Gly Gly Gly Ala Gly Cys Thr Gly Cys Thr Gly Gly Gly Gly
           835                 840                 845
   Thr Gly Cys Ala Gly Gly Ala Gly Ala Gly Gly Ala Gly Ala Ala Cys
       850                 855                 860
   Thr Gly Cys Cys Ala Gly Ala Ala Ala Cys Thr Gly Ala Cys Cys Ala
   865                 870                 875                 880
   Ala Ala Ala Thr Cys Ala Thr Cys Thr Gly Thr Gly Cys Cys Cys Ala
                   885                 890                 895
   Gly Cys Ala Gly Thr Gly Cys Thr Cys Cys Gly Gly Gly Cys Gly Cys
               900                 905                 910
   Thr Gly Cys Cys Gly Thr Gly Gly Cys Ala Ala Gly Thr Cys Cys Cys
   915 920 925
   Cys Cys Ala Gly Thr Gly Ala Cys Thr Gly Cys Thr Gly Cys Cys Ala
   930 935 940
   Cys Ala Ala Cys Cys Ala Gly Thr Gly Thr Gly Cys Thr Gly Cys Ala
   945 950 955 960
   Gly Gly Cys Thr Gly Cys Ala Cys Ala Gly Gly Cys Cys Cys Cys Cys
   965 970 975
   Gly Gly Gly Ala Gly Ala Gly Cys Gly Ala Cys Thr Gly Cys Cys Thr
   980 985 990
   Gly Gly Thr Cys Thr Gly Cys Cys Gly Cys Ala Ala Ala Thr Thr Cys
   995 1000 1005
   Cys Gly Ala Gly Ala Cys Gly Ala Ala Gly Cys Cys Ala Cys Gly
   1010 1015 1020
   Thr Gly Cys Ala Ala Gly Gly Ala Cys Ala Cys Cys Thr Gly Cys
   1025 1030 1035
   Cys Cys Cys Cys Cys Ala Cys Thr Cys Ala Thr Gly Cys Thr Cys
   1040 1045 1050
   Thr Ala Cys Ala Ala Cys Cys Cys Cys Ala Cys Cys Ala Cys Gly
   1055 1060 1065
   Thr Ala Cys Cys Ala Gly Ala Thr Gly Gly Ala Thr Gly Thr Gly
   1070 1075 1080
   Ala Ala Cys Cys Cys Cys Gly Ala Gly Gly Gly Cys Ala Ala Ala
   1085 1090 1095
   Thr Ala Cys Ala Gly Cys Thr Thr Thr Gly Gly Thr Gly Cys Cys
   1100 1105 1110
   Ala Cys Cys Thr Gly Cys Gly Thr Gly Ala Ala Gly Ala Ala Gly
   1115 1120 1125
   Thr Gly Thr Cys Cys Cys Cys Gly Thr Ala Ala Thr Thr Ala Thr
   1130 1135 1140
   Gly Thr Gly Gly Thr Gly Ala Cys Ala Gly Ala Thr Cys Ala Cys
   1145 1150 1155
   Gly Gly Cys Thr Cys Gly Thr Gly Cys Gly Thr Cys Cys Gly Ala
   1160 1165 1170
   Gly Cys Cys Thr Gly Thr Gly Gly Gly Gly Cys Cys Gly Ala Cys
   1175 1180 1185
   Ala Gly Cys Thr Ala Thr Gly Ala Gly Ala Thr Gly Gly Ala Gly
   1190 1195 1200
   Gly Ala Ala Gly Ala Cys Gly Gly Cys Gly Thr Cys Cys Gly Cys
   1205 1210 1215
   Ala Ala Gly Thr Gly Thr Ala Ala Gly Ala Ala Gly Thr Gly Cys
   1220 1225 1230
   Gly Ala Ala Gly Gly Gly Cys Cys Thr Thr Gly Cys Cys Gly Cys
   1235 1240 1245
   Ala Ala Ala Gly Thr Gly Thr Gly Thr Ala Ala Cys Gly Gly Ala
   1250 1255 1260
   Ala Thr Ala Gly Gly Thr Ala Thr Thr Gly Gly Thr Gly Ala Ala
   1265 1270 1275
   Thr Thr Thr Ala Ala Ala Gly Ala Cys Thr Cys Ala Cys Thr Cys
   1280 1285 1290
   Thr Cys Cys Ala Thr Ala Ala Ala Thr Gly Cys Thr Ala Cys Gly
   1295 1300 1305
   Ala Ala Thr Ala Thr Thr Ala Ala Ala Cys Ala Cys Thr Thr Cys
   1310 1315 1320
   Ala Ala Ala Ala Ala Cys Thr Gly Cys Ala Cys Cys Thr Cys Cys
   1325 1330 1335
   Ala Thr Cys Ala Gly Thr Gly Gly Cys Gly Ala Thr Cys Thr Cys
   1340 1345 1350
   Cys Ala Cys Ala Thr Cys Cys Thr Gly Cys Cys Gly Gly Thr Gly
   1355 1360 1365
   Gly Cys Ala Thr Thr Thr Ala Gly Gly Gly Gly Thr Gly Ala Cys
   1370 1375 1380
   Thr Cys  Cys Thr Thr Cys Ala  Cys Ala Cys Ala Thr  Ala Cys Thr
       1385                 1390                 1395
   Cys Cys  Thr Cys Cys Thr Cys  Thr Gly Gly Ala Thr  Cys Cys Ala
       1400                 1405                 1410
   Cys Ala  Gly Gly Ala Ala Cys  Thr Gly Gly Ala Thr  Ala Thr Thr
       1415                 1420                 1425
   Cys Thr  Gly Ala Ala Ala Ala  Cys Cys Gly Thr Ala  Ala Ala Gly
       1430                 1435                 1440
   Gly Ala  Ala Ala Thr Cys Ala  Cys Ala Gly Gly Gly  Thr Thr Thr
       1445                 1450                 1455
   Thr Thr  Gly Cys Thr Gly Ala  Thr Thr Cys Ala Gly  Gly Cys Thr
       1460                 1465                 1470
   Thr Gly  Gly Cys Cys Thr Gly  Ala Ala Ala Ala Cys  Ala Gly Gly
       1475                 1480                 1485
   Ala Cys  Gly Gly Ala Cys Cys  Thr Cys Cys Ala Thr  Gly Cys Cys
       1490                 1495                 1500
   Thr Thr  Thr Gly Ala Gly Ala  Ala Cys Cys Thr Ala  Gly Ala Ala
       1505                 1510                 1515
   Ala Thr  Cys Ala Thr Ala Cys  Gly Cys Gly Gly Cys  Ala Gly Gly
       1520                 1525                 1530
   Ala Cys  Cys Ala Ala Gly Cys  Ala Ala Cys Ala Thr  Gly Gly Thr
       1535                 1540                 1545
   Cys Ala  Gly Thr Thr Thr Thr  Cys Thr Cys Thr Thr  Gly Cys Ala
       1550                 1555                 1560
   Gly Thr  Cys Gly Thr Cys Ala  Gly Cys Cys Thr Gly  Ala Ala Cys
       1565                 1570                 1575
   Ala Thr  Ala Ala Cys Ala Thr  Cys Cys Thr Thr Gly  Gly Gly Ala
       1580                 1585                 1590
   Thr Thr  Ala Cys Gly Cys Thr  Cys Cys Cys Thr Cys  Ala Ala Gly
       1595                 1600                 1605
   Gly Ala  Gly Ala Thr Ala Ala  Gly Thr Gly Ala Thr  Gly Gly Ala
       1610                 1615                 1620
   Gly Ala Thr Gly Thr Gly Ala Thr Ala Ala Thr Thr Thr Cys Ala
   1625 1630 1635
   Gly Gly Ala Ala Ala Cys Ala Ala Ala Ala Ala Thr Thr Thr Gly
   1640 1645 1650
   Thr Gly Cys Thr Ala Thr Gly Cys Ala Ala Ala Thr Ala Cys Ala
   1655 1660 1665
   Ala Thr Ala Ala Ala Cys Thr Gly Gly Ala Ala Ala Ala Ala Ala
   1670 1675 1680
   Cys Thr Gly Thr Thr Thr Gly Gly Gly Ala Cys Cys Thr Cys Cys
   1685 1690 1695
   Gly Gly Thr Cys Ala Gly Ala Ala Ala Ala Cys Cys Ala Ala Ala
   1700 1705 1710
   Ala Thr Thr Ala Thr Ala Ala Gly Cys Ala Ala Cys Ala Gly Ala
   1715 1720 1725
   Gly Gly Thr Gly Ala Ala Ala Ala Cys Ala Gly Cys Thr Gly Cys
   1730 1735 1740
   Ala Ala Gly Gly Cys Cys Ala Cys Ala Gly Gly Cys Cys Ala Gly
   1745 1750 1755
   Gly Thr Cys Thr Gly Cys Cys Ala Thr Gly Cys Cys Thr Thr Gly
   1760 1765 1770
   Thr Gly Cys Thr Cys Cys Cys Cys Cys Gly Ala Gly Gly Gly Cys
   1775 1780 1785
   Thr Gly Cys Thr Gly Gly Gly Gly Cys Cys Cys Gly Gly Ala Gly
   1790 1795 1800
   Cys Cys Cys Ala Gly Gly Gly Ala Cys Thr Gly Cys Gly Thr Cys
   1805 1810 1815
   Thr Cys Thr Thr Gly Cys Cys Gly Gly Ala Ala Thr Gly Thr Cys
   1820 1825 1830
   Ala Gly Cys Cys Gly Ala Gly Gly Cys Ala Gly Gly Gly Ala Ala
   1835 1840 1845
   Thr Gly Cys Gly Thr Gly Gly Ala Cys Ala Ala Gly Thr Gly Cys
   1850 1855 1860
   Ala Ala  Cys Cys Thr Thr Cys  Thr Gly Gly Ala Gly  Gly Gly Thr
       1865                 1870                 1875
   Gly Ala  Gly Cys Cys Ala Ala  Gly Gly Gly Ala Gly  Thr Thr Thr
       1880                 1885                 1890
   Gly Thr  Gly Gly Ala Gly Ala  Ala Cys Thr Cys Thr  Gly Ala Gly
       1895                 1900                 1905
   Thr Gly  Cys Ala Thr Ala Cys  Ala Gly Thr Gly Cys  Cys Ala Cys
       1910                 1915                 1920
   Cys Cys  Ala Gly Ala Gly Thr  Gly Cys Cys Thr Gly  Cys Cys Thr
       1925                 1930                 1935
   Cys Ala  Gly Gly Cys Cys Ala  Thr Gly Ala Ala Cys  Ala Thr Cys
       1940                 1945                 1950
   Ala Cys  Cys Thr Gly Cys Ala  Cys Ala Gly Gly Ala  Cys Gly Gly
       1955                 1960                 1965
   Gly Gly  Ala Cys Cys Ala Gly  Ala Cys Ala Ala Cys  Thr Gly Thr
       1970                 1975                 1980
   Ala Thr  Cys Cys Ala Gly Thr  Gly Thr Gly Cys Cys  Cys Ala Cys
       1985                 1990                 1995
   Thr Ala  Cys Ala Thr Thr Gly  Ala Cys Gly Gly Cys  Cys Cys Cys
       2000                 2005                 2010
   Cys Ala  Cys Thr Gly Cys Gly  Thr Cys Ala Ala Gly  Ala Cys Cys
       2015                 2020                 2025
   Thr Gly  Cys Cys Cys Gly Gly  Cys Ala Gly Gly Ala  Gly Thr Cys
       2030                 2035                 2040
   Ala Thr  Gly Gly Gly Ala Gly  Ala Ala Ala Ala Cys  Ala Ala Cys
       2045                 2050                 2055
   Ala Cys  Cys Cys Thr Gly Gly  Thr Cys Thr Gly Gly  Ala Ala Gly
       2060                 2065                 2070
   Thr Ala  Cys Gly Cys Ala Gly  Ala Cys Gly Cys Cys  Gly Gly Cys
       2075                 2080                 2085
   Cys Ala  Thr Gly Thr Gly Thr  Gly Cys Cys Ala Cys  Cys Thr Gly
   2090 2095 2100
   Thr Gly Cys Cys Ala Thr Cys Cys Ala Ala Ala Cys Thr Gly Cys
   2105 2110 2115
   Ala Cys Cys Thr Ala Cys Gly Gly Ala Thr Gly Cys Ala Cys Thr
   2120 2125 2130
   Gly Gly Gly Cys Cys Ala Gly Gly Thr Cys Thr Thr Gly Ala Ala
   2135 2140 2145
   Gly Gly Cys Thr Gly Thr Cys Cys Ala Ala Cys Gly Ala Ala Thr
   2150 2155 2160
   Gly Gly Gly Cys Cys Thr Ala Ala Gly Ala Thr Cys Cys Cys Gly
   2165 2170 2175
   Thr Cys Cys Ala Thr Cys Gly Cys Cys Ala Cys Thr Gly Gly Gly
   2180 2185 2190
   Ala Thr Gly Gly Thr Gly Gly Gly Gly Gly Cys Cys Cys Thr Cys
   2195 2200 2205
   Cys Thr Cys Thr Thr Gly Cys Thr Gly Cys Thr Gly Gly Thr Gly
   2210 2215 2220
   Gly Thr Gly Gly Cys Cys Cys Thr Gly Gly Gly Gly Ala Thr Cys
   2225 2230 2235
   Gly Gly Cys Cys Thr Cys Thr Thr Cys Ala Thr Gly Cys Gly Ala
   2240 2245 2250
   Ala Gly Gly Cys Gly Cys Cys Ala Cys Ala Thr Cys Gly Thr Thr
   2255 2260 2265
   Cys Gly Gly Ala Ala Gly Cys Gly Cys Ala Cys Gly Cys Thr Gly
   2270 2275 2280
   Cys Gly Gly Ala Gly Gly Cys Thr Gly Cys Thr Gly Cys Ala Gly
   2285 2290 2295
   Gly Ala Gly Ala Gly Gly Gly Ala Gly Cys Thr Thr Gly Thr Gly
   2300 2305 2310
   Gly Ala Gly Cys Cys Thr Cys Thr Thr Ala Cys Ala Cys Cys Cys
   2315 2320 2325
   Ala Gly Thr Gly Gly Ala Gly Ala Ala Gly Cys Thr Cys Cys Cys
   2330 2335 2340
   Ala Ala Cys Cys Ala Ala Gly Cys Thr Cys Thr Cys Thr Thr Gly
   2345 2350 2355
   Ala Gly Gly Ala Thr Cys Thr Thr Gly Ala Ala Gly Gly Ala Ala
   2360 2365 2370
   Ala Cys Thr Gly Ala Ala Thr Thr Cys Ala Ala Ala Ala Ala Gly
   2375 2380 2385
   Ala Thr Cys Ala Ala Ala Gly Thr Gly Cys Thr Gly Gly Gly Cys
   2390 2395 2400
   Thr Cys Cys Gly Gly Thr Gly Cys Gly Thr Thr Cys Gly Gly Cys
   2405 2410 2415
   Ala Cys Gly Gly Thr Gly Thr Ala Thr Ala Ala Gly Gly Gly Ala
   2420 2425 2430
   Cys Thr Cys Thr Gly Gly Ala Thr Cys Cys Cys Ala Gly Ala Ala
   2435 2440 2445
   Gly Gly Thr Gly Ala Gly Ala Ala Ala Gly Thr Thr Ala Ala Ala
   2450 2455 2460
   Ala Thr Thr Cys Cys Cys Gly Thr Cys Gly Cys Thr Ala Thr Cys
   2465 2470 2475
   Ala Ala Gly Gly Ala Ala Thr Thr Ala Ala Gly Ala Gly Ala Ala
   2480 2485 2490
   Gly Cys Ala Ala Cys Ala Thr Cys Thr Cys Cys Gly Ala Ala Ala
   2495 2500 2505
   Gly Cys Cys Ala Ala Cys Ala Ala Gly Gly Ala Ala Ala Thr Cys
   2510 2515 2520
   Cys Thr Cys Gly Ala Thr Gly Ala Ala Gly Cys Cys Thr Ala Cys
   2525 2530 2535
   Gly Thr Gly Ala Thr Gly Gly Cys Cys Ala Gly Cys Gly Thr Gly
   2540 2545 2550
   Gly Ala Cys Ala Ala Cys Cys Cys Cys Cys Ala Cys Gly Thr Gly
   2555 2560 2565
   Thr Gly  Cys Cys Gly Cys Cys  Thr Gly Cys Thr Gly  Gly Gly Cys
       2570                 2575                 2580
   Ala Thr  Cys Thr Gly Cys Cys  Thr Cys Ala Cys Cys  Thr Cys Cys
       2585                 2590                 2595
   Ala Cys  Cys Gly Thr Gly Cys  Ala Gly Cys Thr Cys  Ala Thr Cys
       2600                 2605                 2610
   Ala Cys  Gly Cys Ala Gly Cys  Thr Cys Ala Thr Gly  Cys Cys Cys
       2615                 2620                 2625
   Thr Thr  Cys Gly Gly Cys Thr  Gly Cys Cys Thr Cys  Cys Thr Gly
       2630                 2635                 2640
   Gly Ala  Cys Thr Ala Thr Gly  Thr Cys Cys Gly Gly  Gly Ala Ala
       2645                 2650                 2655
   Cys Ala  Cys Ala Ala Ala Gly  Ala Cys Ala Ala Thr  Ala Thr Thr
       2660                 2665                 2670
   Gly Gly  Cys Thr Cys Cys Cys  Ala Gly Thr Ala Cys  Cys Thr Gly
       2675                 2680                 2685
   Cys Thr  Cys Ala Ala Cys Thr  Gly Gly Thr Gly Thr  Gly Thr Gly
       2690                 2695                 2700
   Cys Ala  Gly Ala Thr Cys Gly  Cys Ala Ala Ala Gly  Gly Gly Cys
       2705                 2710                 2715
   Ala Thr  Gly Ala Ala Cys Thr  Ala Cys Thr Thr Gly  Gly Ala Gly
       2720                 2725                 2730
   Gly Ala  Cys Cys Gly Thr Cys  Gly Cys Thr Thr Gly  Gly Thr Gly
       2735                 2740                 2745
   Cys Ala  Cys Cys Gly Cys Gly  Ala Cys Cys Thr Gly  Gly Cys Ala
       2750                 2755                 2760
   Gly Cys  Cys Ala Gly Gly Ala  Ala Cys Gly Thr Ala  Cys Thr Gly
       2765                 2770                 2775
   Gly Thr  Gly Ala Ala Ala Ala  Cys Ala Cys Cys Gly  Cys Ala Gly
       2780                 2785                 2790
   Cys Ala  Thr Gly Thr Cys Ala  Ala Gly Ala Thr Cys  Ala Cys Ala
       2795                 2800                 2805
   Gly Ala  Thr Thr Thr Thr Gly  Gly Gly Cys Thr Gly  Gly Cys Cys
       2810                 2815                 2820
   Ala Ala  Ala Cys Thr Gly Cys  Thr Gly Gly Gly Thr  Gly Cys Gly
       2825                 2830                 2835
   Gly Ala  Ala Gly Ala Gly Ala  Ala Ala Gly Ala Ala  Thr Ala Cys
       2840                 2845                 2850
   Cys Ala  Thr Gly Cys Ala Gly  Ala Ala Gly Gly Ala  Gly Gly Cys
       2855                 2860                 2865
   Ala Ala  Ala Gly Thr Gly Cys  Cys Thr Ala Thr Cys  Ala Ala Gly
       2870                 2875                 2880
   Thr Gly  Gly Ala Thr Gly Gly  Cys Ala Thr Thr Gly  Gly Ala Ala
       2885                 2890                 2895
   Thr Cys  Ala Ala Thr Thr Thr  Thr Ala Cys Ala Cys  Ala Gly Ala
       2900                 2905                 2910
   Ala Thr  Cys Thr Ala Thr Ala  Cys Cys Cys Ala Cys  Cys Ala Gly
       2915                 2920                 2925
   Ala Gly  Thr Gly Ala Thr Gly  Thr Cys Thr Gly Gly  Ala Gly Cys
       2930                 2935                 2940
   Thr Ala  Cys Gly Gly Gly Gly  Thr Gly Ala Cys Cys  Gly Thr Thr
       2945                 2950                 2955
   Thr Gly  Gly Gly Ala Gly Thr  Thr Gly Ala Thr Gly  Ala Cys Cys
       2960                 2965                 2970
   Thr Thr  Thr Gly Gly Ala Thr  Cys Cys Ala Ala Gly  Cys Cys Ala
       2975                 2980                 2985
   Thr Ala  Thr Gly Ala Cys Gly  Gly Ala Ala Thr Cys  Cys Cys Thr
       2990                 2995                 3000
   Gly Cys  Cys Ala Gly Cys Gly  Ala Gly Ala Thr Cys  Thr Cys Cys
       3005                 3010                 3015
   Thr Cys  Cys Ala Thr Cys Cys  Thr Gly Gly Ala Gly  Ala Ala Ala
       3020                 3025                 3030
   Gly Gly  Ala Gly Ala Ala Cys  Gly Cys Cys Thr Cys  Cys Cys Thr
   3035 3040 3045
   Cys Ala Gly Cys Cys Ala Cys Cys Cys Ala Thr Ala Thr Gly Thr
   3050 3055 3060
   Ala Cys Cys Ala Thr Cys Gly Ala Thr Gly Thr Cys Thr Ala Cys
   3065 3070 3075
   Ala Thr Gly Ala Thr Cys Ala Thr Gly Gly Thr Cys Ala Ala Gly
   3080 3085 3090
   Thr Gly Cys Thr Gly Gly Ala Thr Gly Ala Thr Ala Gly Ala Cys
   3095 3100 3105
   Gly Cys Ala Gly Ala Thr Ala Gly Thr Cys Gly Cys Cys Cys Ala
   3110 3115 3120
   Ala Ala Gly Thr Thr Cys Cys Gly Thr Gly Ala Gly Thr Thr Gly
   3125 3130 3135
   Ala Thr Cys Ala Thr Cys Gly Ala Ala Thr Thr Cys Thr Cys Cys
   3140 3145 3150
   Ala Ala Ala Ala Thr Gly Gly Cys Cys Cys Gly Ala Gly Ala Cys
   3155 3160 3165
   Cys Cys Cys Cys Ala Gly Cys Gly Cys Thr Ala Cys Cys Thr Thr
   3170 3175 3180
   Gly Thr Cys Ala Thr Thr Cys Ala Gly Gly Gly Gly Gly Ala Thr
   3185 3190 3195
   Gly Ala Ala Ala Gly Ala Ala Thr Gly Cys Ala Thr Thr Thr Gly
   3200 3205 3210
   Cys Cys Ala Ala Gly Thr Cys Cys Thr Ala Cys Ala Gly Ala Cys
   3215 3220 3225
   Thr Cys Cys Ala Ala Cys Thr Thr Cys Thr Ala Cys Cys Gly Thr
   3230 3235 3240
   Gly Cys Cys Cys Thr Gly Ala Thr Gly Gly Ala Thr Gly Ala Ala
   3245 3250 3255
   Gly Ala Ala Gly Ala Cys Ala Thr Gly Gly Ala Cys Gly Ala Cys
   3260 3265 3270
   Gly Thr  Gly Gly Thr Gly Gly  Ala Thr Gly Cys Cys  Gly Ala Cys
       3275                 3280                 3285
   Gly Ala  Gly Thr Ala Cys Cys  Thr Cys Ala Thr Cys  Cys Cys Ala
       3290                 3295                 3300
   Cys Ala  Gly Cys Ala Gly Gly  Gly Cys Thr Thr Cys  Thr Thr Cys
       3305                 3310                 3315
   Ala Gly  Cys Ala Gly Cys Cys  Cys Cys Thr Cys Cys  Ala Cys Gly
       3320                 3325                 3330
   Thr Cys  Ala Cys Gly Gly Ala  Cys Thr Cys Cys Cys  Cys Thr Cys
       3335                 3340                 3345
   Cys Thr  Gly Ala Gly Cys Thr  Cys Thr Cys Thr Gly  Ala Gly Thr
       3350                 3355                 3360
   Gly Cys  Ala Ala Cys Cys Ala  Gly Cys Ala Ala Cys  Ala Ala Thr
       3365                 3370                 3375
   Thr Cys  Cys Ala Cys Cys Gly  Thr Gly Gly Cys Thr  Thr Gly Cys
       3380                 3385                 3390
   Ala Thr  Thr Gly Ala Thr Ala  Gly Ala Ala Ala Thr  Gly Gly Gly
       3395                 3400                 3405
   Cys Thr  Gly Cys Ala Ala Ala  Gly Cys Thr Gly Thr  Cys Cys Cys
       3410                 3415                 3420
   Ala Thr  Cys Ala Ala Gly Gly  Ala Ala Gly Ala Cys  Ala Gly Cys
       3425                 3430                 3435
   Thr Thr  Cys Thr Thr Gly Cys  Ala Gly Cys Gly Ala  Thr Ala Cys
       3440                 3445                 3450
   Ala Gly  Cys Thr Cys Ala Gly  Ala Cys Cys Cys Cys  Ala Cys Ala
       3455                 3460                 3465
   Gly Gly  Cys Gly Cys Cys Thr  Thr Gly Ala Cys Thr  Gly Ala Gly
       3470                 3475                 3480
   Gly Ala  Cys Ala Gly Cys Ala  Thr Ala Gly Ala Cys  Gly Ala Cys
       3485                 3490                 3495
   Ala Cys  Cys Thr Thr Cys Cys  Thr Cys Cys Cys Ala  Gly Thr Gly
       3500                 3505                 3510
   Cys Cys Thr Gly Ala Ala Thr Ala Cys Ala Thr Ala Ala Ala Cys
   3515 3520 3525
   Cys Ala Gly Thr Cys Cys Gly Thr Thr Cys Cys Cys Ala Ala Ala
   3530 3535 3540
   Ala Gly Gly Cys Cys Cys Gly Cys Thr Gly Gly Cys Thr Cys Thr
   3545 3550 3555
   Gly Thr Gly Cys Ala Gly Ala Ala Thr Cys Cys Thr Gly Thr Cys
   3560 3565 3570
   Thr Ala Thr Cys Ala Cys Ala Ala Thr Cys Ala Gly Cys Cys Thr
   3575 3580 3585
   Cys Thr Gly Ala Ala Cys Cys Cys Cys Gly Cys Gly Cys Cys Cys
   3590 3595 3600
   Ala Gly Cys Ala Gly Ala Gly Ala Cys Cys Cys Ala Cys Ala Cys
   3605 3610 3615
   Thr Ala Cys Cys Ala Gly Gly Ala Cys Cys Cys Cys Cys Ala Cys
   3620 3625 3630
   Ala Gly Cys Ala Cys Thr Gly Cys Ala Gly Thr Gly Gly Gly Cys
   3635 3640 3645
   Ala Ala Cys Cys Cys Cys Gly Ala Gly Thr Ala Thr Cys Thr Cys
   3650 3655 3660
   Ala Ala Cys Ala Cys Thr Gly Thr Cys Cys Ala Gly Cys Cys Cys
   3665 3670 3675
   Ala Cys Cys Thr Gly Thr Gly Thr Cys Ala Ala Cys Ala Gly Cys
   3680 3685 3690
   Ala Cys Ala Thr Thr Cys Gly Ala Cys Ala Gly Cys Cys Cys Thr
   3695 3700 3705
   Gly Cys Cys Cys Ala Cys Thr Gly Gly Gly Cys Cys Cys Ala Gly
   3710 3715 3720
   Ala Ala Ala Gly Gly Cys Ala Gly Cys Cys Ala Cys Cys Ala Ala
   3725 3730 3735
   Ala Thr Thr Ala Gly Cys Cys Thr Gly Gly Ala Cys Ala Ala Cys
   3740 3745 3750
   Cys Cys  Thr Gly Ala Cys Thr  Ala Cys Cys Ala Gly  Cys Ala Gly
       3755                 3760                 3765
   Gly Ala  Cys Thr Thr Cys Thr  Thr Thr Cys Cys Cys  Ala Ala Gly
       3770                 3775                 3780
   Gly Ala  Ala Gly Cys Cys Ala  Ala Gly Cys Cys Ala  Ala Ala Thr
       3785                 3790                 3795
   Gly Gly  Cys Ala Thr Cys Thr  Thr Thr Ala Ala Gly  Gly Gly Cys
       3800                 3805                 3810
   Thr Cys  Cys Ala Cys Ala Gly  Cys Thr Gly Ala Ala  Ala Ala Thr
       3815                 3820                 3825
   Gly Cys  Ala Gly Ala Ala Thr  Ala Cys Cys Thr Ala  Ala Gly Gly
       3830                 3835                 3840
   Gly Thr  Cys Gly Cys Gly Cys  Cys Ala Cys Ala Ala  Ala Gly Cys
       3845                 3850                 3855
   Ala Gly  Thr Gly Ala Ala Thr  Thr Thr Ala Thr Thr  Gly Gly Ala
       3860                 3865                 3870
   Gly Cys  Ala Thr Gly Ala Cys  Cys Ala Cys Gly Gly  Ala Gly Gly
       3875                 3880                 3885
   Ala Thr  Ala Gly Thr Ala Thr  Gly Ala Gly Cys Cys  Cys Thr Ala
       3890                 3895                 3900
   Ala Ala  Ala Ala Thr Cys Cys  Ala Gly Ala Cys Thr  Cys Thr Thr
       3905                 3910                 3915
   Thr Cys  Gly Ala Thr Ala Cys  Cys Cys Ala Gly Gly  Ala Cys Cys
       3920                 3925                 3930
   Ala Ala  Gly Cys Cys Ala Cys  Ala Gly Cys Ala Gly  Gly Thr Cys
       3935                 3940                 3945
   Cys Thr  Cys Cys Ala Thr Cys  Cys Cys Ala Ala Cys  Ala Gly Cys
       3950                 3955                 3960
   Cys Ala  Thr Gly Cys Cys Cys  Gly Cys Ala Thr Thr  Ala Gly Cys
       3965                 3970                 3975
   Thr Cys  Thr Thr Ala Gly Ala  Cys Cys Cys Ala Cys  Ala Gly Ala
   3980 3985 3990
   Cys Thr Gly Gly Thr Thr Thr Thr Gly Cys Ala Ala Cys Gly Thr
   3995 4000 4005
   Thr Thr Ala Cys Ala Cys Cys Gly Ala Cys Thr Ala Gly Cys Cys
   4010 4015 4020
   Ala Gly Gly Ala Ala Gly Thr Ala Cys Thr Thr Cys Cys Ala Cys
   4025 4030 4035
   Cys Thr Cys Gly Gly Gly Cys Ala Cys Ala Thr Thr Thr Thr Gly
   4040 4045 4050
   Gly Gly Ala Ala Gly Thr Thr Gly Cys Ala Thr Thr Cys Cys Thr
   4055 4060 4065
   Thr Thr Gly Thr Cys Thr Thr Cys Ala Ala Ala Cys Thr Gly Thr
   4070 4075 4080
   Gly Ala Ala Gly Cys Ala Thr Thr Thr Ala Cys Ala Gly Ala Ala
   4085 4090 4095
   Ala Cys Gly Cys Ala Thr Cys Cys Ala Gly Cys Ala Ala Gly Ala
   4100 4105 4110
   Ala Thr Ala Thr Thr Gly Thr Cys Cys Cys Thr Thr Thr Gly Ala
   4115 4120 4125
   Gly Cys Ala Gly Ala Ala Ala Thr Thr Thr Ala Thr Cys Thr Thr
   4130 4135 4140
   Thr Cys Ala Ala Ala Gly Ala Gly Gly Thr Ala Thr Ala Thr Thr
   4145 4150 4155
   Thr Gly Ala Ala Ala Ala Ala Ala Ala Ala Ala Ala Ala Ala Ala
   4160 4165 4170
   Gly Thr Ala Thr Ala Thr Gly Thr Gly Ala Gly Gly Ala Thr Thr
   4175 4180 4185
   Thr Thr Thr Ala Thr Thr Gly Ala Thr Thr Gly Gly Gly Gly Ala
   4190 4195 4200
   Thr Cys Thr Thr Gly Gly Ala Gly Thr Thr Thr Thr Thr Cys Ala
   4205 4210 4215
   Thr Thr Gly Thr Cys Gly Cys Thr Ala Thr Thr Gly Ala Thr Thr
   4220 4225 4230
   Thr Thr Thr Ala Cys Thr Thr Cys Ala Ala Thr Gly Gly Gly Cys
   4235 4240 4245
   Thr Cys Thr Thr Cys Cys Ala Ala Cys Ala Ala Gly Gly Ala Ala
   4250 4255 4260
   Gly Ala Ala Gly Cys Thr Thr Gly Cys Thr Gly Gly Thr Ala Gly
   4265 4270 4275
   Cys Ala Cys Thr Thr Gly Cys Thr Ala Cys Cys Cys Thr Gly Ala
   4280 4285 4290
   Gly Thr Thr Cys Ala Thr Cys Cys Ala Gly Gly Cys Cys Cys Ala
   4295 4300 4305
   Ala Cys Thr Gly Thr Gly Ala Gly Cys Ala Ala Gly Gly Ala Gly
   4310 4315 4320
   Cys Ala Cys Ala Ala Gly Cys Cys Ala Cys Ala Ala Gly Thr Cys
   4325 4330 4335
   Thr Thr Cys Cys Ala Gly Ala Gly Gly Ala Thr Gly Cys Thr Thr
   4340 4345 4350
   Gly Ala Thr Thr Cys Cys Ala Gly Thr Gly Gly Thr Thr Cys Thr
   4355 4360 4365
   Gly Cys Thr Thr Cys Ala Ala Gly Gly Cys Thr Thr Cys Cys Ala
   4370 4375 4380
   Cys Thr Gly Cys Ala Ala Ala Ala Cys Ala Cys Thr Ala Ala Ala
   4385 4390 4395
   Gly Ala Thr Cys Cys Ala Ala Gly Ala Ala Gly Gly Cys Cys Thr
   4400 4405 4410
   Thr Cys Ala Thr Gly Gly Cys Cys Cys Cys Ala Gly Cys Ala Gly
   4415 4420 4425
   Gly Cys Cys Gly Gly Ala Thr Cys Gly Gly Thr Ala Cys Thr Gly
   4430 4435 4440
   Thr Ala Thr Cys Ala Ala Gly Thr Cys Ala Thr Gly Gly Cys Ala
   4445 4450 4455
   Gly Gly Thr Ala Cys Ala Gly Thr Ala Gly Gly Ala Thr Ala Ala
   4460 4465 4470
   Gly Cys Cys Ala Cys Thr Cys Thr Gly Thr Cys Cys Cys Thr Thr
   4475 4480 4485
   Cys Cys Thr Gly Gly Gly Cys Ala Ala Ala Gly Ala Ala Gly Ala
   4490 4495 4500
   Ala Ala Cys Gly Gly Ala Gly Gly Gly Gly Ala Thr Gly Gly Ala
   4505 4510 4515
   Ala Thr Thr Cys Thr Thr Cys Cys Thr Thr Ala Gly Ala Cys Thr
   4520 4525 4530
   Thr Ala Cys Thr Thr Thr Thr Gly Thr Ala Ala Ala Ala Ala Thr
   4535 4540 4545
   Gly Thr Cys Cys Cys Cys Ala Cys Gly Gly Thr Ala Cys Thr Thr
   4550 4555 4560
   Ala Cys Thr Cys Cys Cys Cys Ala Cys Thr Gly Ala Thr Gly Gly
   4565 4570 4575
   Ala Cys Cys Ala Gly Thr Gly Gly Thr Thr Thr Cys Cys Ala Gly
   4580 4585 4590
   Thr Cys Ala Thr Gly Ala Gly Cys Gly Thr Thr Ala Gly Ala Cys
   4595 4600 4605
   Thr Gly Ala Cys Thr Thr Gly Thr Thr Thr Gly Thr Cys Thr Thr
   4610 4615 4620
   Cys Cys Ala Thr Thr Cys Cys Ala Thr Thr Gly Thr Thr Thr Thr
   4625 4630 4635
   Gly Ala Ala Ala Cys Thr Cys Ala Gly Thr Ala Thr Gly Cys Thr
   4640 4645 4650
   Gly Cys Cys Cys Cys Thr Gly Thr Cys Thr Thr Gly Cys Thr Gly
   4655 4660 4665
   Thr Cys Ala Thr Gly Ala Ala Ala Thr Cys Ala Gly Cys Ala Ala
   4670 4675 4680
   Gly Ala Gly Ala Gly Gly Ala Thr Gly Ala Cys Ala Cys Ala Thr
   4685 4690 4695
   Cys Ala  Ala Ala Thr Ala Ala  Thr Ala Ala Cys Thr  Cys Gly Gly
       4700                 4705                 4710
   Ala Thr  Thr Cys Cys Ala Gly  Cys Cys Cys Ala Cys  Ala Thr Thr
       4715                 4720                 4725
   Gly Gly  Ala Thr Thr Cys Ala  Thr Cys Ala Gly Cys  Ala Thr Thr
       4730                 4735                 4740
   Thr Gly  Gly Ala Cys Cys Ala  Ala Thr Ala Gly Cys  Cys Cys Ala
       4745                 4750                 4755
   Cys Ala  Gly Cys Thr Gly Ala  Gly Ala Ala Thr Gly  Thr Gly Gly
       4760                 4765                 4770
   Ala Ala  Thr Ala Cys Cys Thr  Ala Ala Gly Gly Ala  Thr Ala Gly
       4775                 4780                 4785
   Cys Ala  Cys Cys Gly Cys Thr  Thr Thr Thr Gly Thr  Thr Cys Thr
       4790                 4795                 4800
   Cys Gly  Cys Ala Ala Ala Ala  Ala Cys Gly Thr Ala  Thr Cys Thr
       4805                 4810                 4815
   Cys Cys  Thr Ala Ala Thr Thr  Thr Gly Ala Gly Gly  Cys Thr Cys
       4820                 4825                 4830
   Ala Gly  Ala Thr Gly Ala Ala  Ala Thr Gly Cys Ala  Thr Cys Ala
       4835                 4840                 4845
   Gly Gly  Thr Cys Cys Thr Thr  Thr Gly Gly Gly Gly  Cys Ala Thr
       4850                 4855                 4860
   Ala Gly  Ala Thr Cys Ala Gly  Ala Ala Gly Ala Cys  Thr Ala Cys
       4865                 4870                 4875
   Ala Ala  Ala Ala Ala Thr Gly  Ala Ala Gly Cys Thr  Gly Cys Thr
       4880                 4885                 4890
   Cys Thr  Gly Ala Ala Ala Thr  Cys Thr Cys Cys Thr  Thr Thr Ala
       4895                 4900                 4905
   Gly Cys  Cys Ala Thr Cys Ala  Cys Cys Cys Cys Ala  Ala Cys Cys
       4910                 4915                 4920
   Cys Cys  Cys Cys Ala Ala Ala  Ala Thr Thr Ala Gly  Thr Thr Thr
   4925 4930 4935
   Gly Thr Gly Thr Thr Ala Cys Thr Thr Ala Thr Gly Gly Ala Ala
   4940 4945 4950
   Gly Ala Thr Ala Gly Thr Thr Thr Thr Cys Thr Cys Cys Thr Thr
   4955 4960 4965
   Thr Thr Ala Cys Thr Thr Cys Ala Cys Thr Thr Cys Ala Ala Ala
   4970 4975 4980
   Ala Gly Cys Thr Thr Thr Thr Thr Ala Cys Thr Cys Ala Ala Ala
   4985 4990 4995
   Gly Ala Gly Thr Ala Thr Ala Thr Gly Thr Thr Cys Cys Cys Thr
   5000 5005 5010
   Cys Cys Ala Gly Gly Thr Cys Ala Gly Cys Thr Gly Cys Cys Cys
   5015 5020 5025
   Cys Cys Ala Ala Ala Cys Cys Cys Cys Cys Thr Cys Cys Thr Thr
   5030 5035 5040
   Ala Cys Gly Cys Thr Thr Thr Gly Thr Cys Ala Cys Ala Cys Ala
   5045 5050 5055
   Ala Ala Ala Ala Gly Thr Gly Thr Cys Thr Cys Thr Gly Cys Cys
   5060 5065 5070
   Thr Thr Gly Ala Gly Thr Cys Ala Thr Cys Thr Ala Thr Thr Cys
   5075 5080 5085
   Ala Ala Gly Cys Ala Cys Thr Thr Ala Cys Ala Gly Cys Thr Cys
   5090 5095 5100
   Thr Gly Gly Cys Cys Ala Cys Ala Ala Cys Ala Gly Gly Gly Cys
   5105 5110 5115
   Ala Thr Thr Thr Thr Ala Cys Ala Gly Gly Thr Gly Cys Gly Ala
   5120 5125 5130
   Ala Thr Gly Ala Cys Ala Gly Thr Ala Gly Cys Ala Thr Thr Ala
   5135 5140 5145
   Thr Gly Ala Gly Thr Ala Gly Thr Gly Thr Gly Gly Ala Ala Thr
   5150 5155 5160
   Thr Cys  Ala Gly Gly Thr Ala  Gly Thr Ala Ala Ala  Thr Ala Thr
       5165                5170                5175
   Gly Ala  Ala Ala Cys Thr Ala  Gly Gly Gly Thr Thr  Thr Gly Ala
       5180                5185                5190
   Ala Ala  Thr Thr Gly Ala Thr  Ala Ala Thr Gly Cys  Thr Thr Thr
       5195                5200                5205
   Cys Ala  Cys Ala Ala Cys Ala  Thr Thr Thr Gly Cys  Ala Gly Ala
       5210                5215                5220
   Thr Gly  Thr Thr Thr Thr Ala  Gly Ala Ala Gly Gly  Ala Ala Ala
       5225                5230                5235
   Ala Ala  Ala Gly Thr Thr Cys  Cys Thr Thr Cys Cys  Thr Ala Ala
       5240                5245                5250
   Ala Ala  Thr Ala Ala Thr Thr  Thr Cys Thr Cys Thr  Ala Cys Ala
       5255                5260                5265
   Ala Thr  Thr Gly Gly Ala Ala  Gly Ala Thr Thr Gly  Gly Ala Ala
       5270                5275                5280
   Gly Ala  Thr Thr Cys Ala Gly  Cys Thr Ala Gly Thr  Thr Ala Gly
       5285                5290                5295
   Gly Ala  Gly Cys Cys Cys Ala  Cys Cys Thr Thr Thr  Thr Thr Thr
       5300                5305                5310
   Cys Cys  Thr Ala Ala Thr Cys  Thr Gly Thr Gly Thr  Gly Thr Gly
       5315                5320                5325
   Cys Cys  Cys Thr Gly Thr Ala  Ala Cys Cys Thr Gly  Ala Cys Thr
       5330                5335                5340
   Gly Gly  Thr Thr Ala Ala Cys  Ala Gly Cys Ala Gly  Thr Cys Cys
       5345                5350                5355
   Thr Thr  Thr Gly Thr Ala Ala  Ala Cys Ala Gly Thr  Gly Thr Thr
       5360                5365                5370
   Thr Thr  Ala Ala Ala Cys Thr  Cys Thr Cys Cys Thr  Ala Gly Thr
       5375                5380                5385
   Cys Ala  Ala Thr Ala Thr Cys  Cys Ala Cys Cys Cys  Cys Ala Thr
       5390                5395                5400
   Cys Cys Ala Ala Thr Thr Thr Ala Thr Cys Ala Ala Gly Gly Ala
   5405 5410 5415
   Ala Gly Ala Ala Ala Thr Gly Gly Thr Thr Cys Ala Gly Ala Ala
   5420 5425 5430
   Ala Ala Thr Ala Thr Thr Thr Thr Cys Ala Gly Cys Cys Thr Ala
   5435 5440 5445
   Cys Ala Gly Thr Thr Ala Thr Gly Thr Thr Cys Ala Gly Thr Cys
   5450 5455 5460
   Ala Cys Ala Cys Ala Cys Ala Cys Ala Thr Ala Cys Ala Ala Ala
   5465 5470 5475
   Ala Thr Gly Thr Thr Cys Cys Thr Thr Thr Thr Gly Cys Thr Thr
   5480 5485 5490
   Thr Thr Ala Ala Ala Gly Thr Ala Ala Thr Thr Thr Thr Thr Gly
   5495 5500 5505
   Ala Cys Thr Cys Cys Cys Ala Gly Ala Thr Cys Ala Gly Thr Cys
   5510 5515 5520
   Ala Gly Ala Gly Cys Cys Cys Cys Thr Ala Cys Ala Gly Cys Ala
   5525 5530 5535
   Thr Thr Gly Thr Thr Ala Ala Gly Ala Ala Ala Gly Thr Ala Thr
   5540 5545 5550
   Thr Thr Gly Ala Thr Thr Thr Thr Thr Gly Thr Cys Thr Cys Ala
   5555 5560 5565
   Ala Thr Gly Ala Ala Ala Ala Thr Ala Ala Ala Ala Cys Thr Ala
   5570 5575 5580
   Thr Ala Thr Thr Cys Ala Thr Thr Thr Cys Cys Ala Cys Thr Cys
   5585 5590 5595
   Thr Ala Ala Ala Ala Ala Ala Ala Ala Ala Ala Ala Ala Ala Ala
   5600 5605 5610
   Ala Ala Ala
   5615
<210> 11
   <211> 4624
   <212> DNA
   <213> Homo sapiens
<400> 11
   ggaggaggtg gaggaggagg gctgcttgag gaagtataag aatgaagttg tgaagctgag      60
   attcccctcc attgggaccg gagaaaccag gggagccccc cgggcagccg cgcgcccctt     120
   cccacggggc cctttactgc gccgcgcgcc cggcccccac ccctcgcagc accccgcgcc     180
   ccgcgccctc ccagccgggt ccagccggag ccatggggcc ggagccgcag tgagcaccat     240
   ggagctggcg gccttgtgcc gctgggggct cctcctcgcc ctcttgcccc ccggagccgc     300
   gagcacccaa gtgtgcaccg gcacagacat gaagctgcgg ctccctgcca gtcccgagac     360
   ccacctggac atgctccgcc acctctacca gggctgccag gtggtgcagg gaaacctgga     420
   actcacctac ctgcccacca atgccagcct gtccttcctg caggatatcc aggaggtgca     480
   gggctacgtg ctcatcgctc acaaccaagt gaggcaggtc ccactgcaga ggctgcggat     540
   tgtgcgaggc acccagctct ttgaggacaa ctatgccctg gccgtgctag acaatggaga     600
   cccgctgaac aataccaccc ctgtcacagg ggcctcccca ggaggcctgc gggagctgca     660
   gcttcgaagc ctcacagaga tcttgaaagg aggggtcttg atccagcgga acccccagct     720
   ctgctaccag gacacgattt tgtggaagga catcttccac aagaacaacc agctggctct     780
   cacactgata gacaccaacc gctctcgggc ctgccacccc tgttctccga tgtgtaaggg     840
   ctcccgctgc tggggagaga gttctgagga ttgtcagagc ctgacgcgca ctgtctgtgc     900
   cggtggctgt gcccgctgca aggggccact gcccactgac tgctgccatg agcagtgtgc     960
   tgccggctgc acgggcccca agcactctga ctgcctggcc tgcctccact tcaaccacag    1020
   tggcatctgt gagctgcact gcccagccct ggtcacctac aacacagaca cgtttgagtc    1080
   catgcccaat cccgagggcc ggtatacatt cggcgccagc tgtgtgactg cctgtcccta    1140
   caactacctt tctacggacg tgggatcctg caccctcgtc tgccccctgc acaaccaaga    1200
   ggtgacagca gaggatggaa cacagcggtg tgagaagtgc agcaagccct gtgcccgagt    1260
   gtgctatggt ctgggcatgg agcacttgcg agaggtgagg gcagttacca gtgccaatat    1320
   ccaggagttt gctggctgca agaagatctt tgggagcctg gcatttctgc cggagagctt    1380
   tgatggggac ccagcctcca acactgcccc gctccagcca gagcagctcc aagtgtttga    1440
   gactctggaa gagatcacag gttacctata catctcagca tggccggaca gcctgcctga 1500
   cctcagcgtc ttccagaacc tgcaagtaat ccggggacga attctgcaca atggcgccta 1560
   ctcgctgacc ctgcaagggc tgggcatcag ctggctgggg ctgcgctcac tgagggaact 1620
   gggcagtgga ctggccctca tccaccataa cacccacctc tgcttcgtgc acacggtgcc 1680
   ctgggaccag ctctttcgga acccgcacca agctctgctc cacactgcca accggccaga 1740
   ggacgagtgt gtgggcgagg gcctggcctg ccaccagctg tgcgcccgag ggcactgctg 1800
   gggtccaggg cccacccagt gtgtcaactg cagccagttc cttcggggcc aggagtgcgt 1860
   ggaggaatgc cgagtactgc aggggctccc cagggagtat gtgaatgcca ggcactgttt 1920
   gccgtgccac cctgagtgtc agccccagaa tggctcagtg acctgttttg gaccggaggc 1980
   tgaccagtgt gtggcctgtg cccactataa ggaccctccc ttctgcgtgg cccgctgccc 2040
   cagcggtgtg aaacctgacc tctcctacat gcccatctgg aagtttccag atgaggaggg 2100
   cgcatgccag ccttgcccca tcaactgcac ccactcctgt gtggacctgg atgacaaggg 2160
   ctgccccgcc gagcagagag ccagccctct gacgtccatc atctctgcgg tggttggcat 2220
   tctgctggtc gtggtcttgg gggtggtctt tgggatcctc atcaagcgac ggcagcagaa 2280
   gatccggaag tacacgatgc ggagactgct gcaggaaacg gagctggtgg agccgctgac 2340
   acctagcgga gcgatgccca accaggcgca gatgcggatc ctgaaagaga cggagctgag 2400
   gaaggtgaag gtgcttggat ctggcgcttt tggcacagtc tacaagggca tctggatccc 2460
   tgatggggag aatgtgaaaa ttccagtggc catcaaagtg ttgagggaaa acacatcccc 2520
   caaagccaac aaagaaatct tagacgaagc atacgtgatg gctggtgtgg gctccccata 2580
   tgtctcccgc cttctgggca tctgcctgac atccacggtg cagctggtga cacagcttat 2640
   gccctatggc tgcctcttag accatgtccg ggaaaaccgc ggacgcctgg gctcccagga 2700
   cctgctgaac tggtgtatgc agattgccaa ggggatgagc tacctggagg atgtgcggct 2760
   cgtacacagg gacttggccg ctcggaacgt gctggtcaag agtcccaacc atgtcaaaat 2820
   tacagacttc gggctggctc ggctgctgga cattgacgag acagagtacc atgcagatgg 2880
   gggcaaggtg cccatcaagt ggatggcgct ggagtccatt ctccgccggc ggttcaccca 2940
   ccagagtgat gtgtggagtt atggtgtgac tgtgtgggag ctgatgactt ttggggccaa 3000
   accttacgat gggatcccag cccgggagat ccctgacctg ctggaaaagg gggagcggct 3060
   gccccagccc cccatctgca ccattgatgt ctacatgatc atggtcaaat gttggatgat 3120
   tgactctgaa tgtcggccaa gattccggga gttggtgtct gaattctccc gcatggccag 3180
   ggacccccag cgctttgtgg tcatccagaa tgaggacttg ggcccagcca gtcccttgga 3240
   cagcaccttc taccgctcac tgctggagga cgatgacatg ggggacctgg tggatgctga 3300
   ggagtatctg gtaccccagc agggcttctt ctgtccagac cctgccccgg gcgctggggg      3360
   catggtccac cacaggcacc gcagctcatc taccaggagt ggcggtgggg acctgacact      3420
   agggctggag ccctctgaag aggaggcccc caggtctcca ctggcaccct ccgaaggggc      3480
   tggctccgat gtatttgatg gtgacctggg aatgggggca gccaaggggc tgcaaagcct      3540
   ccccacacat gaccccagcc ctctacagcg gtacagtgag gaccccacag tacccctgcc     3600
   ctctgagact gatggctacg ttgcccccct gacctgcagc ccccagcctg aatatgtgaa      3660
   ccagccagat gttcggcccc agcccccttc gccccgagag ggccctctgc ctgctgcccg     3720
   acctgctggt gccactctgg aaaggcccaa gactctctcc ccagggaaga atggggtcgt      3780
   caaagacgtt tttgcctttg ggggtgccgt ggagaacccc gagtacttga caccccaggg     3840
   aggagctgcc cctcagcccc accctcctcc tgccttcagc ccagccttcg acaacctcta      3900
   ttactgggac caggacccac cagagcgggg ggctccaccc agcaccttca aagggacacc      3960
   tacggcagag aacccagagt acctgggtct ggacgtgcca gtgtgaacca gaaggccaag      4020
   tccgcagaag ccctgatgtg tcctcaggga gcagggaagg cctgacttct gctggcatca      4080
   agaggtggga gggccctccg accacttcca ggggaacctg ccatgccagg aacctgtcct      4140
   aaggaacctt ccttcctgct tgagttccca gatggctgga aggggtccag cctcgttgga      4200
   agaggaacag cactggggag tctttgtgga ttctgaggcc ctgcccaatg agactctagg      4260
   gtccagtgga tgccacagcc cagcttggcc ctttccttcc agatcctggg tactgaaagc      4320
   cttagggaag ctggcctgag aggggaagcg gccctaaggg agtgtctaag aacaaaagcg      4380
   acccattcag agactgtccc tgaaacctag tactgccccc catgaggaag gaacagcaat      4440
   ggtgtcagta tccaggcttt gtacagagtg cttttctgtt tagtttttac tttttttgtt      4500
   ttgttttttt aaagatgaaa taaagaccca gggggagaat gggtgttgta tggggaggca      4560
   agtgtggggg gtccttctcc acacccactt tgtccatttg caaatatatt ttggaaaaca      4620
   gcta                                                                   4624
<210> 12
   <211> 1342
   <212> PRT
   <213> Homo sapiens
<400> 12
<210> 13
   <211> 5765
   <212> DNA
   <213> Homo sapiens
<400> 13
<210> 14
   <211> 1282
   <212> PRT
   <213> Homo sapiens
<400> 14
   Met Lys Pro Ala Thr Gly Leu Trp Val Trp Val Ser Leu Leu Val Ala
   1               5                   10                  15
   Ala Gly Thr Val Gln Pro Ser Asp Ser Gln Ser Val Cys Ala Gly Thr
               20                  25                  30
   Glu Asn Lys Leu Ser Ser Leu Ser Asp Leu Glu Gln Gln Tyr Arg Ala
           35                  40                  45
   Leu Arg Lys Tyr Tyr Glu Asn Cys Glu Val Val Met Gly Asn Leu Glu
       50                  55                  60
   Ile Thr Ser Ile Glu His Asn Arg Asp Leu Ser Phe Leu Arg Ser Val
   65                  70                  75                  80
   Arg Glu Val Thr Gly Tyr Val Leu Val Ala Leu Asn Gln Phe Arg Tyr
                   85                  90                  95
   Leu Pro Leu Glu Asn Leu Arg Ile Ile Arg Gly Thr Lys Leu Tyr Glu
               100                 105                 110
   Asp Arg Tyr Ala Leu Ala Ile Phe Leu Asn Tyr Arg Lys Asp Gly Asn
           115                 120                 125
   Phe Gly Leu Gln Glu Leu Gly Leu Lys Asn Leu Thr Glu Ile Leu Asn
       130                 135                 140
   Gly Gly Val Tyr Val Asp Gln Asn Lys Phe Leu Cys Tyr Ala Asp Thr
   145                 150                 155                 160
   Ile His Trp Gln Asp Ile Val Arg Asn Pro Trp Pro Ser Asn Leu Thr
                   165                 170                 175
   Leu Val Ser Thr Asn Gly Ser Ser Gly Cys Gly Arg Cys His Lys Ser
               180                 185                 190
   Cys Thr Gly Arg Cys Trp Gly Pro Thr Glu Asn His Cys Gln Thr Leu
           195                 200                 205
   Thr Arg Thr Val Cys Ala Glu Gln Cys Asp Gly Arg Cys Tyr Gly Pro
       210                 215                 220
   Tyr Val Ser Asp Cys Cys His Arg Glu Cys Ala Gly Gly Cys Ser Gly
   225                 230                 235                 240
   Pro Lys Asp Thr Asp Cys Phe Ala Cys Met Asn Phe Asn Asp Ser Gly
                   245                 250                 255
   Ala Cys Val Thr Gln Cys Pro Gln Thr Phe Val Tyr Asn Pro Thr Thr
               260                 265                 270
   Phe Gln Leu Glu His Asn Phe Asn Ala Lys Tyr Thr Tyr Gly Ala Phe
           275                 280                 285
   Cys Val Lys Lys Cys Pro His Asn Phe Val Val Asp Ser Ser Ser Cys
       290                 295                 300
   Val Arg Ala Cys Pro Ser Ser Lys Met Glu Val Glu Glu Asn Gly Ile
   305                 310                 315                 320
   Lys Met Cys Lys Pro Cys Thr Asp Ile Cys Pro Lys Ala Cys Asp Gly
                   325                 330                 335
   Ile Gly Thr Gly Ser Leu Met Ser Ala Gln Thr Val Asp Ser Ser Asn
               340                 345                 350
   Ile Asp Lys Phe Ile Asn Cys Thr Lys Ile Asn Gly Asn Leu Ile Phe
           355                 360                 365
   Leu Val Thr Gly Ile His Gly Asp Pro Tyr Asn Ala Ile Glu Ala Ile
       370                 375                 380
   Asp Pro Glu Lys Leu Asn Val Phe Arg Thr Val Arg Glu Ile Thr Gly
   385                 390                 395                 400
   Phe Leu Asn Ile Gln Ser Trp Pro Pro Asn Met Thr Asp Phe Ser Val
                   405                 410                 415
   Phe Ser Asn Leu Val Thr Ile Gly Gly Arg Val Leu Tyr Ser Gly Leu
               420                 425                 430
   Ser Leu Leu Ile Leu Lys Gln Gln Gly Ile Thr Ser Leu Gln Phe Gln
           435                 440                 445
   Ser Leu Lys Glu Ile Ser Ala Gly Asn Ile Tyr Ile Thr Asp Asn Ser
       450                 455                 460
   Asn Leu Cys Tyr Tyr His Thr Ile Asn Trp Thr Thr Leu Phe Ser Thr
   465                 470                 475                 480
   Ile Asn Gln Arg Ile Val Ile Arg Asp Asn Arg Lys Ala Glu Asn Cys
                   485                 490                 495
   Thr Ala Glu Gly Met Val Cys Asn His Leu Cys Ser Ser Asp Gly Cys
               500                 505                 510
   Trp Gly Pro Gly Pro Asp Gln Cys Leu Ser Cys Arg Arg Phe Ser Arg
           515                 520                 525
   Gly Arg Ile Cys Ile Glu Ser Cys Asn Leu Tyr Asp Gly Glu Phe Arg
       530                 535                 540
   Glu Phe Glu Asn Gly Ser Ile Cys Val Glu Cys Asp Pro Gln Cys Glu
   545                 550                 555                 560
   Lys Met Glu Asp Gly Leu Leu Thr Cys His Gly Pro Gly Pro Asp Asn
                   565                 570                 575
   Cys Thr Lys Cys Ser His Phe Lys Asp Gly Pro Asn Cys Val Glu Lys
               580                 585                 590
   Cys Pro Asp Gly Leu Gln Gly Ala Asn Ser Phe Ile Phe Lys Tyr Ala
           595                 600                 605
   Asp Pro Asp Arg Glu Cys His Pro Cys His Pro Asn Cys Thr Gln Gly
       610                 615                 620
   Cys Ile Gly Ser Ser Ile Glu Asp Cys Ile Gly Leu Met Asp Arg Thr
   625                 630                 635                 640
   Pro Leu Ile Ala Ala Gly Val Ile Gly Gly Leu Phe Ile Leu Val Ile
                   645                 650                 655
   Val Gly Leu Thr Phe Ala Val Tyr Val Arg Arg Lys Ser Ile Lys Lys
               660                 665                 670
   Lys Arg Ala Leu Arg Arg Phe Leu Glu Thr Glu Leu Val Glu Pro Leu
           675                 680                 685
   Thr Pro Ser Gly Thr Ala Pro Asn Gln Ala Gln Leu Arg Ile Leu Lys
       690                 695                 700
   Glu Thr Glu Leu Lys Arg Val Lys Val Leu Gly Ser Gly Ala Phe Gly
   705                 710                 715                 720
   Thr Val Tyr Lys Gly Ile Trp Val Pro Glu Gly Glu Thr Val Lys Ile
                   725                 730                 735
   Pro Val Ala Ile Lys Ile Leu Asn Glu Thr Thr Gly Pro Lys Ala Asn
               740                 745                 750
   Val Glu Phe Met Asp Glu Ala Leu Ile Met Ala Ser Met Asp His Pro
           755                 760                 765
   His Leu Val Arg Leu Leu Gly Val Cys Leu Ser Pro Thr Ile Gln Leu
       770                 775                 780
   Val Thr Gln Leu Met Pro His Gly Cys Leu Leu Glu Tyr Val His Glu
   785                 790                 795                 800
   His Lys Asp Asn Ile Gly Ser Gln Leu Leu Leu Asn Trp Cys Val Gln
                   805                 810                 815
   Ile Ala Lys Gly Met Met Tyr Leu Glu Glu Arg Arg Leu Val His Arg
               820                 825                 830
   Asp Leu Ala Ala Arg Asn Val Leu Val Lys Ser Pro Asn His Val Lys
           835                 840                 845
   Ile Thr Asp Phe Gly Leu Ala Arg Leu Leu Glu Gly Asp Glu Lys Glu
   850 855 860
   Tyr Asn Ala Asp Gly Gly Lys Met Pro Ile Lys Trp Met Ala Leu Glu
   865 870 875 880
   Cys Ile His Tyr Arg Lys Phe Thr His Gln Ser Asp Val Trp Ser Tyr
   885 890 895
   Gly Val Thr Ile Trp Glu Leu Met Thr Phe Gly Gly Lys Pro Tyr Asp
   900 905 910
   Gly Ile Pro Thr Arg Glu Ile Pro Asp Leu Leu Glu Lys Gly Glu Arg
   915 920 925
   Leu Pro Gln Pro Pro Ile Cys Thr Ile Asp Val Tyr Met Val Met Val
   930 935 940
   Lys Cys Trp Met Ile Asp Ala Asp Ser Arg Pro Lys Phe Lys Glu Leu
   945 950 955 960
   Ala Ala Glu Phe Ser Arg Met Ala Arg Asp Pro Gln Arg Tyr Leu Val
   965 970 975
   Ile Gln Gly Asp Asp Arg Met Lys Leu Pro Ser Pro Asn Asp Ser Lys
   980 985 990
   Phe Phe Gln Asn Leu Leu Asp Glu Glu Asp Leu Glu Asp Met Met Asp
   995 1000 1005
   Ala Glu Glu Tyr Leu Val Pro Gln Ala Phe Asn Ile Pro Pro Pro
   1010 1015 1020
   Ile Tyr Thr Ser Arg Ala Arg Ile Asp Ser Asn Arg Asn Gln Phe
   1025 1030 1035
   Val Tyr Arg Asp Gly Gly Phe Ala Ala Glu Gln Gly Val Ser Val
   1040 1045 1050
   Pro Tyr Arg Ala Pro Thr Ser Thr Ile Pro Glu Ala Pro Val Ala
   1055 1060 1065
   Gln Gly Ala Thr Ala Glu Ile Phe Asp Asp Ser Cys Cys Asn Gly
   1070 1075 1080
   Thr Leu Arg Lys Pro Val Ala Pro His Val Gln Glu Asp Ser Ser
   1085 1090 1095
   Thr Gln  Arg Tyr Ser Ala Asp  Pro Thr Val Phe Ala  Pro Glu Arg
       1100                 1105                 1110
   Ser Pro  Arg Gly Glu Leu Asp  Glu Glu Gly Tyr Met  Thr Pro Met
       1115                 1120                 1125
   Arg Asp  Lys Pro Lys Gln Glu  Tyr Leu Asn Pro Val  Glu Glu Asn
       1130                 1135                 1140
   Pro Phe  Val Ser Arg Arg Lys  Asn Gly Asp Leu Gln  Ala Leu Asp
       1145                 1150                 1155
   Asn Pro  Glu Tyr His Asn Ala  Ser Asn Gly Pro Pro  Lys Ala Glu
       1160                 1165                 1170
   Asp Glu  Tyr Val Asn Glu Pro  Leu Tyr Leu Asn Thr  Phe Ala Asn
       1175                 1180                 1185
   Thr Leu  Gly Lys Ala Glu Tyr  Leu Lys Asn Asn Ile  Leu Ser Met
       1190                 1195                 1200
   Pro Glu  Lys Ala Lys Lys Ala  Phe Asp Asn Pro Asp  Tyr Trp Asn
       1205                 1210                 1215
   His Ser  Leu Pro Pro Arg Ser  Thr Leu Gln His Pro  Asp Tyr Leu
       1220                 1225                 1230
   Gln Glu  Tyr Ser Thr Lys Tyr  Phe Tyr Lys Gln Asn  Gly Arg Ile
       1235                 1240                 1245
   Arg Pro  Ile Val Ala Glu Asn  Pro Glu Tyr Leu Ser  Glu Phe Ser
       1250                 1255                 1260
   Leu Lys  Pro Gly Thr Val Leu  Pro Pro Pro Pro Tyr  Arg His Arg
       1265                 1270                 1275
   Asn Thr  Val Val
       1280
<210> 15
   <211> 3931
   <212> DNA
   <213> Homo sapiens
<400> 15
   cagcacggga tctgagactt ccaaaaaatg aagccggcga caggactttg ggtctgggtg 60
   agccttctcg tggcggcggg gaccgtccag cccagcgatt ctcagtcagt gtgtgcagga 120
   acggagaata aactgagctc tctctctgac ctggaacagc agtaccgagc cttgcgcaag 180
   tactatgaaa actgtgaggt tgtcatgggc aacctggaga taaccagcat tgagcacaac 240
   cgggacctct ccttcctgcg gtctgttcga gaagtcacag gctacgtgtt agtggctctt 300
   aatcagtttc gttacctgcc tctggagaat ttacgcatta ttcgtgggac aaaactttat 360
   gaggatcgat atgccttggc aatattttta aactacagaa aagatggaaa ctttggactt 420
   caagaacttg gattaaagaa cttgacagaa atcctaaatg gtggagtcta tgtagaccag 480
   aacaaattcc tttgttatgc agacaccatt cattggcaag atattgttcg gaacccatgg 540
   ccttccaact tgactcttgt gtcaacaaat ggtagttcag gatgtggacg ttgccataag 600
   tcctgtactg gccgttgctg gggacccaca gaaaatcatt gccagacttt gacaaggacg 660
   gtgtgtgcag aacaatgtga cggcagatgc tacggacctt acgtcagtga ctgctgccat 720
   cgagaatgtg ctggaggctg ctcaggacct aaggacacag actgctttgc ctgcatgaat 780
   ttcaatgaca gtggagcatg tgttactcag tgtccccaaa cctttgtcta caatccaacc 840
   acctttcaac tggagcacaa tttcaatgca aagtacacat atggagcatt ctgtgtcaag 900
   aaatgtccac ataactttgt ggtagattcc agttcttgtg tgcgtgcctg ccctagttcc 960
   aagatggaag tagaagaaaa tgggattaaa atgtgtaaac cttgcactga catttgccca 1020
   aaagcttgtg atggcattgg cacaggatca ttgatgtcag ctcagactgt ggattccagt 1080
   aacattgaca aattcataaa ctgtaccaag atcaatggga atttgatctt tctagtcact 1140
   ggtattcatg gggaccctta caatgcaatt gaagccatag acccagagaa actgaacgtc 1200
   tttcggacag tcagagagat aacaggtttc ctgaacatac agtcatggcc accaaacatg 1260
   actgacttca gtgttttttc taacctggtg accattggtg gaagagtact ctatagtggc 1320
   ctgtccttgc ttatcctcaa gcaacagggc atcacctctc tacagttcca gtccctgaag 1380
   gaaatcagcg caggaaacat ctatattact gacaacagca acctgtgtta ttatcatacc 1440
   attaactgga caacactctt cagcacaatc aaccagagaa tagtaatccg ggacaacaga 1500
   aaagctgaaa attgtactgc tgaaggaatg gtgtgcaacc atctgtgttc cagtgatggc 1560
   tgttggggac ctgggccaga ccaatgtctg tcgtgtcgcc gcttcagtag aggaaggatc 1620
   tgcatagagt cttgtaacct ctatgatggt gaatttcggg agtttgagaa tggctccatc 1680
   tgtgtggagt gtgaccccca gtgtgagaag atggaagatg gcctcctcac atgccatgga 1740
   ccgggtcctg acaactgtac aaagtgctct cattttaaag atggcccaaa ctgtgtggaa 1800
   aaatgtccag atggcttaca gggggcaaac agtttcattt tcaagtatgc tgatccagat 1860
   cgggagtgcc acccatgcca tccaaactgc acccaagggt gcataggctc aagtattgaa 1920
   gactgcatcg gcctgatgga tagaactccc ctgattgcag ctggagtaat tggtgggctc 1980
   ttcattctgg tcattgtggg tctgacattt gctgtttatg ttagaaggaa gagcatcaaa 2040
   aagaaaagag ccttgagaag attcttggaa acagagttgg tggaaccatt aactcccagt 2100
   ggcacagcac ccaatcaagc tcaacttcgt attttgaaag aaactgagct gaagagggta 2160
   aaagtccttg gctcaggtgc ttttggaacg gtttataaag gtatttgggt acctgaagga 2220
   gaaactgtga agattcctgt ggctattaag attcttaatg agacaactgg tcccaaggca 2280
   aatgtggagt tcatggatga agctctgatc atggcaagta tggatcatcc acacctagtc 2340
   cggttgctgg gtgtgtgtct gagcccaacc atccagctgg ttactcaact tatgccccat 2400
   ggctgcctgt tggagtatgt ccacgagcac aaggataaca ttggatcaca actgctgctt 2460
   aactggtgtg tccagatagc taagggaatg atgtacctgg aagaaagacg actcgttcat 2520
   cgggatttgg cagcccgtaa tgtcttagtg aaatctccaa accatgtgaa aatcacagat 2580
   tttgggctag ccagactctt ggaaggagat gaaaaagagt acaatgctga tggaggaaag 2640
   atgccaatta aatggatggc tctggagtgt atacattaca ggaaattcac ccatcagagt 2700
   gacgtttgga gctatggagt tactatatgg gaactgatga cctttggagg aaaaccctat 2760
   gatggaattc caacgcgaga aatccctgat ttattagaga aaggagaacg tttgcctcag 2820
   cctcccatct gcactattga cgtttacatg gtcatggtca aatgttggat gattgatgct 2880
   gacagtagac ctaaatttaa ggaactggct gctgagtttt caaggatggc tcgagaccct 2940
   caaagatacc tagttattca gggtgatgat cgtatgaagc ttcccagtcc aaatgacagc 3000
   aagttctttc agaatctctt ggatgaagag gatttggaag atatgatgga tgctgaggag 3060
   tacttggtcc ctcaggcttt caacatccca cctcccatct atacttccag agcaagaatt 3120
   gactcgaata ggaaccagtt tgtgtaccga gatggaggtt ttgctgctga acaaggagtg 3180
   tctgtgccct acagagcccc aactagcaca attccagaag ctcctgtggc acagggtgct 3240
   actgctgaga tttttgatga ctcctgctgt aatggcaccc tacgcaagcc agtggcaccc 3300
   catgtccaag aggacagtag cacccagagg tacagtgctg accccaccgt gtttgcccca 3360
   gaacggagcc cacgaggaga gctggatgag gaaggttaca tgactcctat gcgagacaaa 3420
   cccaaacaag aatacctgaa tccagtggag gagaaccctt ttgtttctcg gagaaaaaat 3480
   ggagaccttc aagcattgga taatcccgaa tatcacaatg catccaatgg tccacccaag 3540
   gccgaggatg agtatgtgaa tgagccactg tacctcaaca cctttgccaa caccttggga 3600
   aaagctgagt acctgaagaa caacatactg tcaatgccag agaaggccaa gaaagcgttt 3660
   gacaaccctg actactggaa ccacagcctg ccacctcgga gcacccttca gcacccagac 3720
   tacctgcagg agtacagcac aaaatatttt tataaacaga atgggcggat ccggcctatt 3780
   gtggcagaga atcctgaata cctctctgag ttctccctga agccaggcac tgtgctgccg 3840
   cctccacctt acagacaccg gaatactgtg gtgtaagctc agttgtggtt ttttaggtgg 3900
   agagacacac ctgctccaat ttccccaccc c 3931
<210> 16
   <211> 595
   <212> PRT
   <213> Homo sapiens
<400> 16
<210> 17
   <211> 6330
   <212> DNA
   <213> Homo sapiens
<400> 17
   aggagctggc ggagggcgtt cgtcctggga ctgcacttgc tcccgtcggg tcgcccggct        60
   tcaccggacc cgcaggctcc cggggcaggg ccggggccag agctcgcgtg tcggcgggac       120
   atgcgctgcg tcgcctctaa cctcgggctg tgctcttttt ccaggtggcc cgccggtttc       180
   tgagccttct gccctgcggg gacacggtct gcaccctgcc cgcggccacg gaccatgacc       240
   atgaccctcc acaccaaagc atctgggatg gccctactgc atcagatcca agggaacgag       300
   ctggagcccc tgaaccgtcc gcagctcaag atccccctgg agcggcccct gggcgaggtg       360
   tacctggaca gcagcaagcc cgccgtgtac aactaccccg agggcgccgc ctacgagttc       420
   aacgccgcgg ccgccgccaa cgcgcaggtc tacggtcaga ccggcctccc ctacggcccc       480
   gggtctgagg ctgcggcgtt cggctccaac ggcctggggg gtttcccccc actcaacagc       540
   gtgtctccga gcccgctgat gctactgcac ccgccgccgc agctgtcgcc tttcctgcag       600
   ccccacggcc agcaggtgcc ctactacctg gagaacgagc ccagcggcta cacggtgcgc       660
   gaggccggcc cgccggcatt ctacaggcca aattcagata atcgacgcca gggtggcaga 720
   gaaagattgg ccagtaccaa tgacaaggga agtatggcta tggaatctgc caaggagact 780
   cgctactgtg cagtgtgcaa tgactatgct tcaggctacc attatggagt ctggtcctgt 840
   gagggctgca aggccttctt caagagaagt attcaaggac ataacgacta tatgtgtcca 900
   gccaccaacc agtgcaccat tgataaaaac aggaggaaga gctgccaggc ctgccggctc 960
   cgcaaatgct acgaagtggg aatgatgaaa ggtgggatac gaaaagaccg aagaggaggg 1020
   agaatgttga aacacaagcg ccagagagat gatggggagg gcaggggtga agtggggtct 1080
   gctggagaca tgagagctgc caacctttgg ccaagcccgc tcatgatcaa acgctctaag 1140
   aagaacagcc tggccttgtc cctgacggcc gaccagatgg tcagtgcctt gttggatgct 1200
   gagcccccca tactctattc cgagtatgat cctaccagac ccttcagtga agcttcgatg 1260
   atgggcttac tgaccaacct ggcagacagg gagctggttc acatgatcaa ctgggcgaag 1320
   agggtgccag gctttgtgga tttgaccctc catgatcagg tccaccttct agaatgtgcc 1380
   tggctagaga tcctgatgat tggtctcgtc tggcgctcca tggagcaccc agggaagcta 1440
   ctgtttgctc ctaacttgct cttggacagg aaccagggaa aatgtgtaga gggcatggtg 1500
   gagatcttcg acatgctgct ggctacatca tctcggttcc gcatgatgaa tctgcaggga 1560
   gaggagtttg tgtgcctcaa atctattatt ttgcttaatt ctggagtgta cacatttctg 1620
   tccagcaccc tgaagtctct ggaagagaag gaccatatcc accgagtcct ggacaagatc 1680
   acagacactt tgatccacct gatggccaag gcaggcctga ccctgcagca gcagcaccag 1740
   cggctggccc agctcctcct catcctctcc cacatcaggc acatgagtaa caaaggcatg 1800
   gagcatctgt acagcatgaa gtgcaagaac gtggtgcccc tctatgacct gctgctggag 1860
   atgctggacg cccaccgcct acatgcgccc actagccgtg gaggggcatc cgtggaggag 1920
   acggaccaaa gccacttggc cactgcgggc tctacttcat cgcattcctt gcaaaagtat 1980
   tacatcacgg gggaggcaga gggtttccct gccacggtct gagagctccc tggctcccac 2040
   acggttcaga taatccctgc tgcattttac cctcatcatg caccacttta gccaaattct 2100
   gtctcctgca tacactccgg catgcatcca acaccaatgg ctttctagat gagtggccat 2160
   tcatttgctt gctcagttct tagtggcaca tcttctgtct tctgttggga acagccaaag 2220
   ggattccaag gctaaatctt tgtaacagct ctctttcccc cttgctatgt tactaagcgt 2280
   gaggattccc gtagctcttc acagctgaac tcagtctatg ggttggggct cagataactc 2340
   tgtgcattta agctacttgt agagacccag gcctggagag tagacatttt gcctctgata 2400
   agcacttttt aaatggctct aagaataagc cacagcaaag aatttaaagt ggctccttta 2460
   attggtgact tggagaaagc taggtcaagg gtttattata gcaccctctt gtattcctat 2520
   ggcaatgcat ccttttatga aagtggtaca ccttaaagct tttatatgac tgtagcagag 2580
   tatctggtga ttgtcaattc attcccccta taggaataca aggggcacac agggaaggca 2640
   gatcccctag ttggcaagac tattttaact tgatacactg cagattcaga tgtgctgaaa 2700
   gctctgcctc tggctttccg gtcatgggtt ccagttaatt catgcctccc atggacctat 2760
   ggagagcagc aagttgatct tagttaagtc tccctatatg agggataagt tcctgatttt 2820
   tgtttttatt tttgtgttac aaaagaaagc cctccctccc tgaacttgca gtaaggtcag 2880
   cttcaggacc tgttccagtg ggcactgtac ttggatcttc ccggcgtgtg tgtgccttac 2940
   acaggggtga actgttcact gtggtgatgc atgatgaggg taaatggtag ttgaaaggag 3000
   caggggccct ggtgttgcat ttagccctgg ggcatggagc tgaacagtac ttgtgcagga 3060
   ttgttgtggc tactagagaa caagagggaa agtagggcag aaactggata cagttctgag 3120
   gcacagccag acttgctcag ggtggccctg ccacaggctg cagctaccta ggaacattcc 3180
   ttgcagaccc cgcattgccc tttgggggtg ccctgggatc cctggggtag tccagctctt 3240
   cttcatttcc cagcgtggcc ctggttggaa gaagcagctg tcacagctgc tgtagacagc 3300
   tgtgttccta caattggccc agcaccctgg ggcacgggag aagggtgggg accgttgctg 3360
   tcactactca ggctgactgg ggcctggtca gattacgtat gcccttggtg gtttagagat 3420
   aatccaaaat cagggtttgg tttggggaag aaaatcctcc cccttcctcc cccgccccgt 3480
   tccctaccgc ctccactcct gccagctcat ttccttcaat ttcctttgac ctataggcta 3540
   aaaaagaaag gctcattcca gccacagggc agccttccct gggcctttgc ttctctagca 3600
   caattatggg ttacttcctt tttcttaaca aaaaagaatg tttgatttcc tctgggtgac 3660
   cttattgtct gtaattgaaa ccctattgag aggtgatgtc tgtgttagcc aatgacccag 3720
   gtgagctgct cgggcttctc ttggtatgtc ttgtttggaa aagtggattt cattcatttc 3780
   tgattgtcca gttaagtgat caccaaagga ctgagaatct gggagggcaa aaaaaaaaaa 3840
   aaagttttta tgtgcactta aatttgggga caattttatg tatctgtgtt aaggatatgt 3900
   ttaagaacat aattcttttg ttgctgtttg tttaagaagc accttagttt gtttaagaag 3960
   caccttatat agtataatat atattttttt gaaattacat tgcttgttta tcagacaatt 4020
   gaatgtagta attctgttct ggatttaatt tgactgggtt aacatgcaaa aaccaaggaa 4080
   aaatatttag tttttttttt tttttttgta tacttttcaa gctaccttgt catgtataca 4140
   gtcatttatg cctaaagcct ggtgattatt catttaaatg aagatcacat ttcatatcaa 4200
   cttttgtatc cacagtagac aaaatagcac taatccagat gcctattgtt ggatactgaa 4260
   tgacagacaa tcttatgtag caaagattat gcctgaaaag gaaaattatt cagggcagct 4320
   aattttgctt ttaccaaaat atcagtagta atatttttgg acagtagcta atgggtcagt 4380
   gggttctttt taatgtttat acttagattt tcttttaaaa aaattaaaat aaaacaaaaa 4440
   aaaatttcta ggactagacg atgtaatacc agctaaagcc aaacaattat acagtggaag 4500
   gttttacatt attcatccaa tgtgtttcta ttcatgttaa gatactacta catttgaagt 4560
   gggcagagaa catcagatga ttgaaatgtt cgcccagggg tctccagcaa ctttggaaat 4620
   ctctttgtat ttttacttga agtgccacta atggacagca gatattttct ggctgatgtt 4680
   ggtattgggt gtaggaacat gatttaaaaa aaaactcttg cctctgcttt cccccactct 4740
   gaggcaagtt aaaatgtaaa agatgtgatt tatctggggg gctcaggtat ggtggggaag 4800
   tggattcagg aatctgggga atggcaaata tattaagaag agtattgaaa gtatttggag 4860
   gaaaatggtt aattctgggt gtgcaccagg gttcagtaga gtccacttct gccctggaga 4920
   ccacaaatca actagctcca tttacagcca tttctaaaat ggcagcttca gttctagaga 4980
   agaaagaaca acatcagcag taaagtccat ggaatagcta gtggtctgtg tttcttttcg 5040
   ccattgccta gcttgccgta atgattctat aatgccatca tgcagcaatt atgagaggct 5100
   aggtcatcca aagagaagac cctatcaatg taggttgcaa aatctaaccc ctaaggaagt 5160
   gcagtctttg atttgatttc cctagtaacc ttgcagatat gtttaaccaa gccatagccc 5220
   atgccttttg agggctgaac aaataaggga cttactgata atttactttt gatcacatta 5280
   aggtgttctc accttgaaat cttatacact gaaatggcca ttgatttagg ccactggctt 5340
   agagtactcc ttcccctgca tgacactgat tacaaatact ttcctattca tactttccaa 5400
   ttatgagatg gactgtgggt actgggagtg atcactaaca ccatagtaat gtctaatatt 5460
   cacaggcaga tctgcttggg gaagctagtt atgtgaaagg caaatagagt catacagtag 5520
   ctcaaaaggc aaccataatt ctctttggtg caggtcttgg gagcgtgatc tagattacac 5580
   tgcaccattc ccaagttaat cccctgaaaa cttactctca actggagcaa atgaactttg 5640
   gtcccaaata tccatctttt cagtagcgtt aattatgctc tgtttccaac tgcatttcct 5700
   ttccaattga attaaagtgt ggcctcgttt ttagtcattt aaaattgttt tctaagtaat 5760
   tgctgcctct attatggcac ttcaattttg cactgtcttt tgagattcaa gaaaaatttc 5820
   tattcttttt tttgcatcca attgtgcctg aacttttaaa atatgtaaat gctgccatgt 5880
   tccaaaccca tcgtcagtgt gtgtgtttag agctgtgcac cctagaaaca acatattgtc 5940
   ccatgagcag gtgcctgaga cacagacccc tttgcattca cagagaggtc attggttata 6000
   gagacttgaa ttaataagtg acattatgcc agtttctgtt ctctcacagg tgataaacaa 6060
   tgctttttgt gcactacata ctcttcagtg tagagctctt gttttatggg aaaaggctca 6120
   aatgccaaat tgtgtttgat ggattaatat gcccttttgc cgatgcatac tattactgat 6180
   gtgactcggt tttgtcgcag ctttgctttg tttaatgaaa cacacttgta aacctctttt 6240
   gcactttgaa aaagaatcca gcgggatgct cgagcacctg taaacaattt tctcaaccta 6300
   tttgatgttc aaataaagaa ttaaactaaa 6330
<210> 18
   <211> 932
   <212> PRT
   <213> Homo sapiens
<400> 18
   Met Thr Glu Leu Lys Ala Lys Gly Pro Arg Ala Pro His Val Ala Gly
   1               5                   10                  15
   Gly Pro Pro Ser Pro Glu Val Gly Ser Pro Leu Leu Cys Arg Pro Ala
               20                  25                  30
   Ala Gly Pro Phe Pro Gly Ser Gln Thr Ser Asp Thr Leu Pro Glu Val
           35                  40                  45
   Ser Ala Ile Pro Ile Ser Leu Asp Gly Leu Leu Phe Pro Arg Pro Cys
       50                  55                  60
   Gln Gly Gln Asp Pro Ser Asp Glu Lys Thr Gln Asp Gln Gln Ser Leu
   65                  70                  75                  80
   Ser Asp Val Glu Gly Ala Tyr Ser Arg Ala Glu Ala Thr Arg Gly Ala
                   85                  90                  95
   Gly Gly Ser Ser Ser Ser Pro Pro Glu Lys Asp Ser Gly Leu Leu Asp
               100                 105                 110
   Ser Val Leu Asp Thr Leu Leu Ala Pro Ser Gly Pro Gly Gln Ser Gln
           115                 120                 125
   Pro Ser Pro Pro Ala Cys Glu Val Thr Ser Ser Trp Cys Leu Phe Gly
       130                 135                 140
   Pro Glu Leu Pro Glu Asp Pro Pro Ala Ala Pro Ala Thr Gln Arg Val
   145                 150                 155                 160
   Leu Ser Pro Leu Met Ser Arg Ser Gly Cys Lys Val Gly Asp Ser Ser
                   165                 170                 175
   Gly Thr Ala Ala Ala His Lys Val Leu Pro Arg Gly Leu Ser Pro Ala
               180                 185                 190
   Arg Gln Leu Leu Leu Pro Ala Ser Glu Ser Pro His Trp Ser Gly Ala
           195                 200                 205
   Pro Val Lys Pro Ser Pro Gln Ala Ala Ala Val Glu Val Glu Glu Glu
       210                 215                 220
   Asp Gly Ser Glu Ser Glu Glu Ser Ala Gly Pro Leu Leu Lys Gly Lys
   225                 230                 235                 240
   Pro Arg Ala Leu Gly Gly Ala Ala Ala Gly Gly Gly Ala Ala Ala Val
                   245                 250                 255
   Pro Pro Gly Ala Ala Ala Gly Gly Val Ala Leu Val Pro Lys Glu Asp
               260                 265                 270
   Ser Arg Phe Ser Ala Pro Arg Val Ala Leu Val Glu Gln Asp Ala Pro
           275                 280                 285
   Met Ala Pro Gly Arg Ser Pro Leu Ala Thr Thr Val Met Asp Phe Ile
       290                 295                 300
   His Val Pro Ile Leu Pro Leu Asn His Ala Leu Leu Ala Ala Arg Thr
   305                 310                 315                 320
   Arg Gln Leu Leu Glu Asp Glu Ser Tyr Asp Gly Gly Ala Gly Ala Ala
                   325                 330                 335
   Ser Ala Phe Ala Pro Pro Arg Ser Ser Pro Cys Ala Ser Ser Thr Pro
               340                 345                 350
   Val Ala Val Gly Asp Phe Pro Asp Cys Ala Tyr Pro Pro Asp Ala Glu
           355                 360                 365
   Pro Lys Asp Asp Ala Tyr Pro Leu Tyr Ser Asp Phe Gln Pro Pro Ala
       370                 375                 380
   Leu Lys Ile Lys Glu Glu Glu Glu Gly Ala Glu Ala Ser Ala Arg Ser
   385                 390                 395                 400
   Pro Arg Ser Tyr Leu Val Ala Gly Ala Asn Pro Ala Ala Phe Pro Asp
                   405                 410                 415
   Phe Pro Leu Gly Pro Pro Pro Pro Leu Pro Pro Arg Ala Thr Pro Ser
               420                 425                 430
   Arg Pro Gly Glu Ala Ala Val Thr Ala Ala Pro Ala Ser Ala Ser Val
           435                 440                 445
   Ser Ser Ala Ser Ser Ser Gly Ser Thr Leu Glu Cys Ile Leu Tyr Lys
       450                 455                 460
   Ala Glu Gly Ala Pro Pro Gln Gln Gly Pro Phe Ala Pro Pro Pro Cys
   465 470 475 480
   Lys Ala Pro Gly Ala Ser Gly Cys Leu Leu Pro Arg Asp Gly Leu Pro
   485 490 495
   Ser Thr Ser Ala Ser Ala Ala Ala Ala Gly Ala Ala Pro Ala Leu Tyr
   500 505 510
   Pro Ala Leu Gly Leu Asn Gly Leu Pro Gln Leu Gly Tyr Gln Ala Ala
   515 520 525
   Val Leu Lys Glu Gly Leu Pro Gln Val Tyr Pro Pro Tyr Leu Asn Tyr
   530 535 540
   Leu Arg Pro Asp Ser Glu Ala Ser Gln Ser Pro Gln Tyr Ser Phe Glu
   545 550 555 560
   Ser Leu Pro Gln Lys Ile Cys Leu Ile Cys Gly Asp Glu Ala Ser Gly
   565 570 575
   Cys His Tyr Gly Val Leu Thr Cys Gly Ser Cys Lys Val Phe Phe Lys
   580 585 590
   Arg Ala Met Glu Gly Gln His Asn Tyr Leu Cys Ala Gly Arg Asn Asp
   595 600 605
   Cys Ile Val Asp Lys Ile Arg Arg Lys Asn Cys Pro Ala Cys Arg Leu
   610 615 620
   Arg Lys Cys Cys Gln Ala Gly Met Val Leu Gly Gly Arg Lys Phe Lys
   625 630 635 640
   Lys Phe Asn Lys Val Arg Val Val Arg Ala Leu Asp Ala Val Ala Leu
   645 650 655
   Pro Gln Pro Leu Gly Val Pro Asn Glu Ser Gln Ala Leu Ser Gln Arg
   660 665 670
   Phe Thr Phe Ser Pro Gly Gln Asp Ile Gln Leu Ile Pro Pro Leu Ile
   675 680 685
   Asn Leu Leu Met Ser Ile Glu Pro Asp Val Ile Tyr Ala Gly His Asp
   690 695 700
   Asn Thr Lys Pro Asp Thr Ser Ser Ser Leu Leu Thr Ser Leu Asn Gln
   705 710 715 720
   Leu Gly Glu Arg Gln Leu Leu Ser Val Val Lys Trp Ser Lys Ser Leu
                   725                 730                 735
   Pro Gly Phe Arg Asn Leu His Ile Asp Asp Gln Ile Thr Leu Ile Gln
               740                 745                 750
   Tyr Ser Trp Met Ser Leu Met Val Phe Gly Leu Gly Trp Arg Ser Tyr
           755                 760                 765
   Lys His Val Ser Gly Gln Met Leu Tyr Phe Ala Pro Asp Leu Ile Leu
       770                 775                 780
   Asn Glu Gln Arg Met Lys Glu Ser Ser Phe Tyr Ser Leu Cys Leu Thr
   785                 790                 795                 800
   Met Trp Gln Ile Pro Gln Glu Phe Val Lys Leu Gln Val Ser Gln Glu
                   805                 810                 815
   Glu Phe Leu Cys Met Lys Val Leu Leu Leu Leu Asn Thr Ile Pro Leu
               820                 825                 830
   Glu Gly Leu Arg Ser Gln Thr Gln Phe Glu Glu Met Arg Ser Ser Tyr
           835                 840                 845
   Ile Arg Glu Leu Ile Lys Ala Ile Gly Leu Arg Gln Lys Gly Val Val
       850                 855                 860
   Ser Ser Ser Gln Arg Phe Tyr Gln Leu Thr Lys Leu Leu Asp Asn Leu
   865                 870                 875                 880
   His Asp Leu Val Lys Gln Leu His Leu Tyr Cys Leu Asn Thr Phe Ile
                   885                 890                 895
   Gln Ser Arg Ala Leu Ser Val Glu Phe Pro Glu Met Met Ser Glu Val
               900                 905                 910
   Ile Ala Ala Gln Leu Pro Lys Ile Leu Ala Gly Met Val Lys Pro Leu
           915                 920                 925
   Leu Phe His Lys
       930
<210> 19
   <211> 3014
   <212> DNA
   <213> Homo sapiens
<400> 19
   ctgaccagcg ccgccctccc ccgcccccga cccaggaggt ggagatccct ccggtccagc 60
   cacattcaac acccactttc tcctccctct gcccctatat tcccgaaacc ccctcctcct 120
   tcccttttcc ctcctccctg gagacggggg aggagaaaag gggagtccag tcgtcatgac 180
   tgagctgaag gcaaagggtc cccgggctcc ccacgtggcg ggcggcccgc cctcccccga 240
   ggtcggatcc ccactgctgt gtcgcccagc cgcaggtccg ttcccgggga gccagacctc 300
   ggacaccttg cctgaagttt cggccatacc tatctccctg gacgggctac tcttccctcg 360
   gccctgccag ggacaggacc cctccgacga aaagacgcag gaccagcagt cgctgtcgga 420
   cgtggagggc gcatattcca gagctgaagc tacaaggggt gctggaggca gcagttctag 480
   tcccccagaa aaggacagcg gactgctgga cagtgtcttg gacactctgt tggcgccctc 540
   aggtcccggg cagagccaac ccagccctcc cgcctgcgag gtcaccagct cttggtgcct 600
   gtttggcccc gaacttcccg aagatccacc ggctgccccc gccacccagc gggtgttgtc 660
   cccgctcatg agccggtccg ggtgcaaggt tggagacagc tccgggacgg cagctgccca 720
   taaagtgctg ccccggggcc tgtcaccagc ccggcagctg ctgctcccgg cctctgagag 780
   ccctcactgg tccggggccc cagtgaagcc gtctccgcag gccgctgcgg tggaggttga 840
   ggaggaggat ggctctgagt ccgaggagtc tgcgggtccg cttctgaagg gcaaacctcg 900
   ggctctgggt ggcgcggcgg ctggaggagg agccgcggct gtcccgccgg gggcggcagc 960
   aggaggcgtc gccctggtcc ccaaggaaga ttcccgcttc tcagcgccca gggtcgccct 1020
   ggtggagcag gacgcgccga tggcgcccgg gcgctccccg ctggccacca cggtgatgga 1080
   tttcatccac gtgcctatcc tgcctctcaa tcacgcctta ttggcagccc gcactcggca 1140
   gctgctggaa gacgaaagtt acgacggcgg ggccggggct gccagcgcct ttgccccgcc 1200
   gcggagttca ccctgtgcct cgtccacccc ggtcgctgta ggcgacttcc ccgactgcgc 1260
   gtacccgccc gacgccgagc ccaaggacga cgcgtaccct ctctatagcg acttccagcc 1320
   gcccgctcta aagataaagg aggaggagga aggcgcggag gcctccgcgc gctccccgcg 1380
   ttcctacctt gtggccggtg ccaaccccgc agccttcccg gatttcccgt tggggccacc 1440
   gcccccgctg ccgccgcgag cgaccccatc cagacccggg gaagcggcgg tgacggccgc 1500
   acccgccagt gcctcagtct cgtctgcgtc ctcctcgggg tcgaccctgg agtgcatcct 1560
   gtacaaagcg gagggcgcgc cgccccagca gggcccgttc gcgccgccgc cctgcaaggc 1620
   gccgggcgcg agcggctgcc tgctcccgcg ggacggcctg ccctccacct ccgcctctgc 1680
   cgccgccgcc ggggcggccc ccgcgctcta ccctgcactc ggcctcaacg ggctcccgca 1740
   gctcggctac caggccgccg tgctcaagga gggcctgccg caggtctacc cgccctatct 1800
   caactacctg aggccggatt cagaagccag ccagagccca caatacagct tcgagtcatt 1860
   acctcagaag atttgtttaa tctgtgggga tgaagcatca ggctgtcatt atggtgtcct 1920
   tacctgtggg agctgtaagg tcttctttaa gagggcaatg gaagggcagc acaactactt 1980
   atgtgctgga agaaatgact gcatcgttga taaaatccgc agaaaaaact gcccagcatg 2040
   tcgccttaga aagtgctgtc aggctggcat ggtccttgga ggtcgaaaat ttaaaaagtt 2100
   caataaagtc agagttgtga gagcactgga tgctgttgct ctcccacagc cattgggcgt 2160
   tccaaatgaa agccaagccc taagccagag attcactttt tcaccaggtc aagacataca 2220
   gttgattcca ccactgatca acctgttaat gagcattgaa ccagatgtga tctatgcagg 2280
   acatgacaac acaaaacctg acacctccag ttctttgctg acaagtctta atcaactagg 2340
   cgagaggcaa cttctttcag tagtcaagtg gtctaaatca ttgccaggtt ttcgaaactt 2400
   acatattgat gaccagataa ctctcattca gtattcttgg atgagcttaa tggtgtttgg 2460
   tctaggatgg agatcctaca aacatgtcag tgggcagatg ctgtattttg cacctgatct 2520
   aatactaaat gaacagcgga tgaaagaatc atcattctat tcattatgcc ttaccatgtg 2580
   gcagatccca caggagtttg tcaagcttca agttagccaa gaagagttcc tctgtatgaa 2640
   agtattgtta cttcttaata caattccttt ggaagggcta cgaagtcaaa cccagtttga 2700
   ggagatgagg tcaagctaca ttagagagct catcaaggca attggtttga ggcaaaaagg 2760
   agttgtgtcg agctcacagc gtttctatca acttacaaaa cttcttgata acttgcatga 2820
   tcttgtcaaa caacttcatc tgtactgctt gaatacattt atccagtccc gggcactgag 2880
   tgttgaattt ccagaaatga tgtctgaagt tattgctgca caattaccca agatattggc 2940
   agggatggtg aaaccccttc tctttcataa aaagtgaatg tcatcttttt cttttaaaga 3000
   attaaatttt gtgg 3014
<210> 20
   <211> 31
   <212> DNA
   <213> Homo sapiens
<400> 20
   tctagccatt tgatgccaac agtagaaagg g 31
<210> 21
   <211> 19
   <212> DNA
   <213> Homo sapiens
<400> 21
   cttgactgag cgactgagc 19
<210> 22
   <211> 19
   <212> DNA
   <213> Homo sapiens
<400> 22
   ctccagggtt aggcagatc 19
<210> 23
   <211> 19
   <212> DNA
   <213> Homo sapiens
<400> 23
   cagcgggata gtgcgattc 19
<210> 24
   <211> 22
   <212> DNA
   <213> Homo sapiens
<400> 24
   taacttacgg agtcgctcta cg 22
<210> 25
   <211> 22
   <212> DNA
   <213> Homo sapiens
<400> 25
   ggatgggatt ctttaggtcc tg 22
<210> 26
   <211> 58
   <212> DNA
   <213> Homo sapiens
<400> 26
   aatgatacgg cgaccaccga gatctacact ctttccctac acgacgctct tccgatct 58
<210> 27
   <211> 61
   <212> DNA
   <213> Homo sapiens
<400> 27
   caagcagaag acggcatacg agatcggtct cggcattcct gctgaaccgc tcttccgatc 60
   t 61
<210> 28
   <211> 17
   <212> DNA
   <213> Homo sapiens
<400> 28
   cgaccaccga gatctac 17
<210> 29
   <211> 17
   <212> DNA
   <213> Homo sapiens
<400> 29
   acgtgaactt tgaaagg 17
<210> 30
   <211> 21
   <212> DNA
   <213> Homo sapiens
<400> 30
   gtagatcctg caaaaagagt g 21
<210> 31
   <211> 19
   <212> DNA
   <213> Homo sapiens
<400> 31
   cctttctact gttggcatc 19
<210> 32
   <211> 20
   <212> DNA
   <213> Homo sapiens
<400> 32
   ctgagaatga ttctgtctgg 20
<210> 33
   <211> 19
   <212> DNA
   <213> Homo sapiens
<400> 33
   caatgcggtc catatatcc 19
<210> 34
   <211> 42
   <212> DNA
   <213> Homo sapiens
<400> 34
   cattcaaatc cccgagttga actttccttt caaagttcac gt 42
<210> 35
   <211> 31
   <212> DNA
   <213> Homo sapiens
<400> 35
   tctagccatt tgatgccaac agtagaaagg g 31
<210> 36
   <211> 64
   <212> DNA
   <213> Homo sapiens
<400> 36
   taacttacgg agtcgctcta cgcattcaaa tccccgagtt gaactttcct ttcaaagttc 60
   acgt 64
<210> 37
   <211> 53
   <212> DNA
   <213> Homo sapiens
<400> 37
   taacttacgg agtcgctcta cgtctagcca tttgatgcca acagtagaaa ggg 53
<210> 38
   <211> 64
   <212> DNA
   <213> Homo sapiens
<400> 38
   taacttacgg agtcgctcta cgcattcaaa tccccgagtt gaactttcct ttcaaagttc 60
   acgt 64
<210> 39
   <211> 65
   <212> DNA
   <213> Homo sapiens
<400> 39
   taacttacgg agtcgctcta cgcattcaaa tccccgagtt gaactttcct ttcaaagttc 60
   acgtt 65
<210> 40
   <211> 58
   <212> DNA
   <213> Homo sapiens
<400> 40
   taacttacgg agtcgctcta cgtctagcca tttgatgcca acagtagaaa gggaaata 58
<210> 41
   <211> 62
   <212> DNA
   <213> Homo sapiens
<400> 41
   taacttacgg agtcgctcta cgtctagcca tttgatgcca acagtagaaa gggaaatatc 60
   tt 62
<210> 42
   <211> 22
   <212> DNA
   <213> Homo sapiens
<400> 42
   taacttacgg agtcgctcta cg 22
<210> 43
   <211> 30
   <212> DNA
   <213> Homo sapiens
<400> 43
   tctagccatt tgatgccaac agtagaaagg 30
<210> 44
   <211> 53
   <212> DNA
   <213> Homo sapiens
<400> 44
   taacttacgg agtcgctcta cgtctagcca tttgatgcca acagtagaaa ggg 53
<210> 45
   <211> 89
   <212> DNA
   <213> Homo sapiens
<400> 45
   aatgatacgg cgaccaccga gatctacact ctttccctac acgacgctct tccgatcttc 60
   tagccatttg atgccaacag tagaaaggg 89
<210> 46
   <211> 100
   <212> DNA
   <213> Homo sapiens
<400> 46
   aatgatacgg cgaccaccga gatctacact ctttccctac acgacgctct tccgatctca 60
   ttcaaatccc cgagttgaac tttcctttca aagttcacgt 100
<210> 47
   <211> 55
   <212> DNA
   <213> Homo sapiens
<400> 47
   taacttacgg agtcgctcta cggcatggag cacttgcgag aggtgagggc agtta 55
<210> 48
   <211> 60
   <212> DNA
   <213> Homo sapiens
<400> 48
   taacttacgg agtcgctcta cgagtggtgt gagggctcca gcttgtatca ccatctattg 60